(19) Europäisches Patentamt / European Patent Office / Office européen des brevets

(11) **EP 4 678 633 A1**

(12) **EUROPEAN PATENT APPLICATION**

(43) Date of publication:
**14.01.2026 Bulletin 2026/03**

(21) Application number: **24187618.4**

(22) Date of filing: **10.07.2024**

(51) International Patent Classification (IPC):
***C07D 211/18*** *(2006.01)* ***C07D 295/16*** *(2006.01)*
***C07D 207/08*** *(2006.01)* ***A61P 11/00*** *(2006.01)*
***A61K 31/40*** *(2006.01)* ***A61K 31/44*** *(2006.01)*
***A61P 13/12*** *(2006.01)* ***A61P 25/00*** *(2006.01)*

(52) Cooperative Patent Classification (CPC):
**C07D 295/16; A61P 11/00; A61P 13/12; A61P 25/00; C07D 207/08; C07D 211/18**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Universiteit Antwerpen**
**2000 Antwerpen (BE)**

(72) Inventors:
- **Augustyns, Koen**
  **2020 Antwerpen (BE)**
- **Van der Veken, Pieter**
  **2020 Antwerpen (BE)**
- **Vanden Berghe, Tom**
  **2020 Antwerpen (BE)**
- **Hassannia, Behrouz**
  **2020 Antwerpen (BE)**
- **Lanthier, Caroline**
  **2020 Antwerpen (BE)**
- **Scarpellini, Camilla**
  **2020 Antwerpen (BE)**
- **Klejborowska, Greta**
  **2020 Antwerpen (BE)**

(74) Representative: **De Clercq & Partners**
**Edgard Gevaertdreef 10a**
**9830 Sint-Martens-Latem (BE)**

(54) **NOVEL FERROPTOSIS INHIBITORS**

(57) The present invention relates to a compound of formula (I) or a stereoisomer, or tautomer,

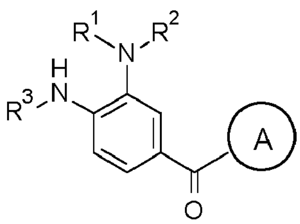

(I)

wherein cycle A, $R^1$, $R^2$, and $R^3$ have the same meaning as that defined in the claims and the description. The present invention also relates to the use of such compounds for the prevention and/or treatment of a disease associated with ferroptosis and/or oxytosis such as liver disease, chronic kidney disease, lung disease, ocular surface diseases, wound healing, multiple organ dysfunction syndrome, neurological disease, acute renal failure, ischemia-reperfusion injury, sepsis, prevention of transplant rejection, iron toxicity, iron metabolism-related disease and genetic disorders of GPX4. The present invention also provides pharmaceutical compositions comprising such compounds, as well as the use of the compounds as a medicament and methods of prevention and/or treatment of such diseases.

EP 4 678 633 A1

## Description

### Field of the invention

The present invention relates to ferrostatin-1 derivative compounds, or pharmaceutically acceptable salts thereof, as defined herein, that are useful for inhibiting undesired cell death occurring in diseases associated with ferroptosis and/or oxytosis such as liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant complications, iron metabolism-related disease, and genetic disorders of GPX4. The invention also provides compositions containing a pharmaceutically acceptable carrier and one or more compounds from the invention methods of prevention and/or treatment of such diseases.

### Background of the invention

Cell death is crucial for normal development, homeostasis, and the prevention of hyperproliferative diseases such as cancer. It was once thought that almost all regulated cell death in mammalian cells resulted from the activation of caspase-dependent apoptosis. This view has been challenged by the discovery of several regulated non-apoptotic cell death pathways activated in specific disease states. Regulated necrosis is defined as a genetically controlled cell death process that eventually results in cellular leakage, and is morphologically characterized by cytoplasmic granulation, as well as organelle and/or cellular swelling. Ferroptosis is one recognized form of regulated necrosis and its hallmark is the production of iron-dependent lipophilic reactive oxygen species (ROS). Cell membrane rupture during ferroptotic cell death is characterized by hydrogen abstraction and oxygenation of poly unsaturated fatty acids (PUFAs) of phospholipids (PLs), which is catalysed by redox-active iron. This subsequently leads to cell death due to disruption of membrane stability and the accumulation of lipid hydroperoxides to lethal levels. Although the process of lipid peroxidation has been linked to several regulated cell death modalities, ferroptosis is exclusively driven by excessive lipid peroxidation. Oxidative damage of PUFA-PLs can be initiated either through non-enzymatic free-radical chain reactions involving Fenton chemistry or enzyme-mediated processes catalysed by iron-dependent lipoxygenases (LOXs) or cytochrome P450 oxidoreductase (POR). In addition to ferroptosis, glutamine- and oxidative stress induced cell death are inhibited by iron chelation. In line with this, iron-dependent neuronal cell death is blocked by metal protein-attenuating compounds (e.g. clioquinol) and iron chelators (e.g. deferoxamine), which are being explored for the treatment of neurodegenerative diseases. Another type of regulated necrosis is oxytosis which is also induced when the $Xc^-$ Cys/Glu antiporter is inhibited through an excess of the neurotransmitter glutamine, the latter process is often designated as excitotoxicity in neuronal cells. Because of the clear mechanistic overlaps between oxytosis and ferroptosis it is likely that the use of modulators of ferroptosis in disease will target the same disease processes which are associated with oxytosis. Disease processes where undesired ferroptosis and/or oxytosis occur are typically disorders where an oxidative stress factor is involved such as for example in several neurodegenerative diseases, liver-, cardiac- and kidney-ischaemia - reperfusion injury, stroke, sepsis, diabetes and epilepsy. Oxidative stress due to iron overload is for example highly relevant in organs accumulating iron such as the brain, kidney and liver. Several compounds have been described in the art which are able to inhibit ferroptosis such as for example WO2013/152039, Skouta R. et al (2014) J. Am. Chem. Soc. 136, 4551-4556). The prior art highlights the importance of the ethyl-ester in the maintenance of the potency of the first-in-class compound ferroptosis inhibitor molecule (designated as Ferrostatin-1) and there have been suggestions for chain modifications of the ester for generating improved molecules. Indeed, the latter reference also teaches that esters modified to amides and sulfonamides at the same position have a lower EC50. It has also been suggested that improved pharmacokinetic variants of Ferrostatin-1 could be ester analogues. It would be desirable to generate additional compounds that can inhibit or reduce ferroptosis, in particular compounds with an improved physicochemical and/or pharmacokinetic profile.

There is a need for small molecules that use ferroptosis and/or oxytosis as their target, have strong selectivity, have properties that are amenable to develop them as pharmaceuticals, such as high solubility, and have significant efficacy for the treatment of the ferroptosis and/or oxytosis related diseases.

### Summary of the invention

The present invention is based on the unexpected finding that at least one of the above-mentioned objectives can be attained by small molecules.

The present invention provides compounds which have surprisingly found to be potent inhibitors of ferroptosis and/or oxytosis, while also displaying excellent solubility; in addition, the compounds of the invention show moderate to high CNS MPO scores and low *in vitro* cytotoxicity. In view thereof, these compounds can be used to treat diseases where an excess of ferroptosis and/or oxytosis occurs.

A first aspect of the present invention provides a compound of formula (I) or a stereoisomer, or tautomer thereof,

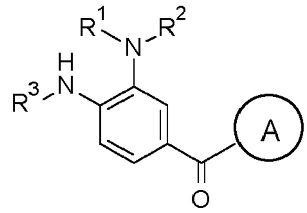

(I)

wherein, cycle A is selected from:

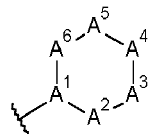

or

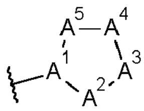

;

wherein the wavy line ( ~ ) indicates the point of attachment of cycle A to the rest of the molecule,

$A^1$ is selected from N or $CR^4$;

$A^2$ is selected from the group consisting of $NR^5$, $CR^6R^7$, S, $SO_2$ and O;

$A^3$ is selected from the group consisting of $NR^5$, $CR^6R^7$, S, $SO_2$ and O;

$A^4$ is selected from the group consisting of $NR^5$, $CR^6R^7$, S, $SO_2$ and O;

$A^5$ is selected from the group consisting of $NR^5$, $CR^6R^7$, S, $SO_2$ and O;

$A^6$ is selected from the group consisting of $NR^5$, $CR^6R^7$, S, $SO_2$ and O;

wherein when $A^1$ is $CR^4$, then at least one of $A^2$, $A^3$, $A^4$, $A^5$, or $A^6$ is selected from the group consisting of $NR^5$, $CR^6R^7$, S, $SO_2$ and O;

$R^1$ is selected from the group consisting of hydrogen, $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl-$C_{1-6}$alkyl, or heteroaryl$C_{1-6}$alkyl can be unsubstituted or substituted with one or more $Z^1$;

$R^2$ is selected from the group consisting of $C_{3-10}$cycloalkyl, $C_{6-12}$aryl$C_{1-6}$alkyl, hydrogen, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl-$C_{1-6}$alkyl, or heteroaryl$C_{1-6}$alkyl can be unsubstituted or substituted with one or more $Z^2$;

$R^3$ is selected from the group consisting of $C_{3-10}$cycloalkyl, $C_{3-10}$cycloalkenyl, $C_{3-10}$cycloalkynyl, $C_{6-12}$aryl, heterocyclyl, and heteroaryl, wherein said $C_{3-10}$cycloalkyl, $C_{3-10}$cycloalkenyl, $C_{3-10}$cycloalkynyl, $C_{6-12}$aryl, heterocyclyl or heteroaryl can be unsubstituted or substituted with one or more $Z^3$;

$R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$, wherein said $C_{1-6}$alkyl, $C_{1-10}$cycloalkyl, $C_{6-12}$aryl, $C_{6-12}$aryl$C_{1-6}$alkyl, heterocyclyl, or heteroaryl can be unsubstituted or substituted with one or more Z4;

each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl, or heteroaryl can be unsubstituted or substituted with one or more $Z^5$;

each $R^6$ is independently selected from the group consisting of hydrogen, $C_{1-6}$alkyl, halogen, hydroxy, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-NR^8R^9$, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl or heteroaryl can be unsubstituted or substituted with one or more $Z^6$;

each $R^7$ is independently selected from the group consisting of hydrogen, $C_{1-6}$alkyl, halogen, hydroxy, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-NR^8R^9$, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl or heteroaryl can be unsubstituted or substituted with one or more $Z^7$;

each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;

each $R^{10}$ is independently selected from the group consisting of $C_{1-6}$alkyl, hydrogen, $C_{3-10}$cycloalkyl, $C_{6-12}$aryl,

$C_{6-12}$aryl$C_{1-6}$alkyl, heterocyclyl, and heteroaryl;
each $Z^1$, $Z^2$, $Z^3$, $Z^4$, $Z^5$, $Z^6$ and $Z^7$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, -$OR^{10}$, cyano, amino, -$NR^8R^9$, -$C(O)_2R^{10}$, -$C(O)NR^8R^9$, -$C(O)R^{10}$, -$S(O)R^{10}$, -$S(O)_2R^{10}$, -$S(O)_2NR^8R^9$, nitro;
or a solvate, hydrate, pharmaceutically acceptable salt, or prodrug thereof;
wherein when the moiety -$NR^1R^2$ is

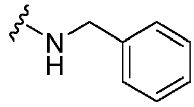
,

cycle A is piperazinyl and $R^3$ is cyclohexyl, then $R^5$ is not substituted benzyl, 4-methylpyridine or methyl.

A second, related aspect of the present invention provides a pharmaceutical composition comprising a compound of formula (I) as described in the first aspect of the invention, and a pharmaceutically acceptable carrier.
A third aspect of the present invention provides a compound of formula (I) as described in the first aspect of the invention or a pharmaceutical composition as described in the second aspect of the invention for use as a medicament.
According to a fourth, related aspect, the present invention also encompasses a compound according to the first aspect of the invention, or a pharmaceutical composition according to the second aspect of the invention for use in the prevention or treatment of a disease associated with ferroptosis and/or oxytosis.
According to a fifth, related aspect, the present invention also encompasses a compound according to the first aspect of the invention or a or a pharmaceutical composition according to the second aspect of the invention for use in the prevention or treatment of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4.
The present invention will now be further described. In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.
The independent and dependent claims set out particular and preferred features of the invention. Features from the dependent claims may be combined with features of the independent or other dependent claims as appropriate.

**Detailed description of the invention**

Before the present invention is described, it is to be understood that this invention is not limited to particular processes, methods, and compounds described, as such processes, methods, and compounds may, of course, vary. It is also to be understood that the terminology used herein is not intended to be limiting, since the scope of the present invention will be limited only by the appended claims.
When describing the compounds and processes of the invention, the terms used are to be construed in accordance with the following definitions, unless a context dictates otherwise.
As used in the specification and the appended claims, the singular forms "a", "an," and "the" include both singular and plural referents unless the context clearly dictates otherwise. By way of example, "a compound" means one compound or more than one compound.
The terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".
The term "about" as used herein when referring to a measurable value such as a parameter, an amount, a temporal duration, and the like, is meant to encompass variations of +/-10% or less, preferably +/-5% or less, more preferably +/-1 % or less, and still more preferably +/-0.1% or less of and from the specified value, insofar such variations are appropriate to perform in the disclosed invention. It is to be understood that the value to which the modifier "about" refers is itself also specifically, and preferably, disclosed.
As used herein, the term "and/or," when used in a list of two or more items, means that any one of the listed items can be employed by itself or any combination of two or more of the listed items can be employed. For example, if a list is described as comprising group A, B, and/or C, the list can comprise A alone; B alone; C alone; A and B in combination; A and C in combination, B and C in combination; or A, B, and C in combination.
The recitation of numerical ranges by endpoints includes all integer numbers and, where appropriate, fractions

subsumed within that range (e.g. 1 to 5 can include 1, 2, 3, 4 when referring to, for example, a number of elements, and can also include 1.5, 2, 2.75 and 3.80, when referring to, for example, measurements). The recitation of end points also includes the end point values themselves (e.g. from 1.0 to 5.0 includes both 1.0 and 5.0). Any numerical range recited herein is intended to include all sub-ranges subsumed therein.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiments but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments combinations of features of different embodiments are meant to be within the scope of the invention, and form different embodiments as would be understood by those in the art. For example, in the following claims, any of the claimed embodiments can be used in any combination.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention.

When describing the present invention, the terms used are to be construed in accordance with the following definitions, unless a context dictates otherwise.

The terms described above and others used in the specification are well understood to those in the art.

Whenever the term "substituted" is used herein, it is meant to indicate that one or more hydrogen atoms on the atom indicated in the expression using "substituted" is replaced with a selection from the indicated group, provided that the indicated atom's normal valence is not exceeded, and that the substitution results in a chemically stable compound, i.e. a compound that is sufficiently robust to survive isolation from a reaction mixture.

Where groups can be substituted, such groups may be substituted with one or more, and preferably one, two or three substituents. Preferred substituents may be selected from but not limited to, for example, the group comprising halo, hydroxyl, alkyl, alkoxy, trifluoromethyl, trifluoromethoxy, cycloalkyl, aryl, arylalkyl, heterocyclyl, heteroaryl, cyano, amino, nitro, carboxyl, and mono- or dialkylamino.

The term "halo" or "halogen" as a group or part of a group is generic for fluoro, chloro, bromo, iodo.

The term "hydroxyl" or "hydroxy" as used herein refers to the group -OH.

The term "cyano" as used herein refers to the group -C=N.

The term "amino" as used herein refers to the $-NH_2$ group.

The term "nitro" as used herein refers to the $-NO_2$ group.

The term "carboxy" or "carboxyl" or "hydroxycarbonyl" as used herein refers to the group $-CO_2H$. The term "aminocarbonyl" as used herein refers to the group $-CONH_2$.

The term "alkyl", as a group or part of a group, refers to a hydrocarbyl group of formula $-C_nH_{2n+1}$ wherein n is a number greater than or equal to 1. Alkyl groups may be linear or branched and may be substituted as indicated herein. Generally, alkyl groups of this invention comprise from 1 to 6 carbon atoms, preferably from 1 to 5 carbon atoms, preferably from 1 to 4 carbon atoms, more preferably from 1 to 3 carbon atoms, still more preferably 1 to 2 carbon atoms. When a subscript is used herein following a carbon atom, the subscript refers to the number of carbon atoms that the named group may contain. For example, "$C_{1-6}$alkyl" includes all linear or branched alkyl groups with between 1 and 6 carbon atoms, and thus includes methyl, ethyl, n-propyl, i-propyl, butyl and its isomers (e.g. n-butyl, i-butyl and t-butyl); pentyl and its isomers, hexyl and its isomers. For example, "$C_{1-5}$alkyl" includes all includes all linear or branched alkyl groups with between 1 and 5 carbon atoms, and thus includes methyl, ethyl, n-propyl, i-propyl, butyl and its isomers (e.g. n-butyl, i-butyl and t-butyl); pentyl and its isomers. For example, "$C_{1-4}$alkyl" includes all linear or branched alkyl groups with between 1 and 4 carbon atoms, and thus includes methyl, ethyl, n-propyl, i-propyl, butyl and its isomers (e.g. n-butyl, i-butyl and t-butyl). For example "$C_{1-3}$alkyl" includes all linear or branched alkyl groups with between 1 and 3 carbon atoms, and thus includes methyl, ethyl, n-propyl, i-propyl.

When the term "alkyl" is used as a suffix following another term, as in "hydroxyalkyl," this is intended to refer to an alkyl group, as defined above, being substituted with one or two (preferably one) substituent(s) selected from the other, specifically-named group, also as defined herein. The term "hydroxyalkyl" therefore refers to a $-R^a$-OH group wherein $R^a$ is alkylene as defined herein.

The term "haloalkyl" as a group or part of a group, refers to an alkyl group having the meaning as defined above wherein one, two, or three hydrogen atoms are each replaced with a halogen as defined herein. Non-limiting examples of such haloalkyl groups include chloromethyl, 1-bromoethyl, fluoromethyl, difluoromethyl, trifluoromethyl, 1,1,1-trifluoroethyl, trichloromethyl, tribromomethyl, and the like.

The term "alkoxy" or "alkyloxy", as a group or part of a group, refers to a group having the formula $-OR^b$ wherein $R^b$ is alkyl as defined herein above. Non-limiting examples of suitable alkoxy include methoxy, ethoxy, propoxy, isopropoxy,

butoxy, isobutoxy, sec-butoxy, tert-butoxy, pentyloxy and hexyloxy.

The term "cycloalkyl", as a group or part of a group, refers to a cyclic alkyl group, that is a monovalent, saturated, hydrocarbyl group having 1 or more cyclic structure, and comprising from 3 to 10 carbon atoms, more preferably from 3 to 9 carbon atoms, more preferably from 3 to 7 carbon atoms; more preferably from 3 to 6 carbon atoms. Cycloalkyl includes all saturated hydrocarbon groups containing 1 or more rings, including monocyclic or bicyclic groups. The further rings of multi-ring cycloalkyls may be either fused, bridged and/or joined through one or more spiro atoms. When a subscript is used herein following a carbon atom, the subscript refers to the number of carbon atoms that the named group may contain. For example, the term "$C_{3-}$scycloalkyl", a cyclic alkyl group comprising from 3 to 8 carbon atoms. For example, the term "$C_{3-}$scycloalkyl", a cyclic alkyl group comprising from 3 to 6 carbon atoms. Examples of $C_{3-10}$cycloalkyl groups include but are not limited to cyclopropyl, cyclobutyl, cyclopentyl, cyclohexyl, cycloheptyl, cyclooctyl, bicycle[2.2.1]heptan-2yl, (1S,4R)-norbornan-2-yl, (1R,4R)-norbornan-2-yl, (1S,4S)-norbornan-2-yl, (1R,4S)-norbornan-2-yl, 1-adamantyl.

The term "cycloalkyloxy", as a group or part of a group, refers to a group having the formula - $OR^f$ wherein $R^f$ is cycloalkyl as defined herein above.

The term "cycloalkenyl", as a group or part of a group, refers to a cyclic alkenyl group, that is a monovalent, unsaturated, hydrocarbyl group having 1 or more cyclic structure, comprising one or more carbon-carbon double bonds; preferably 3 double bonds; preferably 2 double bonds; preferably one double bond. Cycloalkenyl groups according to the present invention comprise from 3 to 10 carbon atoms, more preferably from 3 to 9 carbon atoms, more preferably from 3 to 7 carbon atoms; more preferably from 3 to 6 carbon atoms. Cycloalkenyl includes all unsaturated hydrocarbon groups containing 1 or more rings, including monocyclic or bicyclic groups comprising at least one double bond. The further rings of multi-ring cycloalkenyls may be either fused, bridged and/or joined through one or more spiro atoms. When a subscript is used herein following a carbon atom, the subscript refers to the number of carbon atoms that the named group may contain. For example, the term "$C_{3-8}$cycloalkenyl", a cyclic alkenyl group comprising from 3 to 8 carbon atoms. For example, the term "Ca-scycloalkenyl", a cyclic alkenyl group comprising from 3 to 6 carbon atoms. Examples of $C_{3-10}$cycloalkeyl groups include but are not limited to cyclopropenyl, cyclobutenyl, cyclopentenyl, cyclohexenyl, cycloheptenyl, cyclooctenyl.

The term "cycloalkynyl", as a group or part of a group, refers to a cyclic alkynyl group, that is a monovalent, unsaturated, hydrocarbyl group having 1 or more cyclic structure, comprising one or more carbon-carbon triple bonds; preferably 3 triple bonds; preferably 2 triple bonds; preferably one double bond. Cycloalkynyl groups according to the present invention comprise from 3 to 10 carbon atoms, more preferably from 3 to 9 carbon atoms, more preferably from 3 to 7 carbon atoms; more preferably from 3 to 6 carbon atoms. Cycloalkynyl includes all unsaturated hydrocarbon groups containing 1 or more rings, including monocyclic or bicyclic groups, comprising at least one triple bond. The further rings of multi-ring cycloalkynyls may be either fused, bridged and/or joined through one or more spiro atoms. When a subscript is used herein following a carbon atom, the subscript refers to the number of carbon atoms that the named group may contain. For example, the term "$C_{3-8}$cycloalkynyl", a cyclic alkynyl group comprising from 3 to 8 carbon atoms. For example, the term "Ca-scycloalkynyl", a cyclic alkynnyl group comprising from 3 to 6 carbon atoms. Examples of $C_{3-10}$cycloalkynyl groups include but are not limited to cyclopropynyl, cyclobutynyl, cyclopentynyl, cyclohexynyl, cycloheptynyl, cyclooctynyl.

The term "aryl", as a group or part of a group, refers to a polyunsaturated, aromatic hydrocarbyl group having a single ring (i.e. phenyl) or multiple aromatic rings fused together (e.g. naphthyl), or linked covalently, typically comprising 6 to 12 carbon atoms; wherein at least one ring is aromatic, preferably comprising 6 to 10 carbon atoms, wherein at least one ring is aromatic. The aromatic ring may optionally include one to two additional rings (either cycloalkyl, heterocyclyl or heteroaryl) fused thereto. Examples of suitable aryl include $C_{6-12}$aryl, preferably $C_{6-10}$aryl, more preferably $C_{6-8}$aryl. Non-limiting examples of aryl comprise phenyl, biphenylyl, biphenylenyl, or 1-or 2-naphthanelyl; 5- or 6-tetralinyl, 1-, 2-, 3-, 4-, 5-, 6-, 7- or 8-azulenyl, 4-, 5-, 6 or 7-indenyl, 4- or 5-indanyl, 5-, 6-, 7- or 8-tetrahydronaphthyl, 1,2,3,4-tetrahydronaphthyl, and 1,4-dihydronaphthyl; 1-, 2-, 3-, 4- or 5-pyrenyl. A "substituted aryl" refers to an aryl group having one or more substituent(s) (for example 1, 2 or 3 substituent(s), or 1 to 2 substituent(s)), at any available point of attachment.

The term "aryloxy", as a group or part of a group, refers to a group having the formula $-OR^g$ wherein $R^g$ is aryl as defined herein above.

The term "arylalkyl", as a group or part of a group, means a alkyl as defined herein, wherein at least one hydrogen atom is replaced by at least one aryl as defined herein. Non-limiting examples of arylalkyl group include benzyl, phenethyl, dibenzylmethyl, methylphenylmethyl, 3-(2-naphthyl)-butyl, and the like.

The terms "heterocyclyl" or "heterocycloakyl" or "heterocyclo", as a group or part of a group, refer to non-aromatic, fully saturated or partially unsaturated cyclic groups (for example, 3 to 7 member monocyclic, 7 to 11 member bicyclic, or comprising a total of 3 to 10 ring atoms) which have at least one heteroatom in at least one carbon atom-containing ring; wherein said ring may be fused to an aryl, cycloalkyl, heteroaryl or heterocyclyl ring. Each ring of the heterocyclyl group containing a heteroatom may have 1, 2, 3 or 4 heteroatoms selected from N, O and/or S, where the N and S heteroatoms may optionally be oxidized and the N heteroatoms may optionally be quaternized, and wherein at least one carbon atom of heterocyclyl can be oxidized to form at least one C=O. The heterocyclic group may be attached at any

heteroatom or carbon atom of the ring or ring system, where valence allows. The rings of multi-ring heterocycles may be fused, bridged and/or joined through one or more spiro atoms.

Non limiting exemplary heterocyclic groups include aziridinyl, oxiranyl, thiiranyl, piperidinyl, azetidinyl, oxetanyl, pyrrolidinyl, thietanyl, 2-imidazolinyl, pyrazolidinyl imidazolidinyl, isoxazolinyl, oxazolidinyl, isoxazolidinyl, thiazolidinyl, isothiazolidinyl, piperidinyl, succinimidyl, 3H-indolyl, indolinyl, chromanyl (also known as 3,4-dihydrobenzo[b]pyranyl), isoindolinyl, 2H-pyrrolyl, 1-pyrrolinyl, 2-pyrrolinyl, 3-pyrrolinyl, 4H-quinolizinyl, 2-oxopiperazinyl, piperazinyl, homopiperazinyl, 2-pyrazolinyl, 3-pyrazolinyl, tetrahydro-2H-pyranyl, 2H-pyranyl, 4H-pyranyl, 3,4-dihydro-2H-pyranyl, 3-dioxolanyl, 1,4-dioxanyl, 2,5-dioximidazolidinyl, 2-oxopiperidinyl, 2-oxopyrrolodinyl, indolinyl, tetrahydropyranyl, tetrahydrofuranyl, tetrahydrothiophenyl, tetrahydroquinolinyl, tetrahydroisoquinolin-1-yl, tetrahydroisoquinolin-2-yl, tetrahydroisoquinolin-3-yl, tetrahydroisoquinolin-4-yl, thiomorpholin-4-yl, thiomorpholin-4-ylsulfoxide, thiomorpholin-4-ylsulfone, 1,3-dioxolanyl, 1,4-oxathianyl, 1,4-dithianyl, 1,3,5-trioxanyl, 1H-pyrrolizinyl, tetrahydro-1,1-dioxothiophenyl, N- formylpiperazinyl, and morpholin-4-yl. The term "aziridinyl" as used herein includes aziridin-1-yl and aziridin-2-yl. The term "oxyranyl" as used herein includes oxyranyl-2-yl. The term "thiiranyl" as used herein includes thiiran-2-yl. The term "azetidinyl" as used herein includes azetidin-1-yl, azetidin-2-yl and azetidin-3-yl. The term "oxetanyl" as used herein includes oxetan-2-yl and oxetan-3-yl. The term "thietanyl" as used herein includes thietan-2-yl and thietan-3-yl. The term "pyrrolidinyl" as used herein includes pyrrolidin-1-yl, pyrrolidin-2-yl and pyrrolidin-3-yl. The term "tetrahydrofuranyl" as used herein includes tetrahydrofuran-2-yl and tetrahydrofuran-3-yl. The term "tetrahydrothiophenyl" as used herein includes tetrahydrothiophen-2-yl and tetrahydrothiophen-3-yl. The term "succinimidyl" as used herein includes succinimid-1-yl and succininmid-3-yl. The term "dihydropyrrolyl" as used herein includes 2,3-dihydropyrrol-1-yl, 2,3-dihydro-1H-pyrrol-2-yl, 2,3-dihydro-1H-pyrrol-3-yl, 2,5-dihydropyrrol-1-yl, 2,5-dihydro-1H-pyrrol-3-yl and 2,5-dihydropyrrol-5-yl. The term "2H-pyrrolyl" as used herein includes 2H-pyrrol-2-yl, 2H-pyrrol-3-yl, 2H-pyrrol-4-yl and 2H-pyrrol-5-yl. The term "3H-pyrrolyl" as used herein includes 3H-pyrrol-2-yl, 3H-pyrrol-3-yl, 3H-pyrrol-4-yl and 3H-pyrrol-5-yl. The term "dihydrofuranyl" as used herein includes 2,3-dihydrofuran-2-yl, 2,3-dihydrofuran-3-yl, 2,3-dihydrofuran-4-yl, 2,3-dihydrofuran-5-yl, 2,5-dihydrofuran-2-yl, 2,5-dihydrofuran-3-yl, 2,5-dihydrofuran-4-yl and 2,5-dihydrofuran-5-yl. The term "dihydrothiophenyl" as used herein includes 2,3-dihydrothiophen-2-yl, 2,3-dihydrothiophen-3-yl, 2,3-dihydrothiophen-4-yl, 2,3-dihydrothiophen-5-yl, 2,5-dihydrothiophen-2-yl, 2,5-dihydrothiophen-3-yl, 2,5-dihydrothiophen-4-yl and 2,5-dihydrothiophen-5-yl. The term "imidazolidinyl" as used herein includes imidazolidin-1-yl, imidazolidin-2-yl and imidazolidin-4-yl. The term "pyrazolidinyl" as used herein includes pyrazolidin-1-yl, pyrazolidin-3-yl and pyrazolidin-4-yl. The term "imidazolinyl" as used herein includes imidazolin-1-yl, imidazolin-2-yl, imidazolin-4-yl and imidazolin-5-yl. The term "pyrazolinyl" as used herein includes 1-pyrazolin-3-yl, 1-pyrazolin-4-yl, 2-pyrazolin-1-yl, 2-pyrazolin-3-yl, 2-pyrazolin-4-yl, 2-pyrazolin-5-yl, 3-pyrazolin-1-yl, 3-pyrazolin-2-yl, 3-pyrazolin-3-yl, 3-pyrazolin-4-yl and 3-pyrazolin-5-yl. The term "dioxolanyl" also known as "1,3-dioxolanyl" as used herein includes dioxolan-2-yl, dioxolan-4-yl and dioxolan-5-yl. The term "dioxolyl" also known as "1,3-dioxolyl" as used herein includes dioxol-2-yl, dioxol-4-yl and dioxol-5-yl. The term "oxazolidinyl" as used herein includes oxazolidin-2-yl, oxazolidin-3-yl, oxazolidin-4-yl and oxazolidin-5-yl. The term "isoxazolidinyl" as used herein includes isoxazolidin-2-yl, isoxazolidin-3-yl, isoxazolidin-4-yl and isoxazolidin-5-yl. The term "oxazolinyl" as used herein includes 2-oxazolinyl-2-yl, 2-oxazolinyl-4-yl, 2-oxazolinyl-5-yl, 3-oxazolinyl-2-yl, 3-oxazolinyl-4-yl, 3-oxazolinyl-5-yl, 4-oxazolinyl-2-yl, 4-oxazolinyl-3-yl, 4-oxazolinyl-4-yl and 4-oxazolinyl-5-yl. The term "isoxazolinyl" as used herein includes 2-isoxazolinyl-3-yl, 2-isoxazolinyl-4-yl, 2-isoxazolinyl-5-yl, 3-isoxazolinyl-3-yl, 3-isoxazolinyl-4-yl, 3-isoxazolinyl-5-yl, 4-isoxazolinyl-2-yl, 4-isoxazolinyl-3-yl, 4-isoxazolinyl-4-yl and 4-isoxazolinyl-5-yl. The term "thiazolidinyl" as used herein includes thiazolidin-2-yl, thiazolidin-3-yl, thiazolidin-4-yl and thiazolidin-5-yl. The term "isothiazolidinyl" as used herein includes isothiazolidin-2-yl, isothiazolidin-3-yl, isothiazolidin-4-yl and isothiazolidin-5-yl. The term "chromanyl" as used herein includes chroman-2-yl, chroman-3-yl, chroman-4-yl, chroman-5-yl, chroman-6-yl, chroman-7-yl and chroman-8-yl. The term "thiazolinyl" as used herein includes 2-thiazolinyl-2-yl, 2-thiazolinyl-4-yl, 2-thiazolinyl-5-yl, 3-thiazolinyl-2-yl, 3-thiazolinyl-4-yl, 3-thiazolinyl-5-yl, 4-thiazolinyl-2-yl, 4-thiazolinyl-3-yl, 4-thiazolinyl-4-yl and 4-thiazolinyl-5-yl. The term "isothiazolinyl" as used herein includes 2-isothiazolinyl-3-yl, 2-isothiazolinyl-4-yl, 2-isothiazolinyl-5-yl, 3-isothiazolinyl-3-yl, 3-isothiazolinyl-4-yl, 3-isothiazolinyl-5-yl, 4-isothiazolinyl-2-yl, 4-isothiazolinyl-3-yl, 4-isothiazolinyl-4-yl and 4-isothiazolinyl-5-yl. The term "piperidyl" also known as "piperidinyl" as used herein includes piperid-1-yl, piperid-2-yl, piperid-3-yl and piperid-4-yl. The term "dihydropyridinyl" as used herein includes 1,2-dihydropyridin-1-yl, 1,2-dihydropyridin-2-yl, 1,2-dihydropyridin-3-yl, 1,2-dihydropyridin-4-yl, 1,2-dihydropyridin-5-yl, 1,2-dihydropyridin-6-yl, 1,4-dihydropyridin-1-yl, 1,4-dihydropyridin-2-yl, 1,4-dihydropyridin-3-yl, 1,4-dihydropyridin-4-yl, 2,3-dihydropyridin-2-yl, 2,3-dihydropyridin-3-yl, 2,3-dihydropyridin-4-yl, 2,3-dihydropyridin-5-yl, 2,3-dihydropyridin-6-yl, 2,5-dihydropyridin-2-yl, 2,5-dihydropyridin-3-yl, 2,5-dihydropyridin-4-yl, 2,5-dihydropyridin-5-yl, 2,5-dihydropyridin-6-yl, 3,4-dihydropyridin-2-yl, 3,4-dihydropyridin-3-yl, 3,4-dihydropyridin-4-yl, 3,4-dihydropyridin-5-yl and 3,4-dihydropyridin-6-yl. The term "tetrahydropyridinyl" as used herein includes 1,2,3,4-tetrahydropyridin-1-yl, 1,2,3,4-tetrahydropyridin-2-yl, 1,2,3,4-tetrahydropyridin-3-yl, 1,2,3,4-tetrahydropyridin-4-yl, 1,2,3,4-tetrahydropyridin-5-yl, 1,2,3,4-tetrahydropyridin-6-yl, 1,2,3,6-tetrahydropyridin-1-yl, 1,2,3,6-tetrahydropyridin-2-yl, 1,2,3,6-tetrahydropyridin-3-yl, 1,2,3,6-tetrahydropyridin-4-yl, 1,2,3,6-tetrahydropyridin-5-yl, 1,2,3,6-tetrahydropyridin-6-yl, 2,3,4,5-tetrahydropyridin-2-yl, 2,3,4,5-tetrahydropyridin-3-yl, 2,3,4,5-tetrahydropyridin-3-yl, 2,3,4,5-tetrahydropyridin-4-yl, 2,3,4,5-tetrahydropyridin-5-yl and 2,3,4,5-tetrahydropyridin-6-yl. The term "tetrahydropyranyl"

also known as "oxanyl" or "tetrahydro-2H-pyranyl", as used herein includes tetrahydropyran-2-yl, tetrahydropyran-3-yl and tetrahydropyran-4-yl. The term "2H-pyranyl" as used herein includes 2H-pyran-2-yl, 2H-pyran-3-yl, 2H-pyran-4-yl, 2H-pyran-5-yl and 2H-pyran-6-yl. The term "4H-pyranyl" as used herein includes 4H-pyran-2-yl, 4H-pyran-3-yl and 4H-pyran-4-yl. The term "3,4-dihydro-2H-pyranyl" as used herein includes 3,4-dihydro-2H-pyran-2-yl, 3,4-dihydro-2H-pyran-3-yl, 3,4-dihydro-2H-pyran-4-yl, 3,4-dihydro-2H-pyran-5-yl and 3,4-dihydro-2H-pyran-6-yl. The term "3,6-dihydro-2H-pyranyl" as used herein includes 3,6-dihydro-2H-pyran-2-yl, 3,6-dihydro-2H-pyran-3-yl, 3,6-dihydro-2H-pyran-4-yl, 3,6-dihydro-2H-pyran-5-yl and 3,6-dihydro-2H-pyran-6-yl. The term "tetrahydrothiophenyl", as used herein includes tetrahydrothiophen-2-yl, tetrahydrothiophenyl -3-yl and tetrahydrothiophenyl -4-yl. The term "2H-thiopyranyl" as used herein includes 2H-thiopyran-2-yl, 2H-thiopyran-3-yl, 2H-thiopyran-4-yl, 2H-thiopyran-5-yl and 2H-thiopyran-6-yl. The term "4H-thiopyranyl" as used herein includes 4H-thiopyran-2-yl, 4H-thiopyran-3-yl and 4H-thiopyran-4-yl. The term "3,4-dihydro-2H-thiopyranyl" as used herein includes 3,4-dihydro-2H-thiopyran-2-yl, 3,4-dihydro-2H-thiopyran-3-yl, 3,4-dihydro-2H-thiopyran-4-yl, 3,4-dihydro-2H-thiopyran-5-yl and 3,4-dihydro-2H-thiopyran-6-yl. The term "3,6-dihydro-2H-thiopyranyl" as used herein includes 3,6-dihydro-2H-thiopyran-2-yl, 3,6-dihydro-2H-thiopyran-3-yl, 3,6-dihydro-2H-thiopyran-4-yl, 3,6-dihydro-2H-thiopyran-5-yl and 3,6-dihydro-2H-thiopyran-6-yl. The term "piperazinyl" also known as "piperazidinyl" as used herein includes piperazin-1-yl and piperazin-2-yl. The term "morpholinyl" as used herein includes morpholin-2-yl, morpholin-3-yl and morpholin-4-yl. The term "thiomorpholinyl" as used herein includes thiomorpholin-2-yl, thiomorpholin-3-yl and thiomorpholin-4-yl. The term "dioxanyl" as used herein includes 1,2-dioxan-3-yl, 1,2-dioxan-4-yl, 1,3-dioxan-2-yl, 1,3-dioxan-4-yl, 1,3-dioxan-5-yl and 1,4-dioxan-2-yl. The term "dithianyl" as used herein includes 1,2-dithian-3-yl, 1,2-dithian-4-yl, 1,3-dithian-2-yl, 1,3-dithian-4-yl, 1,3-dithian-5-yl and 1,4-dithian-2-yl. The term "oxathianyl" as used herein includes oxathian-2-yl and oxathian-3-yl. The term "trioxanyl" as used herein includes 1,2,3-trioxan-4-yl, 1,2,3-trioxay-5-yl, 1,2,4-trioxay-3-yl, 1,2,4-trioxay-5-yl, 1,2,4-trioxay-6-yl and 1,3,4-trioxay-2-yl. The term "azepanyl" as used herein includes azepan-1-yl, azepan-2-yl, azepan-1-yl, azepan-3-yl and azepan-4-yl. The term "homopiperazinyl" as used herein includes homopiperazin-1-yl, homopiperazin-2-yl, homopiperazin-3-yl and homopiperazin-4-yl. The term "indolinyl" as used herein includes indolin-1-yl, indolin-2-yl, indolin-3-yl, indolin-4-yl, indolin-5-yl, indolin-6-yl, and indolin-7-yl. The term "quinolizinyl" as used herein includes quinolizidin-1-yl, quinolizidin-2-yl, quinolizidin-3-yl and quinolizidin-4-yl. The term "isoindolinyl" as used herein includes isoindolin-1-yl, isoindolin-2-yl, isoindolin-3-yl, isoindolin-4-yl, isoindolin-5-yl, isoindolin-6-yl, and isoindolin-7-yl. The term "3H-indolyl" as used herein includes 3H-indol-2-yl, 3H-indol-3-yl, 3H-indol-4-yl, 3H-indol-5-yl, 3H-indol-6-yl, and 3H-indol-7-yl. The term "quinolizinyl" as used herein includes quinolizidin-1-yl, quinolizidin-2-yl, quinolizidin-3-yl and quinolizidin-4-yl. The term "quinolizinyl" as used herein includes quinolizidin-1-yl, quinolizidin-2-yl, quinolizidin-3-yl and quinolizidin-4-yl. The term "tetrahydroquinolinyl" as used herein includes tetrahydroquinolin-1-yl, tetrahydroquinolin-2-yl, tetrahydroquinolin-3-yl, tetrahydroquinolin-4-yl, tetrahydroquinolin-5-yl, tetrahydroquinolin-6-yl, tetrahydroquinolin-7-yl and tetrahydroquinolin-8-yl. The term "tetrahydroisoquinolinyl" as used herein includes tetrahydroisoquinolin-1-yl, tetrahydroisoquinolin-2-yl, tetrahydroisoquinolin-3-yl, tetrahydroisoquinolin-4-yl, tetrahydroisoquinolin-5-yl, tetrahydroisoquinolin-6-yl, tetrahydroisoquinolin-7-yl and tetrahydroisoquinolin-8-yl. The term "1H-pyrrolizine" as used herein includes 1H-pyrrolizin-1-yl, 1H-pyrrolizin-2-yl, 1H-pyrrolizin-3-yl, 1H-pyrrolizin-5-yl, 1H-pyrrolizin-6-yl and 1H-pyrrolizin-7-yl. The term "3H-pyrrolizine" as used herein includes 3H-pyrrolizin-1-yl, 3H-pyrrolizin-2-yl, 3H-pyrrolizin-3-yl, 3H-pyrrolizin-5-yl, 3H-pyrrolizin-6-yl and 3H-pyrrolizin-7-yl.

The term "heterocyclyloxy", as a group or part of a group, refers to a group having the formula -O-$R^i$ wherein $R^i$ is heterocyclyl as defined herein above.

The term "heterocyclylalkyl", as a group or part of a group, means a alkyl as defined herein, wherein at least one hydrogen atom is replaced by at least one heterocyclyl as defined herein.

The term "heteroaryl" as a group or part of a group, refers but is not limited to 5 to 12 carbon-atom aromatic rings or ring systems containing 1 or 2 rings which can be fused together or linked covalently, typically containing 5 to 6 atoms; at least one of which is aromatic in which one or more carbon atoms in one or more of these rings can be replaced by N, O and/or S atoms where the N and S heteroatoms may optionally be oxidized and the N heteroatoms may optionally be quaternized, and wherein at least one carbon atom of said heteroaryl can be oxidized to form at least one C=O. Such rings may be fused to an aryl, cycloalkyl, heteroaryl or heterocyclyl ring. Non-limiting examples of such heteroaryl, include: pyrrolyl, furanyl, thiophenyl, pyrazolyl, imidazolyl, oxazolyl, isoxazolyl, thiazolyl, isothiazolyl, triazolyl, oxadiazolyl, thiadiazolyl, tetrazolyl, oxatriazolyl, thiatriazolyl, pyridinyl, pyrimidyl, pyrazinyl, pyridazinyl, oxazinyl, dioxinyl, thiazinyl, triazinyl, imidazo[2,1-b][1,3]thiazolyl, thieno[3,2-b]furanyl, thieno[3,2-b]thiophenyl, thieno[2,3-d][1,3]thiazolyl, thieno[2,3-d]imidazolyl, tetrazolo[1,5-a]pyridinyl, indolyl, indolizinyl, isoindolyl, benzofuranyl, isobenzofuranyl, benzothiophenyl, isobenzothiophenyl, indazolyl, benzimidazolyl, 1,3-benzoxazolyl, 1,2-benzisoxazolyl, 2,1-benzisoxazolyl, 1,3-benzothiazolyl, 1,2-benzoisothiazolyl, 2,1-benzoisothiazolyl, benzotriazolyl, 1,2,3-benzoxadiazolyl, 2,1,3-benzoxadiazolyl, 1,2,3-benzothiadiazolyl, 2,1,3-benzothiadiazolyl, benzo[d]oxazol-2(3H)-one, 2,3-dihydro-benzofuranyl, thienopyridinyl, purinyl, imidazo[1,2-a]pyridinyl, 6-oxo-pyridazin-1(6H)-yl, 2-oxopyridin-1(2H)-yl, 6-oxo-pyridazin-1(6H)-yl, 2-oxopyridin-1(2H)-yl, 1,3-benzodioxolyl, quinolinyl, isoquinolinyl, cinnolinyl, quinazolinyl, quinoxalinyl; preferably said heteroaryl group is selected from the group consisting of pyridyl, 1,3-benzodioxolyl, benzo[d]oxazol-2(3H)-one, 2,3-dihydro-benzofuranyl, pyrazinyl, pyrazolyl, pyrrolyl, isoxazolyl, thiophenyl, imidazolyl, benzimidazolyl, pyrimidinyl, triazolyl and

thiazolyl.

The term "pyrrolyl" (also called azolyl) as used herein includes pyrrol-1-yl, pyrrol-2-yl and pyrrol-3-yl. The term "furanyl" (also called "furyl") as used herein includes furan-2-yl and furan-3-yl (also called furan-2-yl and furan-3-yl). The term "thiophenyl" (also called "thienyl") as used herein includes thiophen-2-yl and thiophen-3-yl (also called thien-2-yl and thien-3-yl). The term "pyrazolyl" (also called 1H-pyrazolyl and 1,2-diazolyl) as used herein includes pyrazol-1-yl, pyrazol-3-yl, pyrazol-4-yl and pyrazol-5-yl. The term "imidazolyl" as used herein includes imidazol-1-yl, imidazol-2-yl, imidazol-4-yl and imidazol-5-yl. The term "oxazolyl" (also called 1,3-oxazolyl) as used herein includes oxazol-2-yl, oxazol-4-yl and oxazol-5-yl. The term "isoxazolyl" (also called 1,2-oxazolyl), as used herein includes isoxazol-3-yl, isoxazol-4-yl, and isoxazol-5-yl. The term "thiazolyl" (also called 1,3-thiazolyl),as used herein includes thiazol-2-yl, thiazol-4-yl and thiazol-5-yl (also called 2-thiazolyl, 4-thiazolyl and 5-thiazolyl). The term "isothiazolyl" (also called 1,2-thiazolyl) as used herein includes isothiazol-3-yl, isothiazol-4-yl, and isothiazol-5-yl. The term "triazolyl" as used herein includes 1H-triazolyl and 4H-1,2,4-triazolyl, "1H-triazolyl" includes 1H-1,2,3-triazol-1-yl, 1H-1,2,3-triazol-4-yl, 1H-1,2,3-triazol-5-yl, 1H-1,2,4-triazol-1-yl, 1H-1,2,4-triazol-3-yl and 1H-1,2,4-triazol-5-yl. "4H-1,2,4-triazolyl" includes 4H-1,2,4-triazol-4-yl, and 4H-1,2,4-triazol-3-yl. The term "oxadiazolyl" as used herein includes 1,2,3-oxadiazol-4-yl, 1,2,3-oxadiazol-5-yl, 1,2,4-oxadiazol-3-yl, 1,2,4-oxadiazol-5-yl, 1,2,5-oxadiazol-3-yl and 1,3,4-oxadiazol-2-yl. The term "thiadiazolyl" as used herein includes 1,2,3-thiadiazol-4-yl, 1,2,3-thiadiazol-5-yl, 1,2,4-thiadiazol-3-yl, 1,2,4-thiadiazol-5-yl, 1,2,5-thiadiazol-3-yl (also called furazan-3-yl) and 1,3,4-thiadiazol-2-yl. The term "tetrazolyl" as used herein includes 1H-tetrazol-1-yl, 1H-tetrazol-5-yl, 2H-tetrazol-2-yl, and 2H-tetrazol-5-yl. The term "oxatriazolyl" as used herein includes 1,2,3,4-oxatriazol-5-yl and 1,2,3,5-oxatriazol-4-yl. The term "thiatriazolyl" as used herein includes 1,2,3,4-thiatriazol-5-yl and 1,2,3,5-thiatriazol-4-yl. The term "pyridinyl" (also called "pyridyl") as used herein includes pyridin-2-yl, pyridin-3-yl and pyridin-4-yl (also called 2-pyridyl, 3-pyridyl and 4-pyridyl). The term "pyrimidyl" as used herein includes pyrimid-2-yl, pyrimid-4-yl, pyrimid-5-yl and pyrimid-6-yl. The term "pyrazinyl" as used herein includes pyrazin-2-yl and pyrazin-3-yl. The term "pyridazinyl as used herein includes pyridazin-3-yl and pyridazin-4-yl. The term "oxazinyl" (also called "1,4-oxazinyl") as used herein includes 1,4-oxazin-4-yl and 1,4-oxazin-5-yl. The term "dioxinyl" (also called "1,4-dioxinyl") as used herein includes 1,4-dioxin-2-yl and 1,4-dioxin-3-yl. The term "thiazinyl" (also called "1,4-thiazinyl") as used herein includes 1,4-thiazin-2-yl, 1,4-thiazin-3-yl, 1,4-thiazin-4-yl, 1,4-thiazin-5-yl and 1,4-thiazin-6-yl. The term "triazinyl" as used herein includes 1,3,5-triazin-2-yl, 1,2,4-triazin-3-yl, 1,2,4-triazin-5-yl, 1,2,4-triazin-6-yl, 1,2,3-triazin-4-yl and 1,2,3-triazin-5-yl. The term "imidazo[2,1-b][1,3]thiazolyl" as used herein includes imidazo[2,1-b][1,3]thiazoi-2-yl, imidazo[2,1-b][1,3]thiazol-3-yl, imidazo[2,1-b][1,3]thiazol-5-yl and imidazo[2,1-b][1,3]thiazol-6-yl. The term "thieno[3,2-b]furanyl" as used herein includes thieno[3,2-b]furan-2-yl, thieno[3,2-b]furan-3-yl, thieno[3,2-b]furan-4-yl, and thieno[3,2-b]furan-5-yl. The term "thieno[3,2-b]thiophenyl" as used herein includes thieno[3,2-b]thien-2-yl, thieno[3,2-b]thien-3-yl, thieno[3,2-b]thien-5-yl and thieno[3,2-b]thien-6-yl. The term "thieno[2,3-d][1,3]thiazolyl" as used herein includes thieno[2,3-d][1,3]thiazol-2-yl, thieno[2,3-d][1,3]thiazol-5-yl and thieno[2,3-d][1,3]thiazol-6-yl. The term "thieno[2,3-d]imidazolyl" as used herein includes thieno[2,3-d]imidazol-2-yl, thieno[2,3-d]imidazol-4-yl and thieno[2,3-d]imidazol-5-yl. The term "tetrazolo[1,5-a]pyridinyl" as used herein includes tetrazolo[1,5-a]pyridine-5-yl, tetrazolo[1,5-a]pyridine-6-yl, tetrazolo[1,5-a]pyridine-7-yl, and tetrazolo[1,5-a]pyridine-8-yl. The term "indolyl" as used herein includes indol-1-yl, indol-2-yl, indol-3-yl,-indol-4-yl, indol-5-yl, indol-6-yl and indol-7-yl. The term "indolizinyl" as used herein includes indolizin-1-yl, indolizin-2-yl, indolizin-3-yl, indolizin-5-yl, indolizin-6-yl, indolizin-7-yl, and indolizin-8-yl. The term "isoindolyl" as used herein includes isoindol-1-yl, isoindol-2-yl, isoindol-3-yl, isoindol-4-yl, isoindol-5-yl, isoindol-6-yl and isoindol-7-yl. The term "benzofuranyl" (also called benzo[b]furanyl) as used herein includes benzofuran-2-yl, benzofuran-3-yl, benzofuran-4-yl, benzofuran-5-yl, benzofuran-6-yl and benzofuran-7-yl. The term "isobenzofuranyl" (also called benzo[c]furanyl) as used herein includes isobenzofuran-1-yl, isobenzofuran-3-yl, isobenzofuran-4-yl, isobenzofuran-5-yl, isobenzofuran-6-yl and isobenzofuran-7-yl. The term "benzothiophenyl" (also called benzo[b]thienyl) as used herein includes 2-benzo[b]thiophenyl, 3-benzo[b]thiophenyl, 4-benzo[b]thiophenyl, 5-benzo[b]thiophenyl, 6-benzo[b]thiophenyl and -7-benzo[b]thiophenyl (also called benzothien-2-yl, benzothien-3-yl, benzothien-4-yl, benzothien-5-yl, benzothien-6-yl and benzothien-7-yl). The term "isobenzothiophenyl" (also called benzo[c]thienyl) as used herein includes isobenzothien-1-yl, isobenzothien-3-yl, isobenzothien-4-yl, isobenzothien-5-yl, isobenzothien-6-yl and isobenzothien-7-yl. The term "indazolyl" (also called 1H-indazolyl or 2-azaindolyl) as used herein includes 1H-indazol-1-yl, 1H-indazol-3-yl, 1H-indazol-4-yl, 1H-indazol-5-yl, 1H-indazol-6-yl, 1H-indazol-7-yl, 2H-indazol-2-yl, 2H-indazol-3-yl, 2H-indazol-4-yl, 2H-indazol-5-yl, 2H-indazol-6-yl, and 2H-indazol-7-yl. The term "benzimidazolyl" as used herein includes benzimidazol-1-yl, benzimidazol-2-yl, benzimidazol-4-yl, benzimidazol-5-yl, benzimidazol-6-yl and benzimidazol-7-yl. The term "1,3-benzoxazolyl" as used herein includes 1,3-benzoxazol-2-yl, 1,3-benzoxazol-4-yl, 1,3-benzoxazol-5-yl, 1,3-benzoxazol-6-yl and 1,3-benzoxazol-7-yl. The term "1,2-benzisoxazolyl" as used herein includes 1,2-benzisoxazol-3-yl, 1,2-benzisoxazol-4-yl, 1,2-benzisoxazol-5-yl, 1,2-benzisoxazol-6-yl and 1,2-benzisoxazol-7-yl. The term "2,1-benzisoxazolyl" as used herein includes 2,1-benzisoxazol-3-yl, 2,1-benzisoxazol-4-yl, 2,1-benzisoxazol-5-yl, 2,1-benzisoxazol-6-yl and 2,1-benzisoxazol-7-yl. The term "1,3-benzothiazolyl" as used herein includes 1,3-benzothiazol-2-yl, 1,3-benzothiazol-4-yl, 1,3-benzothiazol-5-yl, 1,3-benzothiazol-6-yl and 1,3-benzothiazol-7-yl. The term "1,2-benzoisothiazolyl" as used herein includes 1,2-benzisothiazol-3-yl, 1,2-benzisothiazol-4-yl, 1,2-benzisothiazol-5-yl, 1,2-benzisothiazol-6-yl and 1,2-benzisothiazol-7-yl. The term

"2,1-benzoisothiazolyl" as used herein includes 2,1-benzisothiazol-3-yl, 2,1-benzisothiazol-4-yl, 2,1-benzisothiazol-5-yl, 2,1-benzisothiazol-6-yl and 2,1-benzisothiazol-7-yl. The term "benzotriazolyl" as used herein includes benzotriazol-1-yl, benzotriazol-4-yl, benzotriazol-5-yl, benzotriazol-6-yl and benzotriazol-7-yl. The term "1,2,3-benzoxadiazolyl" as used herein includes 1,2,3-benzoxadiazol-4-yl, 1,2,3-benzoxadiazol-5-yl, 1,2,3-benzoxadiazol-6-yl and 1,2,3-benzoxadiazol-7-yl. The term "2,1,3-benzoxadiazolyl" as used herein includes 2,1,3-benzoxadiazol-4-yl, 2,1,3-benzoxadiazol-5-yl, 2,1,3-benzoxadiazol-6-yl and 2,1,3-benzoxadiazol-7-yl. The term "1,2,3-benzothiadiazolyl" as used herein includes 1,2,3-benzothiadiazol-4-yl, 1,2,3-benzothiadiazol-5-yl, 1,2,3-benzothiadiazol-6-yl and 1,2,3-benzothiadiazol-7-yl. The term "2,1,3-benzothiadiazolyl" as used herein includes 2,1,3-benzothiadiazol-4-yl, 2,1,3-benzothiadiazol-5-yl, 2,1,3-benzothiadiazol-6-yl and 2,1,3-benzothiadiazol-7-yl. The term "thienopyridinyl" as used herein includes thieno[2,3-b]pyridinyl, thieno[2,3-c]pyridinyl, thieno[3,2-c]pyridinyl and thieno[3,2-b]pyridinyl. The term "purinyl" as used herein includes purin-2-yl, purin-6-yl, purin-7-yl and purin-8-yl. The term "imidazo[1,2-a]pyridinyl", as used herein includes imidazo[1,2-a]pyridin-2-yl, imidazo[1,2-a]pyridin-3-yl, imidazo[1,2-a]pyridin-4-yl, imidazo[1,2-a]pyridin-5-yl, imidazo[1,2-a]pyridin-6-yl and imidazo[1,2-a]pyridin-7-yl. The term "1,3-benzodioxolyl", as used herein includes 1,3-benzodioxol-4-yl, 1,3-benzodioxol-5-yl, 1,3-benzodioxol-6-yl, and 1,3-benzodioxol-7-yl. The term "quinolinyl" as used herein includes quinolin-2-yl, quinolin-3-yl, quinolin-4-yl, quinolin-5-yl, quinolin-6-yl, quinolin-7-yl and quinolin-8-yl. The term "isoquinolinyl" as used herein includes isoquinolin-1-yl, isoquinolin-3-yl, isoquinolin-4-yl, isoquinolin-5-yl, isoquinolin-6-yl, isoquinolin-7-yl and isoquinolin-8-yl. The term "cinnolinyl" as used herein includes cinnolin-3-yl, cinnolin-4-yl, cinnolin-5-yl, cinnolin-6-yl, cinnolin-7-yl and cinnolin-8-yl. The term "quinazolinyl" as used herein includes quinazolin-2-yl, quinazolin-4-yl, quinazolin-5-yl, quinazolin-6-yl, quinazolin-7-yl and quinazolin-8-yl. The term "quinoxalinyl" as used herein includes quinoxalin-2-yl, quinoxalin-5-yl, and quinoxalin-6-yl.

The term "heteroaryloxy", as a group or part of a group, refers to a group having the formula -O-$R^k$ wherein $R^k$ is heteroaryl as defined herein above.

The term "heteroarylalkyl", as a group or part of a group, means a alkyl as defined herein, wherein at least one hydrogen atom is replaced by at least one heteroaryl as defined herein.

The term "mono- or di-alkylamino", as a group or part of a group, refers to a group of formula -N($R^o$)($R^p$) wherein $R^o$ and $R^p$ are each independently selected from hydrogen, or alkyl, wherein at least one of $R^o$ or $R^p$ is alkyl. Thus, alkylamino include mono-alkyl amino group (e.g. mono-$C_{1-6}$alkylamino group such as methylamino and ethylamino), and di-alkylamino group (e.g. di-$C_{1-6}$alkylamino group such as dimethylamino and diethylamino). Non-limiting examples of suitable mono- or di-alkylamino groups include *n*-propylamino, isopropylamino, *n*-butylamino, *i*-butylamino, *sec*-butylamino, *t*-butylamino, pentylamino, *n*-hexylamino, di-*n*-propylamino, di-*i*-propylamino, ethylmethylamino, methyl-*n*-propylamino, methyl-*i*-propylamino, *n*-butylmethylamino, *i*-butylmethylamino, *t*-butylmethylamino, ethyl-*n*-propylamino, ethyl-*i*-propylamino, *n*-butylethylamino, i-butylethylamino, *t*-butylethylamino, di-*n*-butylamino, di-*i*-butylamino, methylpentylamino, methylhexylamino, ethylpentylamino, ethylhexylamino, propylpentylamino, propylhexylamino, and the like.

The term "mono- or di-arylamino", as a group or part of a group, refers to a group of formula -N($R^q$)($R^r$) wherein $R^q$ and $R^r$ are each independently selected from hydrogen, or aryl, wherein at least one of $R^q$ or $R^r$ is aryl.

The term "mono- or di-arylalkylamino", as a group or part of a group, refers to a group of formula -N($R^{q'}$)($R^{r'}$) wherein $R^{q'}$ and $R^{r'}$ are each independently selected from hydrogen, or arylalkyl, wherein at least one of $R^{q'}$ or $R^{r'}$ is arylalkyl.

The term "mono- or di-cycloalkylamino", as a group or part of a group, refers to a group of formula -N($R^s$)($R^t$) wherein $R^s$ and $R^t$ are each independently selected from hydrogen, or cycloalkyl, wherein at least one of $R^s$ or $R^t$ is cycloalkyl.

The term "mono- or di-heteroarylamino", as a group or part of a group, refers to a group of formula -N($R^u$)($R^v$) wherein $R^u$ and $R^v$ are each independently selected from hydrogen, or heteroaryl, wherein at least one of $R^u$ or $R^v$ is heteroaryl as defined herein.

The term "mono- or di-heterocyclylamino", as a group or part of a group, refers to a group of formula -N($R^w$)($R^x$) wherein $R^w$ and $R^x$ are each independently selected from hydrogen, or heterocyclyl, wherein at least one of $R^w$ or $R^x$ is heterocyclyl as defined herein.

The term "alkyloxycarbonyl", as a group or part of a group, refers to a group of formula -COO-$R^b$, wherein $R^b$ is alkyl as defined herein.

The term "cycloakyloxycarbonyl", as a group or part of a group, refers to a group of formula - COO-$R^b$, wherein $R^b$ is cycloalkyl as defined herein.

The term "aryloxycarbonyl", as a group or part of a group, refers to a group of formula -COO-$R^b$, wherein $R^b$ is aryl as defined herein.

The term "heterocyclyloxycarbonyl", as a group or part of a group, refers to a group of formula - COO-$R^b$, wherein $R^b$ is heterocyclyl as defined herein.

The term "heteroaryloxycarbonyl", as a group or part of a group, refers to a group of formula - COO-$R^b$, wherein $R^b$ is heteroaryl as defined herein.

The term "alkylsulfinyl", as a group or part of a group, refers to a group of formula -SO-$R^b$, wherein $R^b$ is alkyl as

defined herein.

The term "alkylsulfonyl", as a group or part of a group, refers to a group of formula $-S(O)_2-R^b$, wherein $R^b$ is alkyl as defined herein.

The term "cycloalkylsulfonyl", as a group or part of a group, refers to a group of formula - $S(O)_2-R^b$, wherein $R^b$ is cycloalkyl as defined herein.

The term "arylsulfonyl", as a group or part of a group, refers to a group of formula $-S(O)_2-R^b$, wherein $R^b$ is aryl as defined herein.

The term "heterocyclylsulfonyl", as a group or part of a group, refers to a group of formula - $S(O)_2-R^b$, wherein $R^b$ is heterocyclyl as defined herein.

The term "heteroarylsulfonyl", as a group or part of a group, refers to a group of formula - $S(O)_2-R^b$, wherein $R^b$ is heteroaryl as defined herein.

The term "mono- or di-alkylaminosulfonyl", as a group or part of a group, refers to a group of formula $-S(O)_2-NNR^oR^p$, wherein $R^oR^p$ are each independently selected from hydrogen, or alkyl, wherein at least one of R° or $R^p$ is alkyl.

The term "mono- or di-cycloalkylaminosulfonyl", as a group or part of a group, refers to a group of formula $-S(O)_2-NNR^oR^p$, wherein $R^oR^p$ are each independently selected from hydrogen, or alkyl, wherein at least one of R° or $R^p$ is cycloalkyl.

The term "mono- or di-arylaminosulfonyl", as a group or part of a group, refers to a group of formula $-S(O)_2-NNR^oR^p$, wherein $R^oR^p$ are each independently selected from hydrogen, or alkyl, wherein at least one of R° or $R^p$ is aryl.

The term "mono- or di-heterocyclylaminosulfonyl", as a group or part of a group, refers to a group of formula $-S(O)_2-NNR^oR^p$, wherein $R^oR^p$ are each independently selected from hydrogen, or alkyl, wherein at least one of R° or $R^p$ is heterocyclyl.

The term "mono- or di-heteroarylaminosulfonyl", as a group or part of a group, refers to a group of formula $-S(O)_2-NNR^oR^p$, wherein $R^oR^p$ are each independently selected from hydrogen, or alkyl, wherein at least one of R° or $R^p$ is heteroaryl.

The term "mono- or dialkylaminocarbonyl", as a group or part of a group, refers to a group of formula $-CONR^oR^p$ wherein $R^oR^p$ are each independently selected from hydrogen, or alkyl, wherein at least one of R° or $R^p$ is alkyl.

The term "mono- or dicycloalkylaminocarbonyl", as a group or part of a group, refers to a group of formula $-CONR^oR^p$ wherein $R^oR^p$ are each independently selected from hydrogen, or cycloalkyl, wherein at least one of R° or $R^p$ is cycloalkyl.

The term "alkylcarbonyl", as a group or part of a group, refers to a group of formula $-CO-R^b$, wherein $R^b$ is alkyl as defined herein.

The term "cycloalkylcarbonyl", as a group or part of a group, refers to a group of formula $-CO-R^b$, wherein $R^b$ is cycloalkyl as defined herein.

The term "arylcarbonyl", as a group or part of a group, refers to a group of formula $-CO-R^b$, wherein $R^b$ is aryl as defined herein.

The term "heterocyclylcarbonyl", as a group or part of a group, refers to a group of formula - $CO-R^b$, wherein $R^b$ is heterocyclyl as defined herein.

The term "heteroarylcarbonyl", as a group or part of a group, refers to a group of formula $-CO-R^b$, wherein $R^b$ is heteroaryl as defined herein.

The term "alkylcarbonylamino", as a group or part of a group, refers to a group of formula $-NR^o-CO-R^b$, wherein R ° is selected from hydrogen, or alkyl and $R^b$ is alkyl as defined herein.

The term "alkylsulfonylamino", as a group or part of a group, refers to a group of formula $-NR^o-S(O)_2-R^b$, wherein R° is selected from hydrogen, or alkyl and $R^b$ is alkyl as defined herein.

Whenever used in the present invention the term "compounds of the invention" or a similar term is meant to include the compounds of general formula (I), as defined above, as well as (IA), (IB), (IC), (ID), (IE), (IF), (IG), (IH), (IJ) as detailed below and any subgroup thereof. This term also refers to the compounds as depicted in Table 1 and their derivatives, salts, solvates, hydrates, tautomeric forms, analogues, pro-drugs, esters and metabolites, as well as their quaternized nitrogen analogues.

As used herein and unless otherwise stated, the term "stereoisomer" refers to all possible different isomeric as well as conformational forms which the compounds of structural formula herein may possess, in particular all possible stereochemically and conformationally isomeric forms, all diastereomers, enantiomers and/or conformers of the basic molecular structure. Some compounds of the present invention may exist in different tautomeric forms, all of the latter being included within the scope of the present invention.

The present invention includes all possible stereoisomers compounds of formula (I) and any subgroup thereof. When a compound is desired as a single enantiomer, such may be obtained by stereospecific synthesis, by resolution of the final product or any convenient intermediate, or by chiral chromatographic methods as each are known in the art.

Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994), incorporated by reference with regard to stereochemistry. A structural isomer is a type of isomer in which molecules with the same molecular formula have different bonding patterns and atomic organization. Where structural isomers are interconvertible via a low energy barrier, tautomeric isomerism ('tautomerism') can occur. This can take the form of proton tautomerism in compounds of the invention containing, for example, an imino, keto, or oxime group, or so-called valence tautomerism in compounds which contain an aromatic moiety.

The term "prodrug" as used herein means the pharmacologically acceptable derivatives such as esters, amides and phosphates, such that the resulting *in vivo* biotransformation product of the derivative is the active drug. The reference by Goodman and Gilman (The Pharmacological Basis of Therapeutics, 8th Ed, McGraw-Hill, Int. Ed. 1992, "Biotransformation of Drugs", p 13-15) describing pro-drugs generally is hereby incorporated. Prodrugs of the compounds of the invention can be prepared by modifying functional groups present in said component in such a way that the modifications are cleaved, either in routine manipulation or in vivo, to the parent component. Typical examples of prodrugs are described for instance in WO 99/33795, WO 99/33815, WO 99/33793 and WO 99/33792 all incorporated herein by reference. Prodrugs are characterized by increased bio-availability and are readily metabolized into the active inhibitors *in vivo.* The term "prodrug", as used herein, means any compound that will be modified to form a drug species, wherein the modification may take place either inside or outside of the body, and either before or after the pre-drug reaches the area of the body where administration of the drug is indicated.

Preferred statements (features) and embodiments of the compounds and processes of this invention are now set forth. Each statement and embodiment of the invention so defined may be combined with any other statement and/or embodiments unless clearly indicated to the contrary. In particular, any feature indicated as being preferred or advantageous may be combined with any other feature or features indicated as being preferred or advantageous.

1. A compound of formula (I) or a stereoisomer, or tautomer thereof,

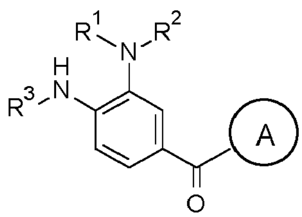

(I)

wherein, cycle A is selected from:

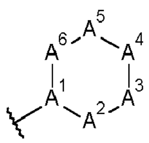

or

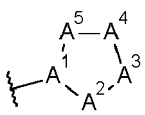

;

wherein the wavy line

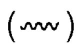

indicates the point of attachment of cycle A to the rest of the molecule,

$A^1$ is selected from N or $CR^4$;

$A^2$ is selected from the group consisting of $NR^5$, $CR^6R^7$, S, $SO_2$ and O;

$A^3$ is selected from the group consisting of $NR^5$, $CR^6R^7$, S, $SO_2$ and O;

$A^4$ is selected from the group consisting of $NR^5$, $CR^6R^7$, S, $SO_2$ and O;

$A^5$ is selected from the group consisting of $NR^5$, $CR^6R^7$, S, $SO_2$ and O;

$A^6$ is selected from the group consisting of $NR^5$, $CR^6R^7$, S, $SO_2$ and O;

wherein when $A^1$ is $CR^4$, then at least one of $A^2$, $A^3$, $A^4$, $A^5$, or $A^6$ is selected from the group consisting of $NR^5$, $CR^6R^7$, S, $SO_2$ and O;

$R^1$ is selected from the group consisting of hydrogen, $C_{3-10}$cycloalkyl, $C_{6-12}$aryl$C_{1-6}$alkyl, $C_{3-10}$cycloalkenyl,

$C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^1$;

$R^2$ is selected from the group consisting of $C_{3-10}$cycloalkyl, $C_{6-12}$aryl$C_{1-6}$alkyl, hydrogen, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^2$;

$R^3$ is selected from the group consisting $C_{3-10}$cycloalkyl, $C_{3-10}$cycloalkenyl, $C_{3-10}$cycloalkynyl, $C_{6-12}$aryl, heterocyclyl, and heteroaryl, wherein said $C_{3-10}$cycloalkyl, $C_{3-10}$cycloalkenyl, $C_{3-10}$cycloalkynyl, $C_{6-12}$aryl, heterocyclyl or heteroaryl can be unsubstituted or substituted with one or more $Z^3$;

$R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$, wherein said $C_{1-6}$alkyl, $C_{1-10}$cycloalkyl, $C_{6-12}$aryl, $C_{6-12}$aryl$C_{1-6}$alkyl, heterocyclyl, or heteroaryl can be unsubstituted or substituted with one or more Z4 ;

each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl, or heteroaryl can be unsubstituted or substituted with one or more $Z^5$;

each $R^6$ is independently selected from the group consisting of hydrogen, $C_{1-6}$alkyl, halogen, hydroxy, halo-$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-NR^8R^9$, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl or heteroaryl can be unsubstituted or substituted with one or more $Z^6$;

each $R^7$ is independently selected from the group consisting of hydrogen, $C_{1-6}$alkyl, halogen, hydroxy, halo-$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-NR^8R^9$, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl or heteroaryl can be unsubstituted or substituted with one or more $Z^7$;

each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;

each $R^{10}$ is independently selected from the group consisting of $C_{1-6}$alkyl, hydrogen, $C_{3-10}$cycloalkyl, $C_{6-12}$aryl, $C_{6-12}$aryl$C_{1-6}$alkyl, heterocyclyl, and heteroaryl;

each $Z^1$, $Z^2$, $Z^3$, $Z^4$, $Z^5$, $Z^6$ and $Z^7$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-C(O)R^{10}$, $-S(O)R^{10}$, $-S(O)_2R^{10}$, $-S(O)_2NR^8R^9$, nitro;

or a solvate, hydrate, pharmaceutically acceptable salt, or prodrug thereof;

wherein when the moiety $-NR^1R^2$ is

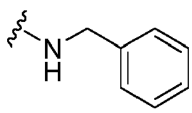,

cycle A is piperazinyl and $R^3$ is cyclohexyl, then $R^5$ is not substituted benzyl, 4-methylpyridine or methyl.

2. The compound according to statement 1, having structural formula (IA),

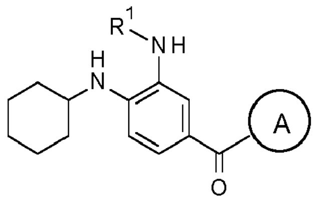

(IA)

wherein $R^1$ and cycle A have the same meaning as that defined in statement 1.

3. The compound according to statements 1 or 2, having structural formula (IB), or (IC),

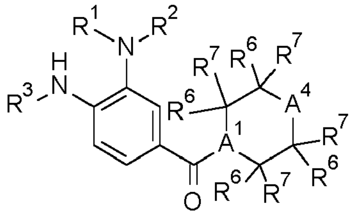
(IB)

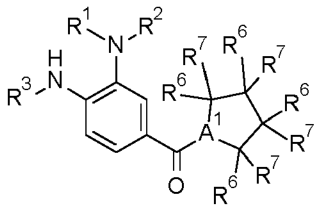
(IC)

wherein $A^1$, $A^4$, $R^1$, $R^2$, $R^3$, $R^6$, $R^7$ and $R^8$ have the same meaning as that defined in statement 1.

4. The compound according to any one of the previous statements, having structural formula (IE), or (IF),

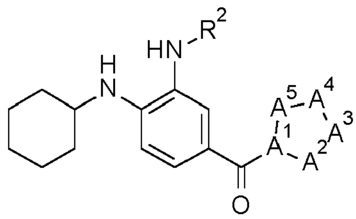
(ID)

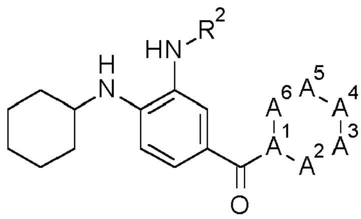
(IE)

wherein $A^1$, $A^2$, $A^3$, $A^4$, $A^5$, $A^6$, and $R^2$ have the same meaning as that defined in statement 1.

5. The compound according to any one of the previous statements, having structural formula (IF), or (IG),

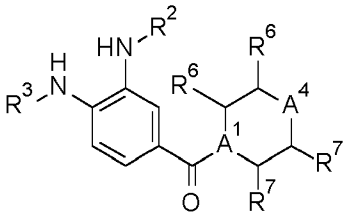
(IF)

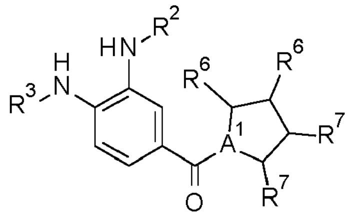
(IG)

wherein $A^1$, $A^4$, $R^2$, $R^3$, $R^6$, and $R^7$ have the same meaning as that defined in statement 1.

6. The compound according to any one of the previous statements, having structural formula (IH), or (IJ),

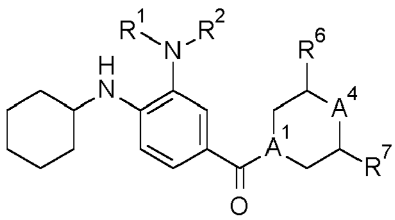
(IH)

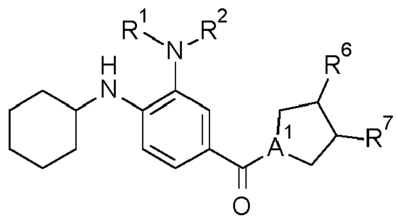
(IJ)

wherein $A^1$, $A^4$, $R^1$, $R^2$, $R^6$ and $R^7$ have the same meaning as that defined in statement 1.

7. The compound according to any one of the previous statements, wherein $A^1$ is N.

8. The compound according to any one of the previous statements, wherein $A^2$ is selected from the group consisting of $NR^5$, $CR^6R^7$, and S.

9. The compound according to any one of the previous statements, wherein $A^2$ is selected from the group consisting of $NR^5$, $CR^6R^7$, and S.

10. The compound according to any one of the previous statements, wherein $A^3$ is selected from the group consisting of $NR^5$, $CR^6R^7$, and S.

11. The compound according to any one of the previous statements, wherein $A^4$ is selected from the group consisting of $NR^5$, $CR^6R^7$, and S.

12. The compound according to any one of the previous statements, wherein $A^5$ is selected from the group consisting of $NR^5$, $CR^6R^7$, and S.

13. The compound according to any one of the previous statements, wherein $A^6$ is selected from the group consisting of $NR^5$, $CR^6R^7$, and S.

14. The compound according to any one of the previous statements, wherein $R^1$ is selected from the group consisting of hydrogen, $C_{3-9}$cycloalkyl, $C_{6-12}$aryl$C_{1-6}$alkyl, heterocyclyl, heterocyclyl$C_{1-6}$alkyl, and heteroaryl$C_{1-6}$alkyl, wherein said $C_{3-9}$cycloalkyl, aryl$C_{1-6}$alkyl, aryl, heterocyclyl, heteroaryl, heterocyclyl$C_{1-6}$alkyl, or heteroaryl$C_{1-6}$alkyl can be unsubstituted or substituted with one two or three $Z^1$.
15. The compound according to any one of the previous statements, wherein $R^1$ is selected from the group consisting of hydrogen, $C_{3-8}$cycloalkyl, $C_{6-12}$aryl$C_{1-4}$alkyl, heterocyclyl, heterocyclyl$C_{1-6}$alkyl, and heteroaryl$C_{1-4}$alkyl, wherein said $C_{3-8}$cycloalkyl, aryl$C_{1-4}$alkyl, aryl, heterocyclyl, heteroaryl, heterocyclyl$C_{1-4}$alkyl, or heteroaryl$C_{1-4}$alkyl can be unsubstituted or substituted with one two or three $Z^1$.
16. The compound according to any one of the previous statements, wherein $R^2$ is selected from the group consisting of hydrogen, $C_{3-9}$cycloalkyl, $C_{6-12}$aryl$C_{1-6}$alkyl, heterocyclyl, heterocyclyl$C_{1-6}$alkyl, and heteroaryl$C_{1-6}$alkyl, wherein said $C_{3-9}$cycloalkyl, aryl$C_{1-6}$alkyl, aryl, heterocyclyl, heteroaryl, heterocyclyl$C_{1-6}$alkyl, or heteroaryl$C_{1-6}$alkyl can be unsubstituted or substituted with one two or three $Z^2$.
17. The compound according to any one of the previous statements, wherein $R^2$ is selected from the group consisting of hydrogen, $C_{3-8}$cycloalkyl, $C_{6-12}$aryl$C_{1-4}$alkyl, heterocyclyl, heterocyclyl$C_{1-6}$alkyl, and heteroaryl$C_{1-4}$alkyl, wherein said $C_{3-8}$cycloalkyl, aryl$C_{1-4}$alkyl, aryl, heterocyclyl, heteroaryl, heterocyclyl$C_{1-4}$alkyl, or heteroaryl$C_{1-4}$alkyl can be unsubstituted or substituted with one two or three $Z^2$.
18. The compound according to any one of the previous statements, wherein $R^3$ is selected from the group consisting of $C_{3-9}$cycloalkyl, $C_{3-9}$cycloalkenyl, $C_{3-9}$cycloalkynyl, $C_{6-12}$aryl, heterocyclyl, and heteroaryl, wherein said $C_{3-9}$cycloalkyl, $C_{3-9}$cycloalkenyl, $C_{3-9}$cycloalkynyl, $C_{6-12}$aryl, heterocyclyl or heteroaryl can be unsubstituted or substituted with one, two or three Z3.
19. The compound according to any one of the previous statements, wherein $R^3$ is selected from the group consisting of Ca-scycloalkyl, Ca-scycloalkenyl, Ca-scycloalkynyl, or heterocyclyl, wherein said Ca-scycloalkyl, Ca-scycloalkenyl, Ca-scycloalkynyl, or heterocyclyl can be unsubstituted or substituted with one, two or three $Z^3$.
20. The compound according to any one of the previous statements, wherein $R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{3-10}$cycloakyloxycarbonyl, $C_{6-12}$aryloxycarbonyl, mono-$C_{1-4}$akylaminocarbonyl, mono-$C_{3-10}$cycloakylaminocarbonyl, di-$C_{1-4}$akylamino, di-$C_{3-10}$cycloakylamino, di-$C_{6-12}$arylamino, $C_{1-4}$akylcarbonyl, $C_{3-10}$cycloakylcarbonyl, $C_{6-12}$arylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl$C_{1-6}$alkyl, heterocyclyl, or heteroaryl can be unsubstituted or substituted with one or more $Z^4$.
21. The compound according to any one of the previous statements, wherein $R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{1-4}$akylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^4$.
22. The compound according to any one of the previous statements, wherein $R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^4$.
23. The compound according to any one of the previous statements, wherein $R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, cyano, wherein said $C_{1-6}$alkyl, $C_{1-10}$cycloalkyl, $C_{6-12}$aryl can be unsubstituted or substituted with one, two or three $Z^4$.
24. The compound according to any one of the previous statements, wherein each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{3-10}$cycloakyloxycarbonyl, $C_{6-12}$aryloxycarbonyl, mono-$C_{1-4}$akylaminocarbonyl, mono-$C_{3-10}$cycloakylaminocarbonyl, di-$C_{1-4}$akylamino, di-$C_{3-10}$cycloakylamino, di-$C_{6-12}$arylamino, $C_{1-4}$akylcarbonyl, $C_{3-10}$cycloakylcarbonyl, $C_{6-12}$arylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl$C_{1-6}$alkyl, heterocyclyl, or heteroaryl can be unsubstituted or substituted with one or more $Z^5$.
25. The compound according to any one of the previous statements, wherein each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{1-4}$akylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^5$.
26. The compound according to any one of the previous statements, wherein each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^5$.

27. The compound according to any one of the previous statements, wherein each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, cyano, wherein said $C_{1-6}$alkyl, $C_{1-10}$cycloalkyl, $C_{6-12}$aryl can be unsubstituted or substituted with one, two or three $Z^5$.

28. The compound according to any one of the previous statements, wherein each $R^6$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{3-10}$cycloakyloxycarbonyl, $C_{6-12}$aryloxycarbonyl, mono-$C_{1-4}$akylaminocarbonyl, mono-$C_{3-10}$cycloakylaminocarbonyl, di-$C_{1-4}$akylamino, di-$C_{3-10}$cycloakylamino, di-$C_{6-12}$arylamino, $C_{1-4}$akylcarbonyl, $C_{3-10}$cycloakylcarbonyl, $C_{6-12}$arylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl$C_{1-6}$alkyl, heterocyclyl, or heteroaryl can be unsubstituted or substituted with one or more $Z^6$.

29. The compound according to any one of the previous statements, wherein each $R^6$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{1-4}$akylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^6$.

30. The compound according to any one of the previous statements, wherein each $R^6$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^6$.

31. The compound according to any one of the previous statements, wherein each $R^6$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, cyano, wherein said $C_{1-6}$alkyl, $C_{1-10}$cycloalkyl, $C_{6-12}$aryl can be unsubstituted or substituted with one, two or three $Z^6$.

32. The compound according to any one of the previous statements, wherein each $R^7$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{3-10}$cycloakyloxycarbonyl, $C_{6-12}$aryloxycarbonyl, mono-$C_{1-4}$akylaminocarbonyl, mono-$C_{3-10}$cycloakylaminocarbonyl, di-$C_{1-4}$akylamino, di-$C_{3-10}$cycloakylamino, di-$C_{6-12}$arylamino, $C_{1-4}$akylcarbonyl, $C_{3-10}$cycloakylcarbonyl, $C_{6-12}$arylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl$C_{1-6}$alkyl, heterocyclyl, or heteroaryl can be unsubstituted or substituted with one or more $Z^7$.

33. The compound according to any one of the previous statements, wherein each $R^7$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, hydroxycarbonyl, $C_{1-4}$akyloxycarbonyl, $C_{1-4}$akylcarbonyl, -$S(O)_2H$ and $C_{1-4}$akylsulfonyl, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^7$.

34. The compound according to any one of the previous statements, wherein each $R^7$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, $C_{6-12}$aryl$C_{1-6}$alkyl, heterocyclyl, heteroaryl, cyano, wherein said $C_{1-6}$alkyl, $C_{1-10}$cycloalkyl, $C_{6-12}$aryl, $C_{6-12}$aryl $C_{1-6}$alkyl, heterocyclyl, or heteroaryl can be unsubstituted or substituted with one, two or three $Z^7$.

35. The compound according to any one of the previous statements, wherein each $R^7$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{1-10}$cycloalkyl, $C_{6-12}$aryl, cyano, wherein said $C_{1-6}$alkyl, $C_{1-10}$cycloalkyl, $C_{6-12}$aryl can be unsubstituted or substituted with one, two or three $Z^7$.

36. The compound according to any one of the previous statements, wherein each $R^8$ is independently selected from the group consisting of $C_{1-6}$alkyl, hydrogen, $C_{6-12}$aryl, and $C_{3-10}$cycloalkyl.

37. The compound according to any one of the previous statements, wherein each $R^9$ is independently selected from the group consisting of $C_{1-6}$alkyl, hydrogen, $C_{6-12}$aryl, and $C_{3-10}$cycloalkyl.

38. The compound according to any one of the previous statements, wherein each $R^{10}$ is independently selected from the group consisting of $C_{1-6}$alkyl, hydrogen, $C_{6-12}$aryl, and $C_{3-10}$cycloalkyl.

39. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - $S(O)H$, $C_{1-4}$akylsulfinyl, -$S(O)_2H$, $C_{1-4}$akylsulfonyl, -$SO_2NH_2$, mono-$C_{1-4}$akylaminosulfonyl, di-$C_{1-4}$akylamino-

sulfonyl nitro.

40. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -$S(O)_2$H, $C_{1-4}$akylsulfonyl, -$SO_2NH_2$, mono-$C_{1-4}$akylaminosulfonyl, nitro.

41. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -$S(O)_2$H, $C_{1-4}$akylsulfonyl, -$SO_2NH_2$, mono-$C_{1-4}$akylaminosulfonyl.

42. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-Ca-$_{10}$cycloakylaminocarbonyl, $C_{1-4}$akylcarbonyl.

43. The compound according to any one of the previous statements, wherein each $Z^1$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3}$-$_{10}$cycloakylaminocarbonyl, $C_{1-4}$akylcarbonyl.

44. The compound according to any one of the previous statements, wherein each $Z^1$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

45. The compound according to any one of the previous statements, wherein each $Z^1$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

46. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -$S(O)_2$H, $C_{1-4}$akylsulfonyl, -$SO_2NH_2$, mono-$C_{1-4}$akylaminosulfonyl, di-$C_{1-4}$akylaminosulfonyl nitro.

47. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -$S(O)_2$H, $C_{1-4}$akylsulfonyl, -$SO_2NH_2$, mono-$C_{1-4}$akylaminosulfonyl, nitro.

48. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -$S(O)_2$H, $C_{1-4}$akylsulfonyl, -$SO_2NH_2$, mono-$C_{1-4}$akylaminosulfonyl.

49. The compound according to any one of the previous statements, wherein each $Z^2$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

50. The compound according to any one of the previous statements, wherein each $Z^2$ is independently selected from

the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

51. The compound according to any one of the previous statements, wherein each $Z^2$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

52. The compound according to any one of the previous statements, wherein each $Z^2$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

53. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -S(O)$_2$H, $C_{1-4}$akylsulfonyl, -$SO_2NH_2$, mono-$C_{1-4}$akylaminosulfonyl, di-$C_{1-4}$akylaminosulfonyl nitro.

54. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -S(O)$_2$H, $C_{1-4}$akylsulfonyl, -$SO_2NH_2$, mono-$C_{1-4}$akylaminosulfonyl, nitro.

55. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -S(O)$_2$H, $C_{1-4}$akylsulfonyl, -$SO_2NH_2$, mono-$C_{1-4}$akylaminosulfonyl.

56. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

57. The compound according to any one of the previous statements, wherein each $Z^3$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

58. The compound according to any one of the previous statements, wherein each $Z^3$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

59. The compound according to any one of the previous statements, wherein each $Z^3$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

60. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -S(O)$_2$H, $C_{1-4}$akylsulfonyl, -$SO_2NH_2$, mono-$C_{1-4}$akylaminosulfonyl, di-$C_{1-4}$akylaminosulfonyl nitro.

61. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$aky-

loxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -$S(O)_2H$, $C_{1-4}$akylsulfonyl, -$SO_2NH_2$, mono-$C_{1-4}$akylaminosulfonyl, nitro.

62. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -$S(O)_2H$, $C_{1-4}$akylsulfonyl, -$SO_2NH_2$, mono-$C_{1-4}$akylaminosulfonyl.

63. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-Ca-$_{10}$cycloakylaminocarbonyl, $C_{1-4}$akylcarbonyl.

64. The compound according to any one of the previous statements, wherein each $Z^4$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-Ca-$_{10}$cycloakylaminocarbonyl, $C_{1-4}$akylcarbonyl.

65. The compound according to any one of the previous statements, wherein each $Z^4$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

66. The compound according to any one of the previous statements, wherein each $Z^4$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

67. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, -$S(O)_2H$, $C_{1-4}$akylsulfonyl, -$SO_2NH_2$, mono-$C_{1-4}$akylaminosulfonyl, di-$C_{1-4}$akylaminosulfonyl nitro.

68. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, -$S(O)_2H$, $C_{1-4}$akylsulfonyl, -$SO_2NH_2$, mono-$C_{1-4}$akylaminosulfonyl, nitro.

69. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -$S(O)_2H$, $C_{1-4}$akylsulfonyl, -$SO_2NH_2$, mono-$C_{1-4}$akylaminosulfonyl.

70. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-Ca-$_{10}$cycloakylaminocarbonyl, $C_{1-4}$akylcarbonyl.

71. The compound according to any one of the previous statements, wherein each $Z^5$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-Ca-$_{10}$cycloakylaminocarbonyl, $C_{1-4}$akylcarbonyl.

72. The compound according to any one of the previous statements, wherein each $Z^5$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl,

$C_{1-6}$alkyloxy, cyano, amino.

73. The compound according to any one of the previous statements, wherein each $Z^5$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

74. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, $-S(O)_2H$, $C_{1-4}$akylsulfonyl, $-SO_2NH_2$, mono-$C_{1-4}$akylaminosulfonyl, di-$C_{1-4}$akylaminosulfonyl nitro.

75. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl, nitro.

76. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl.

77. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

78. The compound according to any one of the previous statements, wherein each $Z^6$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

79. The compound according to any one of the previous statements, wherein each $Z^6$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

80. The compound according to any one of the previous statements, wherein each $Z^6$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

81. The compound according to any one of the previous statements, wherein each $Z^7$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, di-$C_{1-4}$akylamino, di-$C_{3-10}$cycloakylamino, di-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, di-$C_{1-4}$akylaminocarbonyl, di-$C_{3-10}$cycloakylaminocarbonyl, $C_{1-4}$akylcarbonyl, $C_{3-12}$cycloakylcarbonyl, $C_{6-12}$arylcarbonyl, - S(O)H, $C_{1-4}$akylsulfinyl, $-S(O)_2H$, $C_{1-4}$akylsulfonyl, $-SO_2NH_2$, mono-$C_{1-4}$akylaminosulfonyl, di-$C_{1-4}$akylaminosulfonyl nitro.

82. The compound according to any one of the previous statements, wherein each $Z^7$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl, heterocyclyl, heteroaryl, hydroxyl, $C_{1-6}$alkyloxy, $C_{3-10}$cycloalkyloxy, $C_{6-12}$aryloxy, heterocyclyloxy, heteroaryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-12}$cycloakylaminocarbonyl, $C_{1-4}$akylcarbonyl, $C_{3-12}$cycloakylcarbonyl, $C_{6-12}$arylcarbonyl, -S(O)H, $C_{1-4}$akylsulfinyl, $-S(O)_2H$, $C_{1-4}$akylsulfonyl, $-SO_2NH_2$, mono-$C_{1-4}$akylaminosulfonyl, nitro.

83. The compound according to any one of the previous statements, wherein each $Z^7$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, $C_{6-12}$aryl$C_{1-6}$alkyl,

heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, mono-$C_{3-10}$cycloakylamino, mono-$C_{6-12}$arylamino, hydroxycarbonyl, $C_{1-4}$akyloxycarbonyl, $C_{3-10}$cycloakyloxycarbonyl, $C_{6-12}$aryloxycarbonyl, aminocarbonyl, mono-$C_{1-4}$akylaminocarbonyl, mono-$C_{3-10}$cycloakylaminocarbonyl, $C_{1-4}$akylcarbonyl, $C_{3-12}$cycloakylcarbonyl, -S(O)H, $C_{1-4}$akylsulfinyl, -$S(O)_2$H, $C_{1-4}$akylsulfonyl, -$SO_2NH_2$, mono-$C_{1-4}$akylaminosulfonyl.

84. The compound according to any one of the previous statements, wherein each $Z^7$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, $C_{3-10}$cycloalkyloxy, $C_{6-12}$aryloxy, cyano, amino, mono-$C_{1-4}$akylamino, hydroxycarbonyl, $C_{1-4}$akyloxycarbonyl, aminocarbonyl, mono-$C_{1-4}$akylaminocarbonyl, mono-$C_{3-10}$cycloakylaminocarbonyl, $C_{1-4}$akylcarbonyl.

85. The compound according to any one of the previous statements, wherein each $Z^7$ is independently selected from the group consisting of halo, $C_{1-6}$alkyl, halo$C_{1-4}$akyl, halo$C_{1-4}$akyloxy, $C_{3-10}$cycloalkyl, $C_{6-12}$aryl, heterocyclyl, heteroaryl, hydroxyl, $C_{1-6}$alkyloxy, cyano, amino, mono-$C_{1-4}$akylamino, aminocarbonyl, mono-$C_{1-4}$akylaminocarbonyl, mono-$C_{3-10}$cycloakylaminocarbonyl, $C_{1-4}$akylcarbonyl.

86. The compound according to any one of the previous statements, wherein each $Z^7$ is independently selected from the group consisting of halo, $C_{1-6}$alkyl, halo$C_{1-4}$akyl, $C_{3-10}$cycloalkyl, $C_{6-12}$aryl, heterocyclyl, heteroaryl, hydroxyl, $C_{1-6}$alkyloxy, cyano, amino.

87. The compound according to any one of the previous statements, wherein each $Z^7$ is independently selected from the group consisting of halo, $C_{1-6}$alkyl, halo$C_{1-4}$akyl, $C_{3-10}$cycloalkyl, $C_{6-12}$aryl, hydroxyl, $C_{1-6}$alkyloxy, cyano.

88. A pharmaceutical composition comprising a compound of formula (I) according to any one of the previous statements and a pharmaceutically acceptable carrier.

89. A compound of formula (I) according to any one of statements 1 to 87, or a pharmaceutical composition according to statement 88, for use as a medicament.

90. A compound of formula (I) according to any one of statement 1 to 87, or a pharmaceutical composition according to statement 88, for use in the prevention or treatment of a disease associated with ferroptosis and/or oxytosis.

91. The compound for use according to statement 90, wherein the disease associated with ferroptosis and/or oxytosis is selected from the group consisting of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4.

92. The compound for use according to statement 90, wherein the liver disease is selected from hemochromatosis, primary biliary cholangitis, non-alcoholic steatohepatitis or liver fibrosis.

93. The compound for use according to statement 90, wherein the neurological disease is selected from Alzheimer's Disease, Parkinson's Disease, Amyotrophic lateral sclerosis, Multiple Sclerosis, Huntington's Disease, Dementia with Lewy bodies, Friedreich's ataxia, multiple sclerosis, stroke, periventricular leukomalacia, intracerebral haemorrhage, frontotemporal dementia, neurodegeneration with brain iron accumulation, or traumatic brain injury.

94. The compound for use according to statement 90, wherein the ischemia-reperfusion injury is selected from myocardial ischemia-reperfusion injury, liver ischemia-reperfusion injury, renal ischemia-reperfusion injury, intestinal ischemia-reperfusion injury, cerebral ischemia-reperfusion injury, lung ischemia- reperfusion injury, or any surgical ischemia-reperfusion injury.

95. The compound for use according to statement 90, wherein the iron metabolism-related disease is selected from atherosclerosis or diabetes.

96. The compound for use according to statement 90, wherein the lung disease is selected from acute respiratory distress syndrome (ARDS), lung diseases caused by infections such as COVID-19 pulmonary disease, mucoviscidosis, or asthma.

97. The compound for use according to statement 90, wherein the disease is attributable to a genetic disorder of GPX4, preferably the disease is Sedaghatian-type spondylometaphyseal dysplasia.

98. A method of prevention and/or of treatment of a disease associated with ferroptosis and/or oxytosis, said method comprising administering to a subject in need thereof an effective amount of a compound according to any one of statements 1 to 87, or a pharmaceutical composition according to statement 88.

99. The method according to statement 98, wherein the disease associated with ferroptosis and/or oxytosis is selected from the group consisting of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4.

100. The method according to statement 98, wherein the liver disease is selected from hemochromatosis, primary biliary cholangitis, non-alcoholic steatohepatitis or liver fibrosis.

101. The method according to statement 98, wherein neurological disease is selected from Alzheimer's Disease,

Parkinson's Disease, Amyotrophic lateral sclerosis, Multiple Sclerosis, Huntington's Disease, Dementia with Lewy bodies, Friedreich's ataxia, multiple sclerosis, stroke, periventricular leukomalacia, intracerebral haemorrhage, frontotemporal dementia, neurodegeneration with brain iron accumulation, or traumatic brain injury.

102. The method according to statement 98, wherein the ischemia-reperfusion injury is selected from myocardial ischemia-reperfusion injury, liver ischemia-reperfusion injury, renal ischemia-reperfusion injury, intestinal ischemia-reperfusion injury, cerebral ischemia-reperfusion injury, lung ischemia- reperfusion injury, or any surgical ischemia-reperfusion injury.

103. The method according to statement 98, wherein the iron metabolism-related disease is selected from atherosclerosis or diabetes.

104. The method according to statement 98, wherein the lung disease is selected from acute respiratory distress syndrome (ARDS), mucoviscidosis, or asthma.

105. The method according to statement 98, wherein the disease is attributable to a genetic disorder of GPX4, preferably the disease is Sedaghatian-type spondylometaphyseal dysplasia.

The present invention provides a compound of formula (I) or a stereoisomer, or tautomer thereof,

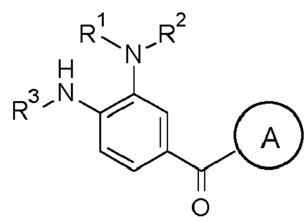

(I)

wherein, cycle A is selected from:

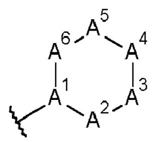

or

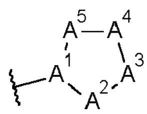

;

wherein the wavy line ( ) indicates the point of attachment of cycle A to the rest of the molecule,

$A^1$ is selected from N or $CR^4$; preferably $A^1$ is N;

$A^2$ is selected from the group consisting of $NR^5$, $CR^6R^7$, S, $SO_2$ and O; preferably $A^2$ is selected from the group consisting of $NR^5$, $CR^6R^7$, and O;

$A^3$ is selected from the group consisting of $NR^5$, $CR^6R^7$, S, $SO_2$ and O; preferably $A^3$ is selected from the group consisting of $NR^5$, $CR^6R^7$, and O;

$A^4$ is selected from the group consisting of $NR^5$, $CR^6R^7$, S, $SO_2$ and O; preferably $A^4$ is selected from the group consisting of $NR^5$, $CR^6R^7$, and O;

$A^5$ is selected from the group consisting of $NR^5$, $CR^6R^7$, S, $SO_2$ and O; preferably $A^5$ is selected from the group consisting of $NR^5$, $CR^6R^7$, and O;

$A^6$ is selected from the group consisting of $NR^5$, $CR^6R^7$, S, $SO_2$ and O; preferably $A^6$ is selected from the group consisting of $NR^5$, $CR^6R^7$, and O;

wherein when $A^1$ is $CR^4$, then at least one of $A^2$, $A^3$, $A^4$, $A^5$, or $A^6$ is selected from the group consisting of $NR^5$, $CR^6R^7$, S, $SO_2$ and O;

$R^1$ is selected from the group consisting of hydrogen, $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl; preferably $R^1$ is selected from the group consisting of hydrogen, $C_{3-10}$cycloalkyl, $C_{6-12}$aryl$C_{1-6}$alkyl, $C_{3-10}$cycloalkenyl, $C_{6-12}$aryl, heterocyclyl, heterocyclyl$C_{1-6}$alkyl, and heteroaryl$C_{1-6}$alkyl; preferably $R^1$ is selected from the group consisting of hydrogen, $C_{3-8}$cycloalkyl, $C_{6-12}$aryl$C_{1-6}$alkyl, $C_{3-8}$cycloalkenyl, heterocyclyl, heterocyclyl$C_{1-6}$alkyl, and heteroaryl-$C_{1-6}$alkyl; preferably $R^1$ is selected from the group consisting of hydrogen, $C_{3-8}$cycloalkyl, $C_{6-12}$aryl$C_{1-6}$alkyl, heterocyclyl, heterocyclyl$C_{1-6}$alkyl, and heteroaryl$C_{1-6}$alkyl;

wherein said groups can be unsubstituted or substituted with one or more $Z^1$; preferably said groups are unsubstituted or substituted with one, two or three $Z^1$;

$R^2$ is selected from the group consisting of hydrogen, $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl; preferably $R^2$ is selected from the group consisting of hydrogen, $C_{3-10}$cycloalkyl, $C_{6-12}$aryl$C_{1-6}$alkyl, $C_{3-10}$cycloalkenyl, $C_{6-12}$aryl, heterocyclyl, heterocyclyl$C_{1-6}$alkyl, and heteroaryl$C_{1-6}$alkyl; preferably $R^2$ is selected from the group consisting of hydrogen, $C_{3-8}$cycloalkyl, $C_{6-12}$aryl$C_{1-6}$alkyl, $C_{3-8}$cycloalkenyl, heterocyclyl, heterocyclyl$C_{1-6}$alkyl, and heteroaryl-$C_{1-6}$alkyl; preferably $R^2$ is selected from the group consisting of hydrogen, $C_{3-8}$cycloalkyl, $C_{6-12}$aryl$C_{1-6}$alkyl, heterocyclyl, heterocyclyl$C_{1-6}$alkyl, and heteroaryl$C_{1-6}$alkyl;

wherein said groups can be unsubstituted or substituted with one or more $Z^2$; preferably said groups are unsubstituted or substituted with one, two or three $Z^2$;

$R^3$ is selected from the group consisting of $C_{3-10}$cycloalkyl, $C_{3-10}$cycloalkenyl, $C_{3-10}$cycloalkynyl, $C_{6-12}$aryl, heterocyclyl, and heteroaryl; preferably $R^3$ is selected from the group consisting $C_{3-8}$cycloalkyl, $C_{3-8}$cycloalkenyl and heterocyclyl;

wherein said groups can be unsubstituted or substituted with one or more $Z^3$; preferably said groups are unsubstituted or substituted with one, two or three $Z^3$;

$R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$; preferably $R^4$ is selected from the group consisting of hydrogen, $C_{1-6}$alkyl, halogen, halo$C_{1-6}$alkyl, $C_{1-6}$alkyloxy, halo$C_{1-6}$alkyloxy, cyano, $-C(O)_2R^{10}$ and $-S(O)_2R^{10}$; preferably $R^4$ is selected from the group consisting of hydrogen, $C_{1-4}$alkyl, halogen, halo$C_{1-4}$alkyl, $C_{1-4}$alkyloxy, halo$C_{1-4}$alkyloxy and cyano;

wherein said groups can be unsubstituted or substituted with one or more $Z^4$; preferably said groups are unsubstituted or substituted with one, two or three $Z^4$;

each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$; preferably each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-8}$cycloalkyl, $C_{6-12}$aryl, heterocyclyl, heteroaryl, cyano, $-C(O)_2R^{10}$ and $-S(O)_2R^{10}$; preferably each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy and cyano;

wherein said groups can be unsubstituted or substituted with one or more $Z^5$; preferably said groups are unsubstituted or substituted with one, two or three $Z^5$;

each $R^6$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$; preferably each $R^6$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-8}$cycloalkyl, $C_{6-12}$aryl, heterocyclyl, heteroaryl, cyano, $-C(O)_2R^{10}$ and $-S(O)_2R^{10}$; preferably each $R^6$ is independently selected from the group consisting of hydrogen, $C_{1-6}$alkyl, halogen, halo$C_{1-6}$alkyl, $C_{1-6}$alkyloxy, halo$C_{1-6}$alkyloxy and cyano;

wherein said groups can be unsubstituted or substituted with one or more $Z^6$; preferably said groups are unsubstituted or substituted with one, two or three $Z^6$;

each $R^7$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$; preferably each $R^7$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-8}$cycloalkyl, $C_{6-12}$aryl, heterocyclyl, heteroaryl, cyano, $-C(O)_2R^{10}$ and $-S(O)_2R^{10}$; preferably each $R^7$ is independently selected from the group consisting of hydrogen, $C_{1-6}$alkyl, halogen, halo$C_{1-6}$alkyl, $C_{1-6}$alkyloxy, halo$C_{1-6}$alkyloxy and cyano;

wherein said groups can be unsubstituted or substituted with one or more $Z^7$; preferably said groups are unsubstituted or substituted with one, two or three $Z^7$;

each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;

each $R^{10}$ is independently selected from the group consisting of $C_{1-6}$alkyl, hydrogen, $C_{3-10}$cycloalkyl, $C_{6-12}$aryl, $C_{6-12}$aryl$C_{1-6}$alkyl, heterocyclyl, and heteroaryl;

each $Z^1$, $Z^2$, $Z^3$, $Z^4$, $Z^5$, $Z^6$ and $Z^7$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-C(O)R^{10}$, $-S(O)R^{10}$, $-S(O)_2R^{10}$, $-S(O)_2NR^8R^9$, nitro; preferably each $Z^1$, $Z^2$, $Z^3$, $Z^4$, $Z^5$, $Z^6$ and $Z^7$ is independently selected from the group consisting of halo, halo$C_{1-6}$alkyl, $C_{1-6}$alkyl, halo-$C_{1-6}$alkyloxy, $C_{3-8}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-S(O)R^{10}$, $-S(O)_2R^{10}$, $-S(O)_2NR^8R^9$, nitro; preferably each $Z^1$, $Z^2$, $Z^3$, $Z^4$, $Z^5$, $Z^6$ and $Z^7$ is independently selected from the group consisting of halo, halo$C_{1-6}$alkyl, $C_{1-6}$alkyl, halo$C_{1-6}$alkyloxy, hydroxyl, $-OR^{10}$, cyano, amino, $-NR^8R^9$, $-C(O)_2R^{10}$, $-C(O)NR^8R^9$, $-S(O)_2R^{10}$, $-S(O)_2NR^8R^9$;

or a solvate, hydrate, pharmaceutically acceptable salt, or prodrug thereof;

wherein when the moiety $-NR^1R^2$ is

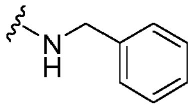,

cycle A is piperazinyl and $R^3$ is cyclohexyl, then $R^5$ is not substituted benzyl, 4-methylpyridine or methyl.

Particularly preferred compounds of the invention are those compounds listed in Table 1.

Table 1.

| Compound code | Structure |
|---|---|
| CPD-001 | 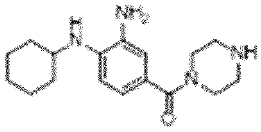 |
| CPD-002 | 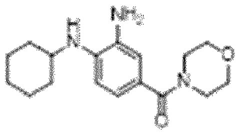 |
| CPD-003 | 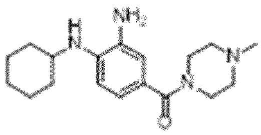 |
| CPD-004 | 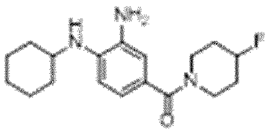 |
| CPD-005 | 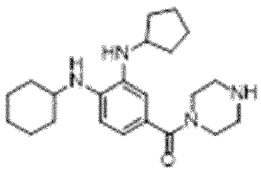 |
| CPD-006 | 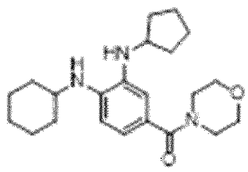 |
| CPD-007 | 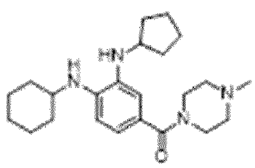 |
| CPD-008 | 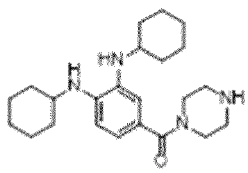 |

(continued)

| Compound code | Structure |
|---|---|
| CPD-009 | 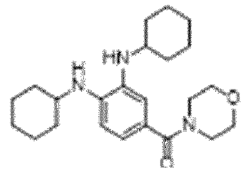 |
| CPD-010 | 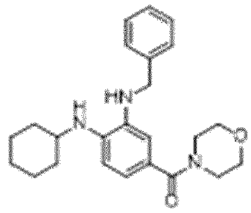 |
| CPD-011 | 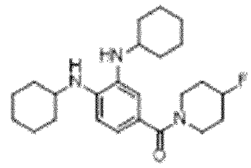 |
| CPD-012 | 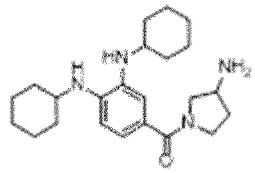 |
| CPD-013 | 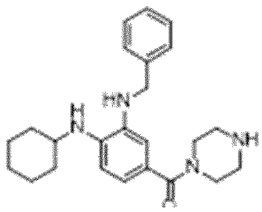 |
| CPD-014 | 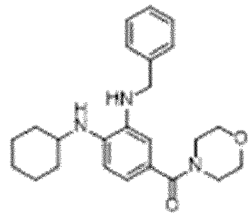 |
| CPD-015 | 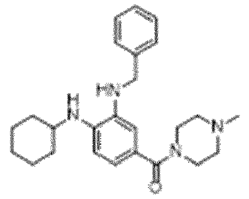 |

(continued)

| Compound code | Structure |
|---|---|
| CPD-016 | 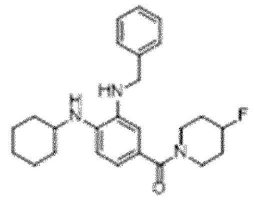 |
| CPD-017 | 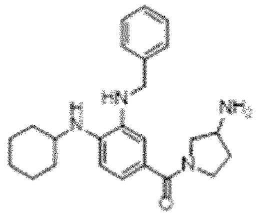 |
| CPD-018 | 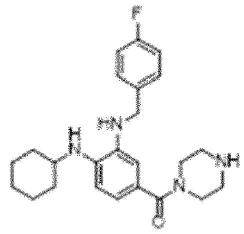 |
| CPD-019 | 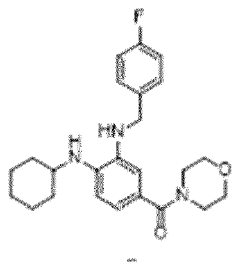 |
| CPD-020 | 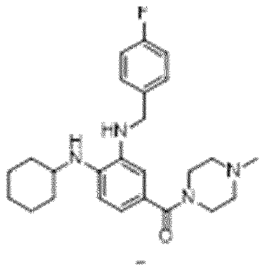 |
| CPD-021 | 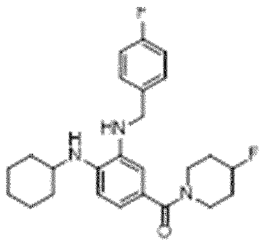 |

The compounds of the present invention have been found to be potent inhibitors of ferroptosis and/or oxytosis. Accordingly, the invention provides for the compounds of the invention for use in therapy. Their use is particularly advantageous in view of their properties making them particularly suited for use *in vivo,* i.e. demonstrating an improved effect *in vivo.*

More particularly, the compounds have been found to have moderate to high solubility and/or show high CNS

MPO scores indicating that they are compounds with increased probability of success for use in the CNS.

A number of diseases are characterized by a dysregulation of ferroptosis and/or oxytosis, such as, but not limited to an excess in ferroptosis and/or oxytosis, causing cell-death. Accordingly, the present invention provides compounds of formula (I), and any subgroup thereof such as (I), (IA), (IB), (IC), (ID), (IE), (IF), (IG), (IH), (IJ) for use in the prevention or treatment of a disease associated with ferroptosis and/or oxytosis, more particularly an excess of ferroptosis and/or oxytosis leading to cell-death.

In a particular embodiment the invention provides the compounds of the invention or a pharmaceutical composition comprising one or more compounds of the invention for use in the treatment of a mammal suffering from excessive ferroptosis in one or more organs. The compounds of the invention are of use in a method of treatment or prevention of a disease characterized by a dysregulation of ferroptosis and/or oxytosis, such as, but not limited to an excess in ferroptosis and/or oxytosis, causing cell-death, which comprises administering one or more of the compounds of the invention to a patient suffering from said disease.

In some embodiments the disease associated with ferroptosis and/or oxytosis is selected from the group consisting of liver disease (NASH, NAFLD), lung disease, chronic kidney disease, ocular surface diseases, wound healing, multiple organ dysfunction syndrome, neurological disease, acute renal failure, ischemia-reperfusion injury, sepsis, iron toxicity or iron poisoning, prevention of transplant rejection, iron metabolism-related disease and genetic disorders of GPX4.

Non-limiting examples of disorders according to the present disclosure include epilepsy, kidney disease, stroke, myocardial infarction, type I diabetes, traumatic brain injury (TBI), periventricular leukomalacia (PVL), and neurological disease. Non-limiting examples of neurological diseases according to the present disclosure include Alzheimer's, Parkinson's, Amyotrophic lateral sclerosis, Friedreich's ataxia, Multiple sclerosis, Huntington's Disease, Transmissible spongiform encephalopathy, Charcot-Marie-Tooth disease, Dementia with Lewy bodies, Corticobasal degeneration, Progressive supranuclear palsy, Chronic Traumatic Encephalopathy (CTE), and Hereditary spastic paraparesis.

Non-limiting examples of liver disease include hemochromatosis, primary biliary cholangitis, non-alcoholic steatohepatitis or liver fibrosis.

Non-limiting examples of neurological disease include Alzheimer's Disease, Parkinson's Disease, Amyotrophic lateral sclerosis, Multiple Sclerosis, Huntington's Disease, Dementia with Lewy bodies, Friedreich's ataxia, multiple sclerosis, stroke, periventricular leukomalacia, intracerebral haemorrhage, frontotemporal dementia, neurodegeneration with brain iron accumulation, or traumatic brain injury.

Non-limiting examples of ischemia-reperfusion injury include myocardial ischemia-reperfusion injury, liver ischemia-reperfusion injury, lung ischemia-reperfusion injury, intestinal ischemia-reperfusion injury, renal ischemia-reperfusion injury, cerebral ischemia-reperfusion injury or any surgical ischemia-reperfusion injury.

Non-limiting examples of iron metabolism-related disease include atherosclerosis or diabetes.

Non-limiting examples of lung disease include acute respiratory distress syndrome (ARDS), lung diseases caused by infections such as COVID-19 pulmonary disease, mucoviscidosis, or asthma.

Non-limiting examples of genetic disorder of GPX4, for instance a mutation-related disease of GPX4, such as Sedaghatian-type spondylometaphyseal dysplasia.

In a particular embodiment, the compounds of the invention are envisaged for use in the treatment or prevention of a disease or disorder such as those described above, wherein the disease or disorder is attributable to a genetic disorder of GPX4, for instance a mutation-related disease of GPX4, such as Sedaghatian-type spondylometaphyseal dysplasia.

In a particular embodiment the invention provides the compounds of the invention or a pharmaceutical composition comprising one or more compounds of the invention for the treatment of diseases caused by excitatory amino acids. A well-known example of an excitatory amino acid is glutamate and the condition is designated as glutamine excitotoxicity.

In a particular embodiment the invention provides the compounds of the invention or a pharmaceutical composition comprising one or more compounds of the invention for the treatment of diseases caused by increased levels of phospholipid peroxides.

In a particular embodiment the invention provides the compounds of the invention or a pharmaceutical composition comprising one or more compounds of the invention for the treatment of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, and iron metabolism-related disease.

In the present invention the potency of a compound inhibiting (or reducing) ferroptosis and/or oxytosis are determined in (bio)chemical antioxidant activity assays, *in vitro* assays. Typically FENIX assay is used to quantify radical trapping antioxidant activity in phospholipid bilayers to predict anti-ferroptotic potency of lipophilic antioxidants in cells. Typically, *in vitro* assays are used to measure the potency of a candidate compound. Examples of suitable *in vitro* assays are cellular assays. One non-limiting example of an assay involves the use of the IMR-32 neuroblastoma cell line. The latter is stimulated to enter into ferroptosis upon stimulation with 10μM erastin, a documented ferroptosis inducer (see for

example Dixon et al (2012) Cell 149, 1060-1072 and ferroptosis inhibitors are evaluated for the prevention of erastin induced ferroptosis. Another assay involves the use of ML162-induced ferroptosis in HT1080 human fibrosarcoma cells. Yet another assay is based on the glutamate-induced cell death in the hippocampal cell line HT22 and ferroptosis/oxytosis inhibitors are evaluated for the prevention of cell death (see Henke N. et al (2013) Cell Death and Disease 4, e470). Still another assay is based on the sorafenib induced cell death (described to be iron dependent cell death) in hepatocellular carcinoma cells and ferroptosis inhibitors are evaluated for the prevention of cell death (see Louandre C. et al (2013) Int. J. Cancer 133, 1732). The calculated potency of acompound inhibiting ferroptosis and/or oxytosis is typically depicted as an IC50 value. Examples of suitable *in vivo* assays are typically pre-clinical disease models of for example mice for the diseases benefiting the application of ferroptosis and/or oxytosis inhibitors, as described herein.

One non-limiting example of an *in vivo* assay is based on inducing organ injury by iron overload or using liver, kidney, lung, brain or intestine-specific *Gpx4*-deficient mouse lines and ferroptosis inhibitors are evaluated based on the level of reduction in plasma injury biomarkers LDH, CK, AST and ALT, and body temperature (see Van Coillie et al. (2022) Nat Comm 13: 1046).

The compounds of this invention can be administered as the sole pharmaceutical agent or in combination with one or more other pharmaceutical agents where the combination causes no unacceptable adverse effects. The present invention relates also to such combinations. For example, the compounds of this invention can be combined with known therapeutic agents for the treatment of diseases mentioned herein, as well as with admixtures and combinations thereof. Particularly preferred combinations are necroptosis inhibitors (e.g. necrostatin-1) and ferroptosis inhibitors. Examples of these combinations are described in Linkermann *et al* 2014.

The compounds of the invention may be in the form of salts, preferably pharmaceutically acceptable salts, as generally described below. Some preferred, but non-limiting examples of suitable pharmaceutically acceptable organic and/or inorganic acids are as hydrochloric acid, trifluoroacetic acid (or triflate), hydrobromic acid, sulfuric acid, nitric acid, acetic acid and citric acid, as well as other pharmaceutically acceptable acids known per se (for which reference is made to the prior art referred to below).

When the compounds of the invention contain an acidic group as well as a basic group the compounds of the invention may also form internal salts, and such compounds are within the scope of the invention. When the compounds of the invention contain a hydrogen-donating heteroatom (e.g. NH), the invention also covers salts and/or isomers formed by transfer of said hydrogen atom to a basic group or atom within the molecule.

Pharmaceutically acceptable salts of the compounds of formula (I) and any subgroup thereof include the acid addition and base salts thereof. Suitable acid addition salts are formed from acids which form non-toxic salts. Examples include the acetate, adipate, aspartate, benzoate, besylate, bicarbonate/carbonate, bisulfate/sulfate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate (or triflate) and xinofoate salts. Suitable base salts are formed from bases which form non-toxic salts. Examples include the aluminium, arginine, benzathine, calcium, choline, diethylamine, diolamine, glycine, lysine, magnesium, meglumine, olamine, potassium, sodium, tromethamine and zinc salts. Hemisalts of acids and bases may also be formed, for example, hemisulphate and hemicalcium salts. For a review on suitable salts, see Handbook of Pharmaceutical Salts: Properties, Selection, and Use by Stahl and Wermuth (Wiley-VCH, 2002), incorporated herein by reference.

The compounds of the invention may exist in a continuum of solid states ranging from fully amorphous to fully crystalline. The term 'amorphous' refers to a state in which the material lacks long range order at the molecular level and, depending upon temperature, may exhibit the physical properties of a solid or a liquid. Typically, such materials do not give distinctive X-ray diffraction patterns and, while exhibiting the properties of a solid, are more formally described as a liquid. Upon heating, a change from solid to liquid properties occurs which is characterized by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterized by a phase change, typically first order ('melting point').

Pharmaceutically acceptable salts of compounds of formula (I) may be prepared by one or more of these methods:

(i) by reacting the compound of formula (I) with the desired acid;

(ii) by reacting the compound of formula (I) with the desired base;

(iii) by removing an acid- or base-labile protecting group from a suitable precursor of the compound of formula (I) or by

ring-opening a suitable cyclic precursor, for example, a lactone or lactam, using the desired acid; or

(iv) by converting one salt of the compound of formula (I) to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost non-ionized.

The compounds of the invention may also exist in unsolvated and solvated forms. The term 'solvate' is used herein to describe a molecular complex comprising the compound of the invention and one or more pharmaceutically acceptable solvent molecules, for example, ethanol. The term 'hydrate' is employed when said solvent is water.

A currently accepted classification system for organic hydrates is one that defines isolated site, channel, or metal-ion coordinated hydrates - see Polymorphism in Pharmaceutical Solids by K. R. Morris (Ed. H. G. Britain, Marcel Dekker, 1995), incorporated herein by reference. Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

Also included within the scope of the invention are multi-component complexes (other than salts and solvates) wherein the drug and at least one other component are present in stoichiometric or non-stoichiometric amounts. Complexes of this type include clathrates (drug-host inclusion complexes) and co-crystals. The latter are typically defined as crystalline complexes of neutral molecular constituents which are bound together through non-covalent interactions but could also be a complex of a neutral molecule with a salt. Co-crystals may be prepared by melt crystallization, by recrystallization from solvents, or by physically grinding the components together - see Chem Commun, 17, 1889-1896, by O. Almarsson and M. J. Zaworotko (2004), incorporated herein by reference. For a general review of multi-component complexes, see J Pharm Sci, 64 (8), 1269-1288, by Haleblian (August 1975), incorporated herein by reference.

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as $-COO^-Na^+$, $-COO^-K^+$, or $-SO_3^-Na^+$) or non-ionic (such as $-N^-N^+(CH_3)_3$) polar head group. For more information, see Crystals and the Polarizing Microscope by N. H. Hartshorne and A. Stuart, 4th Edition (Edward Arnold, 1970), incorporated herein by reference.

All references to compounds of formula (I) or any subgroups thereof include references to salts, solvates, multi-component complexes and liquid crystals thereof and to solvates, multi-component complexes and liquid crystals of salts thereof.

The compounds of the invention include compounds of formula (I) or any subgroups thereof as hereinbefore defined, including all polymorphs and crystal habits thereof, prodrugs and isomers thereof (including optical, geometric and tautomeric isomers) as hereinafter defined and isotopically-labelled compounds of formula (I).

In addition, although generally, with respect to the salts of the compounds of the invention, pharmaceutically acceptable salts are preferred, it should be noted that the invention in its broadest sense also included non-pharmaceutically acceptable salts, which may for example be used in the isolation and/or purification of the compounds of the invention.

A further related aspect of the present invention provides a pharmaceutical composition comprising a compound of formula (I) or a stereoisomer, or tautomer thereof, and a pharmaceutically acceptable carrier.

The term "pharmaceutically acceptable" as used herein is consistent with the art and means compatible with the other ingredients of a pharmaceutical composition and not deleterious to the recipient thereof.

As used herein, "carrier" or "excipient" includes any and all solvents, diluents, buffers (such as, e.g., neutral buffered saline or phosphate buffered saline), solubilisers, colloids, dispersion media, vehicles, fillers, chelating agents (such as, e.g., EDTA or glutathione), amino acids (such as, e.g., glycine), proteins, disintegrants, binders, lubricants, wetting agents, emulsifiers, sweeteners, colorants, flavourings, aromatisers, thickeners, agents for achieving a depot effect, coatings, antifungal agents, preservatives, antioxidants, tonicity controlling agents, absorption delaying agents, and the like. The use of such media and agents for pharmaceutical active substances is well known in the art. Except insofar as any conventional media or agent is incompatible with the active substance, its use in the therapeutic

compositions may be contemplated.

Illustrative, non-limiting carriers for use in formulating the pharmaceutical compositions include, for example, oil-in-water or water-in-oil emulsions, aqueous compositions with or without inclusion of organic co-solvents suitable for intravenous (IV) use, liposomes or surfactant-containing vesicles, microspheres, microbeads and microsomes, powders, tablets, capsules, suppositories, aqueous suspensions, aerosols, and other carriers apparent to one of ordinary skill in the art.

Pharmaceutical compositions as intended herein may be formulated for essentially any route of administration, such as without limitation, oral administration (such as, e.g., oral ingestion or inhalation), intranasal administration (such as, e.g., intranasal inhalation or intranasal mucosal application), parenteral administration (such as, e.g., subcutaneous, intravenous (I.V.), intramuscular, intraperitoneal or intrasternal injection or infusion), transdermal or transmucosal (such as, e.g., oral, sublingual, intranasal) administration, topical administration, rectal, vaginal or intra-tracheal instillation, and the like. In this way, the therapeutic effects attainable by the methods and compositions can be, for example, systemic, local, tissue-specific, etc., depending of the specific needs of a given application.

In preferred embodiments, the compound or the pharmaceutical composition as taught herein is administered parenterally. More preferably, the compound or the pharmaceutical composition as taught herein is administered intravenously, for example by infusion.

The dosage or amount of the agent as taught herein, optionally in combination with one or more other active compounds to be administered, depends on the individual case and is, as is customary, to be adapted to the individual circumstances to achieve an optimum effect. Thus, the unit dose and regimen depend on the nature and the severity of the disorder to be treated, and also on factors such as the species of the subject, the sex, age, body weight, general health, diet, mode and time of administration, immune status, and individual responsiveness of the human or animal to be treated, efficacy, metabolic stability and duration of action of the compounds used, on whether the therapy is acute or chronic or prophylactic, or on whether other active compounds are administered in addition to the agent of the invention. To optimize therapeutic efficacy, the compound or the pharmaceutical composition as taught herein can be first administered at different dosing regimes. Typically, levels of the agent in a tissue can be monitored using appropriate screening assays as part of a clinical testing procedure, e.g., to determine the efficacy of a given treatment regimen. The frequency of dosing is within the skills and clinical judgement of medical practitioners (e.g., doctors, veterinarians or nurses). Typically, the administration regime is established by clinical trials which may establish optimal administration parameters. However, the practitioner may vary such administration regimes according to the one or more of the aforementioned factors, e.g., subject's age, health, weight, sex and medical status. The frequency of dosing can be varied depending on whether the treatment is prophylactic or therapeutic.

Toxicity and therapeutic efficacy of the agent as described herein or pharmaceutical compositions comprising the same can be determined by known pharmaceutical procedures in, for example, cell cultures or experimental animals. These procedures can be used, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Pharmaceutical compositions that exhibit high therapeutic indices are preferred. While pharmaceutical compositions that exhibit toxic side effects can be used, care should be taken to design a delivery system that targets such compounds to the site of affected tissue to minimize potential damage to normal cells (e.g., non-target cells) and, thereby, reduce side effects.

The data obtained from the cell culture assays and animal studies can be used in formulating a range of dosage for use in appropriate subjects. The dosage of such pharmaceutical compositions lies generally within a range of circulating concentrations that include the ED50 with little or no toxicity. The dosage may vary within this range depending upon the dosage form employed and the route of administration utilised. For a pharmaceutical composition used as described herein, the therapeutically effective dose can be estimated initially from cell culture assays. A dose can be formulated in animal models to achieve a circulating plasma concentration range that includes the IC50 (i.e., the concentration of the pharmaceutical composition which achieves a half-maximal inhibition of symptoms) as determined in cell culture. Such information can be used to more accurately determine useful doses in humans. Levels in plasma can be measured, for example, by high performance liquid chromatography.

In particular embodiment, the compound as taught herein is the main or only active ingredient of the pharmaceutical composition.

## Examples

The following examples are provided for the purpose of illustrating the present invention and by no means should be interpreted to limit the scope of the present invention.

## 1. Chemical synthesis of representative compounds of the invention

Unless otherwise stated, laboratory reagent grade solvents were used. Reagents were obtained from various commercial sources and were used without any prior purification.

Characterisation of all compounds was done with $^{1}H$ and $^{13}C$ NMR and mass spectrometry. NMR spectra were recorded with a 400 MHz Bruker Avance III Nanobay spectrometer with Ultrashield. All obtained spectra were analysed using MestReNova analytical chemistry software. Chemical shifts are displayed in ppm and coupling constants are shown in hertz (Hz). ES mass spectra were obtained from an Esquire 3000plus Ion Trap Mass Spectrometer from Bruker Daltonics.

The UPLC (ultra performance liquid chromatography), used to quantify the purity of the products, was an ACQUITY UPLC H-Class system with a TUV detector Waters coupled to an MS detector Waters Qda. Waters Acquity UPLC BEH C18 1.7 μm, 2.1 mm × 50 mm column was used. The eluent was composed of two different solvents. Solvent C consisted of water with 0.1% formic acid, solvent D was acetonitrile. For most of the experiments, unless stated otherwise, the following general method was used. The column was first equilibrated for 0.15 min with a mixture of 95% solvent C and 5% solvent D. After that, solvent D was increased linearly to 100% over 1.75 min before being held constant for 0.25 min, followed by a mixture of 95% solvent C and 5% solvent D for 0.75 min (flow rate 0.7 ml/min). All mass spectra were recorded over a *m*/*z* range of 100-1000. The wavelength for UV detection was 254 nm.

Method II starts with equilibration of column for 0.15 min with a mixture of 95% solvent C and 5% solvent D. After that, solvent D was increased linearly to 100% over 2.50 min before being held constant for 0.75 min, followed by a mixture of 95% solvent C and 5% solvent D for 0.75 min (flow rate 0.7 ml/min).

Selected compounds of the invention were analyzed by high resolution mass spectrometry: 10μL of each sample (concentration = $10^{-5}$ M) was injected using the CapLC system (Waters, Manchester, UK) and electrosprayed using a standard electrospray source. Samples were injected with an interval of 5 min. Positive ion mode accurate mass spectra were acquired usinga Q-TOF II instrument (Waters, Man-chester, UK). The MS was calibrated prior to use with a 0.2% $H_3PO_4$ solution. The spectra were lock mass corrected using the known mass of the nearest $H_3PO_4$ cluster.

During the chemical synthesis, flash purification was performed when necessary, on a Biotage ISOLERA One flash system equipped with an internal variable dual wavelength diode array detector (200-400 nm). Reverse phase purifications were done using Buchi EcoFlex C18 cartridges, dry sample loading was done by self-packing sample cartridges using Celite® 545. Gradients used varied for each purification. Several synthesis procedures that were used in the preparation of intermediates and final products are summarized here as "General Procedures".

All reactions were performed under argon unless otherwise stated. The final products have a purity above 95% unless otherwise stated.

*Scheme 1.*

*Scheme 1.*

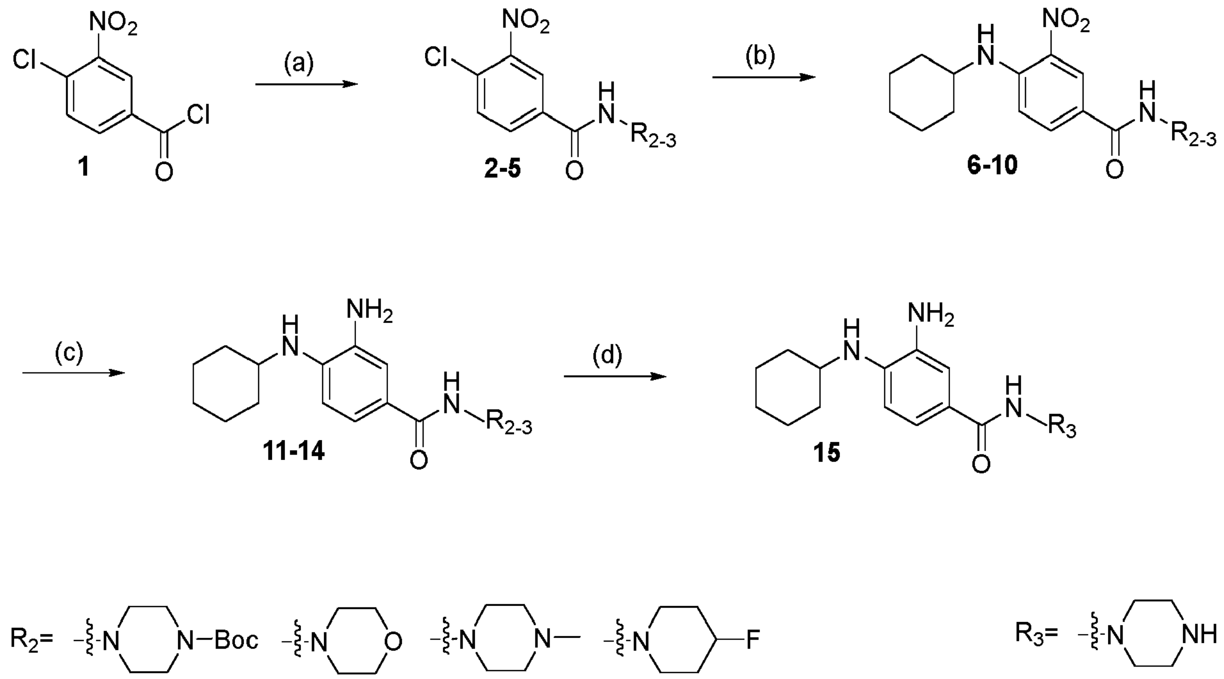


*Scheme 2.*

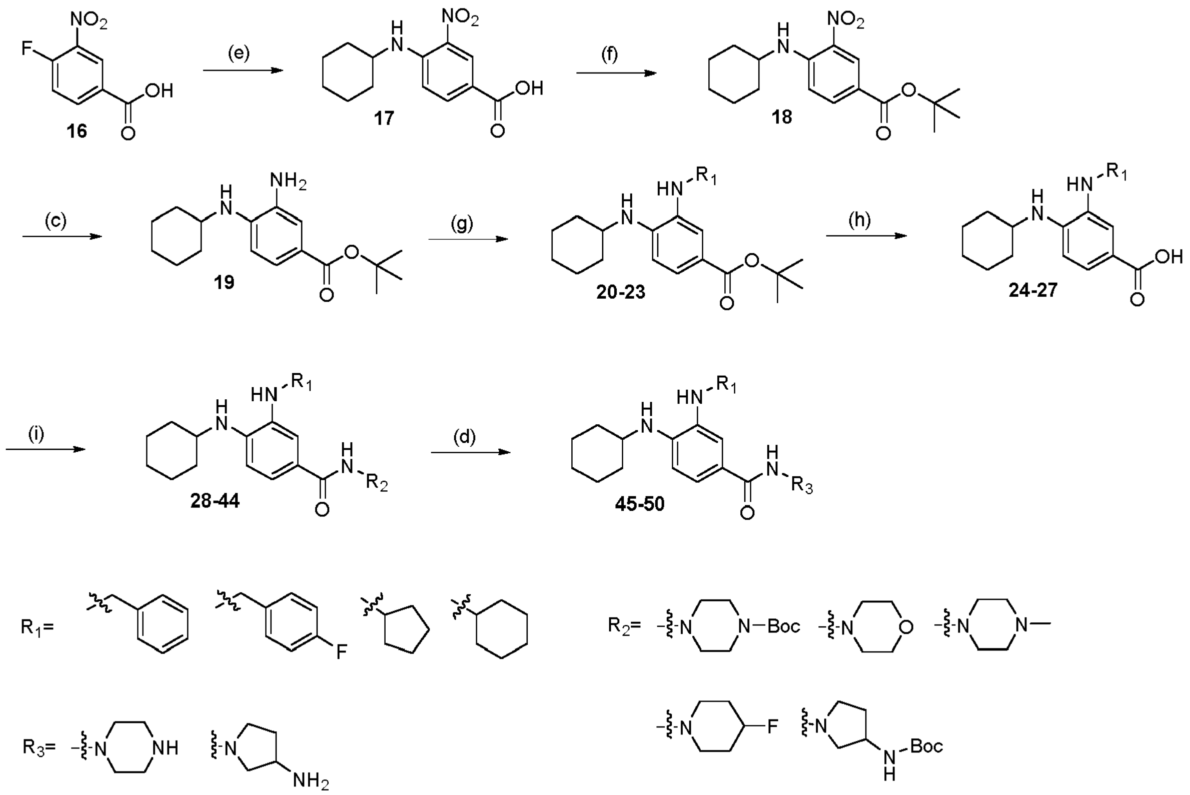


General procedure A: nucleophilic substitution (Schemes 1 (a)).

A stirred solution of 4-chloro-3-nitrobenzoyl chloride**1** (1.0 eq) in DCM, pyridine (2.0 eq) and the appropriate cycloalkyl amine substituent (1.0 eq) were added and the reaction was stirred at room temperature for 16 hours. After completion, the reaction mixture was diluted with EtOAc (10 ml) and the organic layers were washed with water (3 × 10 ml) and HCl 1N, dried using anhydrous sodium sulfate before being concentrated in vacuo. The crude compounds **2-5** were

used without any further purification for the next step.

General procedure B: nucleophilic aromatic substitution (Schemes 1 (b)):

To a solution of compound **2-5** (1.0 eq) in ACN cyclohexylamine (3.0 eq) and DIPEA (3.0 eq) were added. The reaction was stirred for 72-96 hours at 90 °C. After completion, the mixture was cooled down to room temperature and extracted with EtOAc (3 × 10 mL). The collected organic layers were washed with water (20 mL) then dried using anhydrous sodium sulfate and concentrated in vacuo. The residue was purified by flash chromatography (ACN/Water +0.1% formic acid) to obtain compounds **6-10.**

General procedure C: catalytic hydrogenation (Schemes 1 and 2 step (c))

Compounds **6-10** (1.0 eq Scheme 1) or **18** (1.0 eq Scheme 2) were dissolved in methanol and the solution was purged with argon. Palladium hydroxide on carbon (20% $Pd(OH)_2/C$, 50% of water, 0.1 eq) was added under inert atmosphere while continuously stirring. Next, the reaction mixture was put under hydrogen atmosphere and stirred for 12-72 hours at room temperature. After completion of the reaction, the solution was filtered through a patch of Celite® and the filtrate was concentrated under reduced pressure. The crude compound was purified by reversed-phase flash chromatography (water/MeOH) to yield products **11-14** or **19.**

General procedure D: Boc deprotection (Scheme 1 and 2 step (d))

Compounds **11** (1.0 eq Scheme 1) or **28, 31, 35, 36, 40, 41** (1.0 eq Scheme 2) were dissolved in dichloromethane followed by the addition of a 4 M solution of HCl in 1,4-dioxane (15.0 eq) The reaction mixture was stirred at room temperature for 2 hours. After completion of the reaction, diethyl ether was added to the reaction mixture in order to precipitate compounds **15** or **45- 50.** The obtained HCl salts were subsequently washed with a minimal amount of diethyl ether.

General procedure E: nucleophilic aromatic substitution (Scheme 2 step (e)):

To a stirred solution of compounds 4-fluoro-3-nitrobenzoic acid **16** (1.0 eq) in ACN, DIPEA (3.0 eq) was added, followed by the addition of cyclohexylamine (3.0 eq). The reaction mixture was stirred for 72-96 hours at room temperature. After completion of the reaction, the yellow precipitate was filtered, washed with water and dried under vacuum, providing compounds 4-(cyclohexylamino)-3-nitrobenzoic acid **17** which was used for the next step without further purification.

General procedure F: esterification (Schemes 2 step (f))

To a stirred solution of 4-(cyclohexylamino)-3-nitrobenzoic acid **17** (1.0 eq) in THF, DIPEA (3.0 eq) and DMAP (1.05 eq) were added followed by the addition of di-t-butyldicarbonate (2.5 eq). The reaction mixture was refluxed for 72-96 hours. Then, the reaction mixture was directly loaded on Celite® and the crude compound was purified by flash chromatography (Hep/EtOAc) to yield *tert*-butyl 4-(cyclohexylamino)-3-nitrobenzoate **18.**

General procedure G: reductive amination (Schemes 2 step (g))

Compound **19** (1.0 eq) was dissolved in THF, followed by the addition of acetic acid (23.0 eq) and the corresponding aldehyde or ketone (3.0 eq). After 15 minutes, sodium cyanoborohydride (2.5 eq) was added and the reaction mixture was stirred at room temperature for 12-16 hours. After completion, the reaction was quenched with water and the product was extracted with DCM (2 × 10 ml). The combined organic layers were dried over sodium sulphate, filtered and loaded on Celite®. The crude compounds were purified by flash chromatography (Hep/EtOAc) to yield products **20-23.**

General procedure H: *t*-Bu ester hydrolysis (Schemes 2 step (h))

To a stirred solution of compounds **20-23** (1.0 eq.) in DCM, TFA (10 eq.) was added and the reaction mixture was stirred 16 hours at room temperature. After completion, acetonitrile was added to the reaction mixture in order to precipitate compounds **24-27** obtained as TFA salts.

General procedure I: amide coupling (Schemes 2 (i)).

A stirred solution of compound **24-27** (1.0 eq) in THF was cooled to 0°C in an ice bath, followed by the addition of HATU (1.4 eq), DIPEA (3.0 eq) and the corresponding amine (1.5 eq). The reaction mixture was stirred for 16 hours at room temperature. After completion, the reaction mixture was directly loaded on Celite® without any additional work-up. The crude compound was purified by reverse-phase flash chromatography (water/MeOH) to yield products **28-44.**

*tert*-butyl 4-(4-chloro-3-nitrobenzoyl)piperazine-1-carboxylate **(2)**

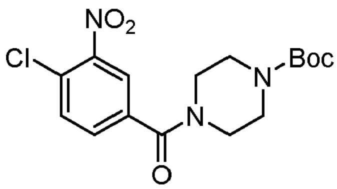

General procedure A was followed using compound **1** (1.000 g, 4.960 mmol) and *tert*-butyl piperazine-1-carboxylate (0.920 g, 4.96 mmol) as the corresponding amine to provide *tert*-butyl 4-(4-chloro-3-nitrobenzoyl)piperazine-1-carboxylate **2** (1.631 g, 4.410 mmol, 97 % yield).
**$^1$H NMR (400 MHz, $CDCl_3$)** δ 1.49 (s, 9H), 3.24 (t, *J* = 5.2 Hz, 2H), 3.38 - 3.62 (m, 4H), 3.76 (d, *J* = 5.3 Hz, 2H), 7.64 (d, *J* = 3.3 Hz, 1H), 8.23 (dd, *J* = 8.3, 1.9 Hz, 1H), 8.59 (d, *J* = 2.0 Hz, 1H).
**MS (ESI)** m/z = 314.0 $[M\text{-}t\text{-}bu]^+$.

(4-chloro-3-nitrophenyl)(morpholino)methanone **(3)**

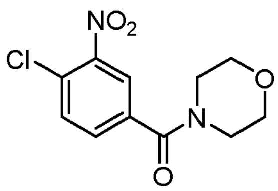

General procedure A was followed using compound **1** (4.000 g, 18.20 mmol) and morpholine (0.518 ml, 6.000 mmol) as the corresponding amine to provide (4-chloro-3-nitrophenyl)(morpholino)methanone **3** (1.600 g, 5.910 mmol, 98 % yield).
**$^1$H NMR (400 MHz, MeOD)** δ 3.45 (br. s, 2H), 3.63 - 3.67 (m, 2H), 3.73 - 3.77 (m, 4H), 7.78 (d, *J* = 0.9 Hz, 1H), 8.21 (dd, *J* = 2.0, 8.4 Hz, 1H), 8.48 (d, *J* = 1.9 Hz, 1H). **$^{13}$C NMR (101 MHz, MeOD)** δ 43.94, 67.59, 125.58, 127.45, 132.96, 133.33, 134.92, 149.58, 168.87.
**MS (ESI)** m/z = 271.1 $[M+H]^+$.

(4-chloro-3-nitrophenyl)(4-methylpiperazin-1-yl)methanone **(4)**

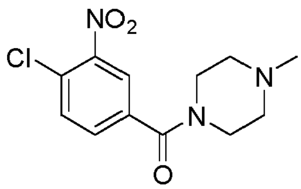

General procedure A was followed using compound **1** (1.500 g, 7.440 mmol) and 1-methylpiperazine (0.745 g, 7.440 mmol) as the corresponding amine to provide (4-chloro-3-nitrophenyl)(4-methylpiperazin-1-yl)methanone **4** (1.863 g, 6.570 mmol, 96 % yield).

**$^1$H NMR (400 MHz, $CDCl_3$)** δ 2.48 (s, 3H), 2.60-2.80 (m, 4H), 3.76 (d, *J*= 119.0 Hz, 4H), 7.95 (d, *J* = 1.9 Hz, 1H), 8.18 (dd, *J* = 2.0, 8.6 Hz, 1H), 8.52 - 8.57 (m, 1H).

**MS (ESI)** m/z = 284.1 $[M+H]^+$.

(4-chloro-3-nitrophenyl)(4-fluoropiperidin-1-yl)methanone **(5)**

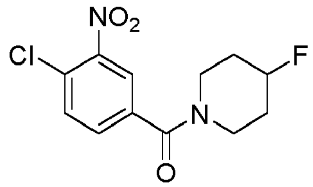

General procedure A was followed using compound **4** (0.200 g, 0.909 mmol) and 4-fluoropiperidine (0.094 g, 0.909 mmol) as the corresponding amine to provide (4-chloro-3-nitrophenyl)(4-fluoropiperidin-1-yl)methanone (0.137 g, 0.478 mmol, 53 % yield).

**$^1$H NMR (400 MHz, $CDCl_3$)** δ 1.93 (d, J = 40.8 Hz, 4H), 3.42 (s, 1H), 3.57 (td, J = 3.7, 11.2 Hz, 2H), 4.07 (s, 1H), 4.93 (dtt, J = 3.0, 5.5, 47.6 Hz, 1H), 7.56 (dd, J = 1.9, 8.2 Hz, 1H), 7.62 (d, J = 8.2 Hz, 1H), 7.93 (d, J = 1.9 Hz, 1H).

**$^{13}$C NMR (101 MHz, $CDCl_3$)** δ 30.69, 31.55, 38.32, 43.61, 86.10, 87.81, 124.43, 128.59, 131.59, 132.34, 135.47, 147.79, 166.87.

**MS (ESI)** m/z = 288 $[M+H]^+$.

*tert*-butyl 4-(4-(cyclohexylamino)-3-nitrobenzoyl)piperazine-1-carboxylate **(6)**

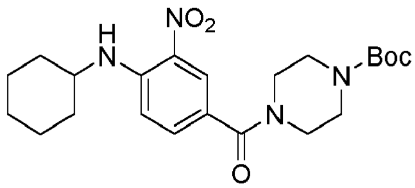

General procedure B was followed using compound **2** (1.631 g, 4.410 mmol), to provide *tert*-butyl 4-(4-(cyclohexylamino)-3-nitrobenzoyl)piperazine-1-carboxylate **6** (1.730 g, 4.000 mmol, 91 % yield).

**$^1$H NMR (400 MHz, $CDCl_3$)** δ 1.31 - 1.40 (m, 1H), 1.42 - 1.46 (m, 2H), 1.49 (s, 9H), 1.62 - 1.66 (m, 2H), 1.66 - 1.73 (m, 1H), 1.79 - 1.89 (m, 2H), 2.03 - 2.11 (m, 2H), 3.40 - 3.54 (m, 4H), 3.53 - 3.59 (m, 1H), 3.59 - 3.69 (m, 4H), 6.93 (d, *J*= 9.0 Hz, 1H), 7.56 (dd, *J* = 2.1, 8.9 Hz, 1H), 8.28 - 8.35 (m, 2H).

**$^{13}$C NMR (101 MHz, $CDCl_3$)** δ 24.47, 25.48, 28.38, 32.61, 51.26, 77.23, 80.43, 114.49, 121.11, 127.06, 130.46, 135.52, 145.52, 154.57, 168.95.

**MS (ESI)** m/z = 377.1 $[M+H]^+$.

(4-(cyclohexylamino)-3-nitrophenyl)(morpholino)methanone **(7)**

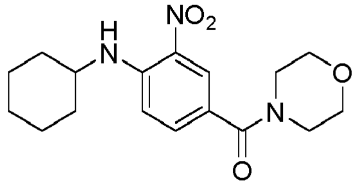

General procedure B was followed using compound **3** (1.600 g, 5.910 mmol), to provide (4-(cyclohexylamino)-3-nitrophenyl)(morpholino)methanone **7** (1.780 g, 5.340 mmol, 90 % yield).

**$^1$H NMR (400 MHz, MeOD)** δ 1.25 - 1.53 (m, 5H), 1.63 - 1.73 (m, 1H), 1.92 - 2.01 (m, 2H), 2.01 - 2.12 (m, 2H), 2.96 -

3.06 (m, 1H), 3.65 (s, 4H), 3.66 - 3.73 (m, 4H), 7.12 (d, *J* = 9.1 Hz, 1H), 7.56 (dd, *J* = 2.1, 9.0 Hz, 1H), 8.27 (d, *J* = 2.2 Hz, 1H).

**$^{13}$C NMR (101 MHz, MeOD)** δ 25.40, 25.95, 33.59, 51.51, 52.24, 67.78, 115.88, 122.11, 128.06, 132.26, 134.69, 146.74, 170.87.

**MS (ESI)** m/z 334.2 $[M+H]^+$.

(4-(cyclohexylamino)-3-nitrophenyl)(4-methylpiperazin-1-yl)methanone **(8)**

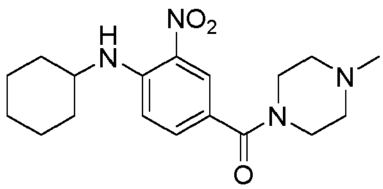

General procedure B was followed using compound **4** (1.863 g, 3.940 mmol), to provide (4-(cyclohexylamino)-3-nitrophenyl)(4-methylpiperazin-1-yl)methanone **8** (1.200 g, 3.460 mmol, 88 % yield).

**$^1$H NMR (400 MHz, $CDCl_3$)** δ 1.28 - 1.37 (m, 1H), 1.36 - 1.50 (m, 4H), 1.62 - 1.69 (m, 1H), 1.78 - 1.84 (m, 2H), 1.98 - 2.11 (m, 2H), 2.35 (s, 3H), 2.39 - 2.58 (m, 4H), 3.48 - 3.59 (m, 1H), 3.59 - 3.81 (m, 4H), 6.90 (d, *J* = 9.0 Hz, 1H), 7.54 (dd, *J* = 2.1, 8.9 Hz, 1H), 8.28 (d, *J* = 2.2 Hz, 2H).

**$^{13}$C NMR (101 MHz, $CDCl_3$)** δ 24.48, 25.49, 32.62, 45.93, 51.24, 54.90, 77.24, 114.39, 121.44, 126.97, 130.44, 135.58, 145.44, 168.66.

**MS (ESI)** m/z = 347.1 $[M+H]^+$.

(4-(cyclohexylamino)-3-nitrophenyl)(4-fluoropiperidin-1-yl)methanone **(9)**

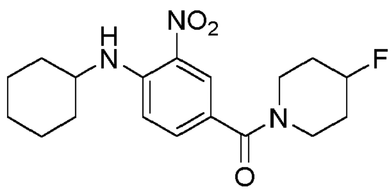

General procedure B was followed using compound **5** (0.137 g, 0.478 mmol), to provide ((4-(cyclohexylamino)-3-nitrophenyl)(4-fluoropiperidin-1-yl)methanone **9** (0.062 g, 0.177 mmol, 37 % yield).

**$^1$H NMR (400 MHz, $CDCl_3$)** δ 1.26 - 1.38 (m, 1H), 1.40 - 1.48 (m, 3H), 1.62 (s, 3H), 1.66 - 1.73 (m, 1H), 1.80 - 1.88 (m, 2H), 1.88 - 1.93 (m, 1H), 1.93 - 2.00 (m, 2H), 2.07 (dd, *J* = 10.1, 4.5 Hz, 2H), 3.51 - 3.60 (m, 1H), 3.60 - 3.71 (m, 2H), 3.79 (s, 1H), 4.85 - 5.04 (m, 1H), 6.93 (d, *J* = 9.0 Hz, 1H), 7.57 (dd, *J* = 9.0, 2.1 Hz, 1H), 8.31 (d, *J* = 2.2 Hz, 2H).

**$^{13}$C NMR (101 MHz, $CDCl_3$)** δ 24.48, 25.49, 31.20, 32.62, 51.25, 77.23, 77.34, 86.73, 88.43, 114.43, 121.51, 126.76, 130.44, 135.46, 145.46, 168.85.

**MS (ESI)** m/z = 350.1 $[M+H]^+$.

*tert*-butyl 4-(3-amino-4-(cyclohexylamino)benzoyl)piperazine-1-carboxylate **(10)**

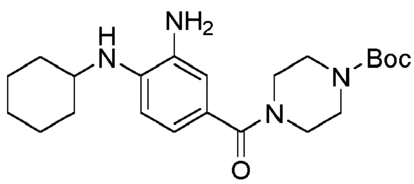

General procedure C was followed using compound **6** (1.730 g, 4.00 mmol), to provide *tert*-butyl 4-(3-amino-4-(cyclohexylamino)benzoyl)piperazine-1-carboxylate **10** (0.934 g, 2.320 mmol, 58 % yield).

**$^1$H NMR (400 MHz, MeOD)** δ 1.17 - 1.35 (m, 3H), 1.49 (s, 11H), 1.71 (dt, *J* = 12.8, 3.8 Hz, 1H), 1.82 (dt, *J* = 13.3, 3.8 Hz, 2H), 2.09 (dd, *J* = 12.6, 4.0 Hz, 2H), 3.36 (d, *J* = 3.6 Hz, 1H), 3.48 (s, 4H), 3.56 - 3.70 (m, 4H), 6.62 (d, *J* = 8.7 Hz, 1H), 6.77 - 6.96 (m, 2H).

**$^{13}$C NMR (101 MHz, MeOD)** δ 24.86, 25.72, 27.20, 32.83, 51.37, 80.22, 109.67, 115.18, 119.80, 122.07, 133.49, 138.54, 154.90, 172.77.

**MS (ESI)** m/z 403.4 [M+H]+.

(3-amino-4-(cyclohexylamino)phenyl)(morpholino)methanone **(CPD-002, 12)**

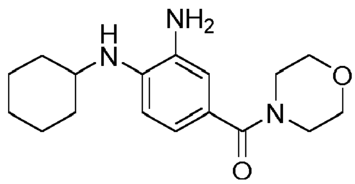

General procedure C was followed using compound **7** (1.760 g, 5.280 mmol), to provide (3-amino-4-(cyclohexylamino)phenyl)(morpholino)methanone **12** (0.911 g, 3.000 mmol, 56 % yield).

**$^1$H NMR (400 MHz, DMSO)** δ 1.10 - 1.26 (m, 3H), 1.26 - 1.42 (m, 2H), 1.61 (dt, *J* = 3.6, 12.6 Hz, 1H), 1.73 (dt, *J* = 3.7, 13.1 Hz, 2H), 1.96 (dd, *J* = 3.9, 12.8 Hz, 2H), 3.15 - 3.29 (m, 1H), 3.48 (q, *J* = 4.8 Hz, 4H), 3.56 (q, *J* = 4.8 Hz, 4H), 4.53 (d, *J* = 7.5 Hz, 1H), 4.69 (br. s, 1H), 6.41 (d, *J* = 8.2 Hz, 1H), 6.59 (dd, *J* = 2.0, 8.1 Hz, 1H), 6.65 (d, *J* = 2.0 Hz, 1H).

**$^{13}$C NMR (101 MHz, DMSO)** δ 24.53, 25.46, 32.54, 50.58, 66.09, 108.23, 113.65, 117.66, 121.87, 134.09, 136.32, 170.27.

**MS (ESI)** m/z = 304.2 $[M+H]^+$

**HRMS:** calcd for $C_{17}H_{25}N_3O_2$ 304.2020, found 304.2028 $[M + H]^+$.

(3-amino-4-(cyclohexylamino)phenyl)(4-methylpiperazin-1-yl)methanone **(CPD-003, 13)**

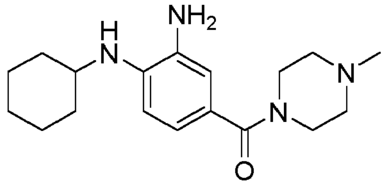

General procedure C was followed using compound **8** (1.200 g, 3.460 mmol), to provide (3-amino-4-(cyclohexylamino)phenyl)(4-methylpiperazin-1-yl)methanone **13** ( 0.394 g, 1.250 mmol, 36 % yield).

**$^1$H NMR (400 MHz, MeOD)** δ 1.17 - 1.34 (m, 3H), 1.42 (qt, *J* = 3.3, 12.7 Hz, 2H), 1.69 (dt, *J* = 3.8, 12.6 Hz, 1H), 1.80 (dt, *J* = 3.8, 13.3 Hz, 2H), 2.07 (dd, *J* = 3.9, 12.9 Hz, 2H), 2.31 (s, 3H), 2.44 (t, 4H), 3.35 (s, 1H), 3.66 (t, *J* = 4.9 Hz, 4H), 6.59 (d, *J* = 8.6 Hz, 1H), 6.77 - 6.84 (m, 2H).

**$^{13}$C NMR (101 MHz, MeOD)** δ 26.23, 27.10, 34.22, 46.02, 49.85, 52.74, 55.88, 111.07, 116.52, 121.03, 123.63, 134.92, 139.78, 173.78.

**MS (ESI)** m/z = 317.3 $[M+H]^+$.

**HRMS:** calcd for $C_{18}H_{28}N_4O_1$ 317.2336, found 317.2343 $[M + H]^+$.

(3-amino-4-(cyclohexylamino)phenyl)(4-fluoropiperidin-1-yl)methanone **(CPD-004, 14)**

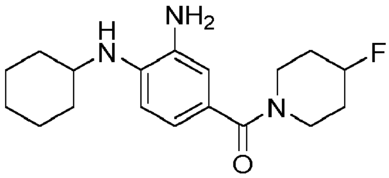

General procedure C was followed using compound **9** (0.062 g, 0.177 mmol), to provide (3-amino-4-(cyclohexylamino)phenyl)(4-fluoropiperidin-1-yl)methanone **14** (0.048 g, 0.150 mmol, 85 % yield).

**$^1$H NMR (400 MHz, MeOD)** δ 1.20 - 1.33 (m, 3H), 1.36 - 1.48 (m, 2H), 1.69 (dt, *J* = 3.7, 12.9 Hz, 1H), 1.81 (dt, *J* = 3.8, 13.1 Hz, 7H), 2.08 (dd, *J* = 3.7, 12.8 Hz, 2H), 3.67 (d, *J*= 8.1 Hz, 4H), 4.81 (tt, *J* = 3.3, 6.4 Hz, 1H), 6.60 (d, *J* = 8.7 Hz, 1H), 6.78 - 6.85 (m, 2H).

**$^{13}$C NMR (101 MHz, MeOD)** δ 24.86, 25.73, 30.97, 32.84, 51.40, 86.84, 88.54, 109.79, 114.96, 119.42, 122.57, 133.52, 138.35, 172.66.

**MS (ESI)** m/z = 320.2 $[M+H]^+$.

**HRMS:** calcd for $C_{18}H_{26}N_3O_1F_1$ 320.2133, found 320.2136 $[M + H]^+$.

(3-amino-4-(cyclohexylamino)phenyl)(piperazin-1-yl)methanone **(CPD-001, 15)**

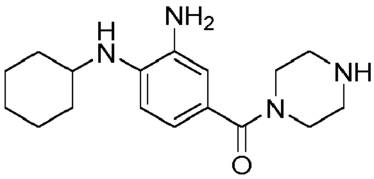

General procedure D was followed using compound **13** (0.100 g, 0.248 mmol), to provide (3-amino-4-(cyclohexylamino)phenyl)(piperazin-1-yl)methanone hydrochloride **15** (0.086 g, 0.284 mmol, 74 % yield).

**$^1$H NMR (400 MHz, MeOD)** δ 1.17- 1.30 (m, 1H), 1.30- 1.46 (m, 4H), 1.67 (d, *J* = 11.4 Hz, 1H), 1.78 - 1.86 (m, 2H), 2.01 - 2.09 (m, 2H), 3.38 - 3.52 (m, 1H), 3.85 (t, *J* = 5.2 Hz, 4H), 7.03 (d, *J* = 8.4 Hz, 1H), 7.20 (d, *J*= 8.5 Hz, 1H), 7.25 - 7.30 (m, 1H), 8.10 (t, *J* = 7.0 Hz, 1H), 8.65 (tt, *J* = 1.6, 8.0 Hz, 1H), 8.86 (d, *J* = 5.8 Hz, 1H).

**$^{13}$C NMR (101 MHz, MeOD)** δ 25.84, 26.51, 32.52, 44.42, 55.83, 122.60, 125.89, 128.76, 143.03, 148.23, 171.73.

**MS (ESI)** m/z = 303.2 $[M+H]^+$.

**HRMS:** calcd for $C_{17}H_{26}N_4O_1$ 303.2179, found 303.2191 $[M + H]^+$.

4-(cyclohexylamino)-3-nitrobenzoic acid **(17)**

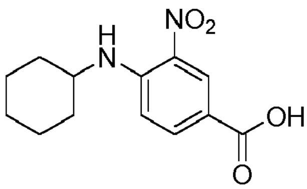

General procedure E was followed using compound **16** (2.250 g, 12.15 mmol), to provide crude 4-(cyclohexylamino)-3-nitrobenzoic acid **17** (3.210 g, 12.15 mmol, 100 % yield). The compound was used for the next step without further purification.

**MS (ESI)** m/z = 265 $[M+H]^+$.

*tert*-butyl 4-(cyclohexylamino)-3-nitrobenzoate **(18)**

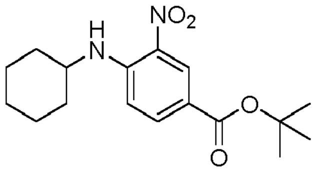

General procedure F was followed using compound **17** (3.210 g, 12.15 mmol), to provide *tert*-butyl 4-(cyclohexylamino)-3-nitrobenzoate **18** (3.750 g, 11.70 mmol, 96 % yield).

**$^1$H NMR (400 MHz, $CDCl_3$)** δ 1.03 - 1.19 (m, 3H), 1.26 - 1.35 (m, 2H), 1.58 (s, 9H), 1.81 (dd, *J* = 4.4, 9.1 Hz, 2H), 1.91 (dd, *J* = 4.0, 12.2 Hz, 2H), 2.06 (d, *J* = 9.9 Hz, 1H), 3.51 - 3.61 (m, 1H), 4.41 (br. s, 1H), 6.86 (d, *J*= 9.1 Hz, 1H), 7.97 (ddd, *J* = 0.7, 2.1, 9.2 Hz, 1H), 8.38 (d, *J*= 7.5 Hz, 1H), 8.79 (d, *J* = 2.1 Hz, 1H).

**$^{13}$C NMR (101 MHz, $CDCl_3$)** δ 25.05, 25.62, 28.40, 32.71, 51.47, 81.31, 113.80, 118.78, 129.65, 131.09, 136.32, 146.82, 164.59.

**MS (ESI)** m/z = 321 $[M+H]^+$.

*tert*-butyl 3-amino-4-(cyclohexylamino)benzoate **(19)**

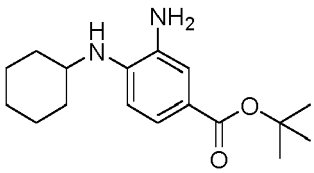

General procedure C was followed using compound **18** (3.750 g, 11.70 mmol), to provide *tert*-butyl 3-amino-4-(cyclohexylamino)benzoate **19** (1.230 g, 4.24 mmol, 36 % yield).

**$^1$H NMR (400 MHz, MeOD)** δ 1.19 - 1.35 (m, 3H), 1.37 - 1.51 (m, 2H), 1.55 (s, 9H), 1.65 - 1.74 (m, 1H), 1.81 (dt, *J* = 3.7, 13.1 Hz, 2H), 2.03 - 2.13 (m, 2H), 3.32 - 3.39 (m, 0H), 6.55 (dd, *J* = 0.6, 8.4 Hz, 1H), 7.30 (d, *J* = 2.0 Hz, 1H), 7.35 (dd, *J* = 2.1, 8.4 Hz, 1H).

**$^{13}$C NMR (101 MHz, DMSO)** δ 26.23, 27.07, 28.61, 34.21, 52.63, 80.89, 110.07, 118.29, 120.19, 124.16, 133.71, 142.39, 168.80.

**MS (ESI)** m/z =291.0 $[M+H]^+$.

*tert*-butyl 4-(cyclohexylamino)-3-(cyclopentylamino)benzoate **(20)**

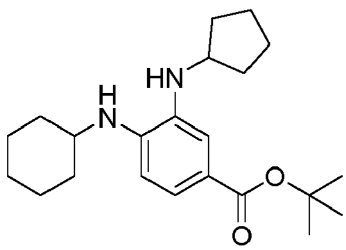

General procedure G was followed using compound **19** (1.000 g, 3.440 mmol) and cyclopentanone (0,915 ml, 10,33 mmol) as the corresponding ketone to provide *tert*-butyl 4-(cyclohexylamino)-3-(cyclopentylamino)benzoate **20** (1.150 g, 3.210 mmol, 91% yield).

**$^1$H NMR (400 MHz, MeOD)** δ 1. 1.19 - 1.36 (m, 6H), 1.38 - 1.47 (m, 2H), 1.56 (s, 9H), 1.60 - 1.72 (m, 2H), 1.72 - 1.85 (m, 4H), 1.98 - 2.11 (m, 4H), 3.32 - 3.39 (m, 1H), 3.78 (ddd, *J* = 5.4, 6.9, 12.3 Hz, 1H), 6.54 (d, *J* = 8.5 Hz, 1H), 7.23 (d, *J* = 2.0 Hz, 1H), 7.35 (dd, *J* = 2.0, 8.4 Hz, 1H).

**$^{13}$C NMR (101 MHz, MeOD)** δ 25.35, 26.31. 27.09, 28.63, 34.14, 34.17, 52.76, 56.37, 80.85, 109.62, 115.10, 120.12, 123.28, 135.64, 142.75, 169.13.

**MS (ESI)** m/z =359.0 $[M+H]^+$.

*tert*-butyl 3,4-bis(cyclohexylamino)benzoate **(21)**

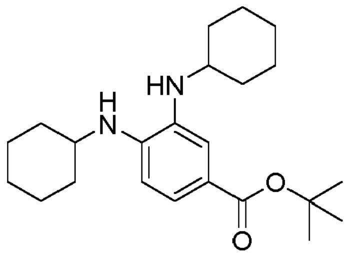

General procedure G was followed using compound **19** (4.740 g, 16.32 mmol) and cyclohexanone (5,07 ml, 49,0 mmol) as the corresponding ketone to provide *tert*-butyl 3,4-bis(cyclohexylamino)benzoate **21** (5.520 g, 14.82 mmol, 91% yield).

**$^1$H NMR(400 MHz, MeOD)** δ 0.87-0.93 (m, 1H), 1.18 - 1.50 (m, 12H), 1.56 (s, 9H), 1.69 (dt, *J* = 3.5, 12.3 Hz, 2H), 1.75 - 1.85 (m, 4H), 2.05 (dt, *J* = 3.9, 12.2 Hz, 5H), 3.15 (tt, *J* = 3.7, 10.5 Hz, 1H), 3.32 - 3.37 (m, 1H), 6.55 (d, *J* = 8.5 Hz, 1H), 7.24 (d, *J* = 2.0 Hz, 1H), 7.35 (dd, *J* = 2.0, 8.4 Hz, 1H).

**$^{13}$C NMR (101 MHz, MeOD)** δ 26.24, 26.44, 27.08, 27.24, 28.61, 34.18, 34.40, 52.70, 54.14, 80.90, 110.04, 116.26, 120.11, 123.68, 134.65, 143.33, 169.05.

**MS (ESI)** m/z =373.0 $[M+H]^+$.

*tert*-butyl 3-(benzylamino)-4-(cyclohexylamino)benzoate **(22)**

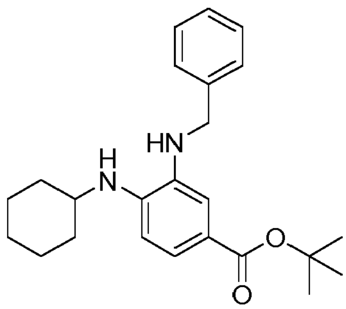

General procedure G was followed using compound **19** (2.000 g, 6.890 mmol) and benzaldehyde (2.108 ml, 20.66 mmol) as the corresponding aldehyde to provide *tert*-butyl 3-(benzylamino)-4-(cyclohexylamino)benzoate **22** (0.780 g, 2.050 mmol, 30% yield).

**$^1$H NMR (400 MHz, MeOD)** δ 1.14 - 1.35 (m, 3H), 1.35 - 1.46 (m, 2H), 1.50 (s, 9H), 1.68 (dd, *J* = 6.6, 10.5 Hz, 1H), 1.80 (dt, *J* = 3.7, 12.7 Hz, 2H), 2.07 (dd, *J* = 3.8, 13.1 Hz, 2H), 3.31 - 3.41 (m, 1H), 4.32 (s, 2H), 6.55 (d, *J* = 8.4 Hz, 1H), 7.12 (d, *J*= 2.0 Hz, 1H), 7.19 - 7.26 (m, 1H), 7.28 - 7.34 (m, 3H), 7.38 (d, *J* = 7.1 Hz, 2H).

**$^{13}$C NMR (101 MHz, MeOD)** δ 13.02, 24.85, 25.27, 25.68, 27.19, 27.39, 32.80, 48.10, 51.33, 79.40, 108.30, 112.82, 118.84, 121.80, 126.53, 127.26, 128.03, 134.40, 139.82, 140.89, 167.58.

**MS (ESI)** m/z = 381.4 $[M+H]^+$.

*tert*-butyl 4-(cyclohexylamino)-3-((4-fluorobenzyl)amino)benzoate **(23)**

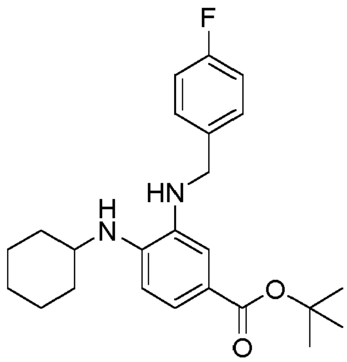

General procedure G was followed using compound **19** (1.230 g, 4.240 mmol) and 4-fluorobenzaldehyde (0.454 ml, 4.240 mmol) as the corresponding aldehyde to provide *tert*-butyl 4-(cyclohexylamino)-3-((4-fluorobenzyl)amino) benzoate **23** (0.893 g, 2.241 mmol, 53 % yield).

**$^1$H NMR (400 MHz, DMSO)** δ 1.11 - 1.23 (m, 2H), 1.23 - 1.41 (m, 3H), 1.44 (s, 9H), 1.62 (dt, *J* = 4.2, 15.3 Hz, 1H), 1.74 (dt, *J* = 3.7, 13.4 Hz, 2H), 1.99 (d, *J* = 3.8 Hz, 2H), 3.26 - 3.33 (m, 1H), 4.28 (d, *J* = 5.3 Hz, 2H), 5.08 (d, *J* = 7.3 Hz, 1H), 5.50 (t, *J* = 5.5 Hz, 1H), 6.47 (d, *J* = 8.4 Hz, 1H), 6.87 (d, *J* = 2.0 Hz, 1H), 7.10 - 7.20 (m, 3H), 7.35 - 7.44 (m, 2H).

**$^{13}$C NMR (101 MHz, DMSO)** δ 24.68, 25.60, 27.97, 32.49, 32.52, 46.33, 46.41, 50.76, 50.85, 78.54, 107.72, 110.61, 110.67, 114.99 (d, *J*= 21.3 Hz), 118.14, 118.17, 120.58, 120.62, 129.10 (d, *J*= 8.0 Hz), 133.86, 133.92, 136.10 (d, *J*= 2.9 Hz), 139.36, 139.42, 161.15 (d, *J*= 241.9 Hz), 165.74.

**MS (ESI) m/z** = 399.4 [M+H]+.

4-(cyclohexylamino)-3-(cyclopentylamino)benzoic acid **(24)**

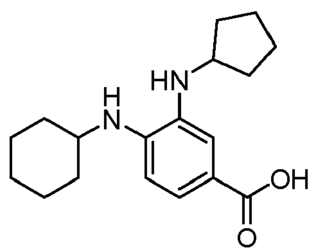

General procedure H was followed using compound **20** (1.348 g, 3.760 mmol), to provide 4-(cyclohexylamino)-3-((4-fluorobenzyl)amino)benzoic acid **24** (1.093 g, 3.610 mmol, 96 % yield).

**$^1$H NMR (400 MHz, DMSO)** δ 1.11 - 1.28 (m, 4H), 1.35 (qt, *J* = 3.2, 12.5 Hz, 2H), 1.47 - 1.78 (m, 8H), 1.85 - 2.01 (m, 4H),3.31 (ddt, *J* = 3.8, 7.4, 10.6 Hz, 1H), 3.73 (p, *J* = 6.3 Hz, 1H), 6.55 (d, *J*= 8.5 Hz, 1H), 7.18 (s, 1H), 7.32 (d, *J*= 8.4 Hz, 1H).

**$^{13}$C NMR (101 MHz, DMSO)** δ, 23.91, 24.74, 25.57, 31.89, 32.46, 51.03, 55.55, 108.78, 112.90, 117.50, 122.88, 131.14, 140.26, 167.85.

**MS (ESI)** m/z = 303.2 $[M+H]^+$.

3,4-bis(cyclohexylamino)benzoic acid **(25)**

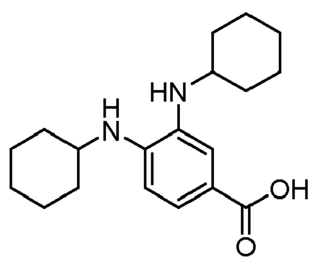

General procedure H was followed using compound **21** (2.940 g, 7.890 mmol), to provide 4-(cyclohexylamino)-3-((4-fluorobenzyl)amino)benzoic acid **25** (2.219 g, 7.010 mmol, 89 % yield).

**$^1$H NMR (400 MHz, MeOD)** δ 1.22 - 1.54 (m, 11H), 1.72 (dt, *J* = 3.6, 13.2 Hz, 2H), 1.78 - 1.91 (m, 3H), 1.98 - 2.12 (m, 5H), 3.37 - 3.54 (m, 2H), 6.93 (d, *J* = 8.7 Hz, 1H), 7.76 (s, 1H), 7.86 (d, *J*= 8.7 Hz, 1H).

**$^{13}$C NMR (101 MHz, MeOD)** δ 25.68, 26.00, 26.14, 26.77, 31.03, 33.55, 53.23, 60.62, 113.86, 116.53, 119.43, 126.67, 132.03, 145.07, 169.08.

**MS (ESI)** m/z = 317.3 $[M+H]^+$.

3-(benzylamino)-4-(cyclohexylamino)benzoic acid **(26)**

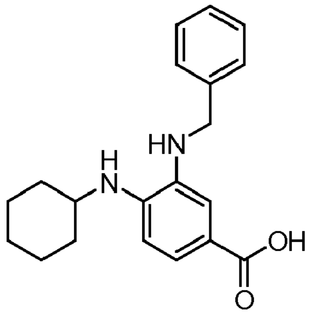

General procedure H was followed using compound **22** (0.790 g, 2.080 mmol), to provide 4-(cyclohexylamino)-3-((4-fluorobenzyl)amino)benzoic acid **26** (0.800 g, 2.460 mmol, 88 % yield).

**$^1$H NMR (400 MHz, DMSO)** δ 1.23 - 1.26 (m, 2H), 1.29 - 1.41 (m, 2H), 1.56 - 1.65 (m, 1H), 1.69 - 1.74 (m, 2H), 1.81 - 1.93 (m, 1H), 1.98 (dd, *J* = 3.6, 12.5 Hz, 2H), 2.90 - 3.04 (m, 1H), 3.33 (td, *J* = 3.3, 6.8 Hz, 1H), 4.31 (s, 2H), 6.53 (d, *J* = 8.4 Hz, 1H), 6.97 (d, *J* = 2.0 Hz, 1H), 7.21 - 7.27 (m, 2H), 7.34 (td, *J*= 6.1, 8.3 Hz, 4H), 7.72 (s, 1H).

**$^{13}$C NMR (101 MHz, DMSO)** δ 24.68, 25.57, 32.46, 40.15, 47.27, 51.05, 111.40, 114.43, 117.34, 121.26, 126.83, 127.40, 128.32, 133.69, 136.54, 139.50, 167.91.

**MS (ESI)** m/z = 325.0 $[M+H]^+$.

4-(cyclohexylamino)-3-((4-fluorobenzyl)amino)benzoic acid **(27)**

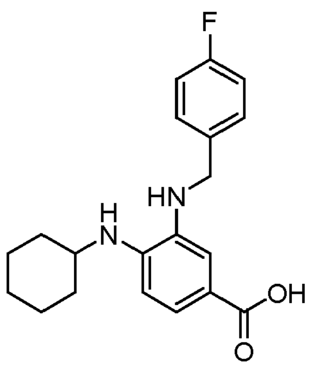

General procedure H was followed using compound **23** (0.890 g, 2.230 mmol), to provide 4-(cyclohexylamino)-3-((4-fluorobenzyl)amino)benzoic acid **27** (1.000 g, 2.190 mmol, 97 % yield).

**$^1$H NMR (400 MHz, MeOD)** δ 1.20 - 1.34 (m, 3H), 1.34 - 1.50 (m, 3H), 1.80 - 1.88 (m, 2H), 2.01 -2.10 (m, 2H), 3.44 (ddt, *J* = 3.8, 7.4, 10.5 Hz, 1H), 4.42 (s, 2H), 6.84 (d, *J* = 8.5 Hz, 1H), 7.03 - 7.13 (m, 2H), 7.34 - 7.43 (m, 3H), 7.57 (dd, *J* = 1.9, 8.4 Hz, 1H).

**MS (ESI)** m/z = 343.2 $[M+H]^+$.

*tert*-butyl 4-(4-(cyclohexylamino)-3-(cyclopentylamino)benzoyl)piperazine-1-carboxylate **(28)**

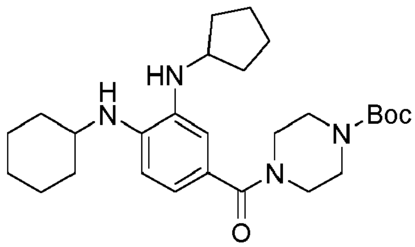

General procedure I was followed using compound **24** (0.236 g, 0.780 mmol) and *tert*-butyl piperazine-1-carboxylate (0.203 g, 1.093 mmol) as the corresponding amine to provide tert-butyl 4-(4-(cyclohexylamino)-3-(cyclopentylamino)benzoyl)piperazine-1-carboxylate **28** (0.148 g, 0.314 mmol, 40 % yield).
**$^1$H NMR (400 MHz, MeOD)** δ 1.18 - 1.33 (m, 3H), 1.35 - 1.44 (m, 2H), 1.47 (s, 9H), 1.52 - 1.72 (m, 3H), 1.73 - 1.85 (m, 3H), 1.96 - 2.11 (m, 4H), 3.24 - 3.30 (m, 1H), 3.48 (d, *J* = 6.0 Hz, 3H), 3.63 (dd, *J* = 3.8, 6.7 Hz, 4H), 3.78 (p, *J*= 6.3 Hz, 1H), 6.61 (d, *J*= 8.2 Hz, 1H), 6.71 (d, *J*= 1.9 Hz, 1H), 6.80 (dd, *J* = 1.9, 8.1 Hz, 1H).
**$^{13}$C NMR (101 MHz, MeOD)** δ 25.30, 26.34, 27.14, 28.61, 30.90, 34.16, 34.22, 53.00, 56.10, 81.64, 110.91, 113.11, 119.97, 123.74, 126.13, 136.77, 140.07, 156.31, 174.58.
**MS (ESI)** m/z = 471.5 $[M+H]^+$.

(4-(cyclohexylamino)-3-(cyclopentylamino)phenyl)(morpholino)methanone **(CPD-006, 29)**

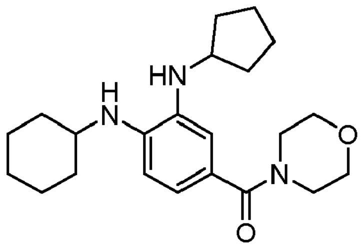

General procedure I was followed using compound **24** (0.150 g, 0.496 mmol) and morpholine (0.060 g, 0.694 mmol) as the corresponding amine to provide (4-(cyclohexylamino)-3-(cyclopentylamino)phenyl)(morpholino)methanone **29** (0.150g, 0.404 mmol, 81 % yield).
**$^1$H NMR (400 MHz, MeOD)** δ 1.18 - 1.34 (m, 3H), 1.42 (qt, *J* = 3.3, 12.7 Hz, 2H), 1.52 - 1.74 (m, 2H), 1.74-1.84 (m, 4H), 1.96 - 2.11 (m, 4H), 3.24 - 3.29 (m, 1H), 3.66 (dd, *J* = 4.0, 7.2 Hz, 9H), 3.72 - 3.84 (m, 1H), 6.60 (d, *J* = 8.2 Hz, 1H), 6.70 (d, *J* = 2.0 Hz, 1H), 6.79 (dd, *J* = 2.0, 8.1 Hz, 1H).
**$^{13}$C NMR (101 MHz, MeOD)** δ 25.30, 26.34, 27.14, 34.16, 34.22, 53.00, 56.09, 67.96, 110.94, 113.10, 119.91, 123.67, 136.80, 139.99, 174.38.
**MS (ESI)** m/z = 372.3 $[M+H]^+$.
**HRMS:** calcd for $C_{22}H_{33}N_3O_2$ 372.2646, found 372.2653 $[M + H]^+$.

(4-(cyclohexylamino)-3-(cyclopentylamino)phenyl)(4-methylpiperazin-1-yl)methanone **(CPD-007, 30)**

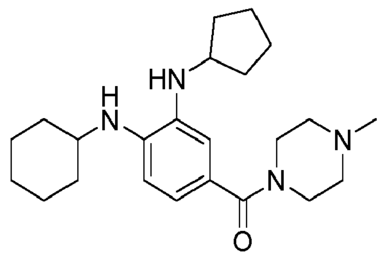

General procedure I was followed using compound **24** (0.100 g, 3.310 mmol) and 1-methylpiperazine (0.046 g, 0.463 mmol) as the corresponding amine to provide (4-(cyclohexylamino)-3-(cyclopentylamino)phenyl)(4-methylpiperazin-1-yl)methanone **30** (0.120 g, 0.312 mmol, 94 % yield).

**$^1$H NMR (400 MHz, MeOD)** δ 1.26 (ddt, *J* = 6.6, 12.3, 15.1 Hz, 3H), 1.36 - 1.48 (m, 3H), 1.51 - 1.73 (m, 3H), 1.74 - 1.85 (m, 3H), 1.96 - 2.12 (m, 4H), 2.35 (s, 3H), 2.50 (t, *J* = 5.1 Hz, 4H), 3.24 - 3.29 (m, 0H), 3.64 - 3.73 (m, 4H), 3.75 - 3.81 (m, 1H), 6.60 (d, *J*= 8.2 Hz, 1H), 6.69 (d, *J*= 1.9 Hz, 1H), 6.78 (dd, *J* = 1.9, 8.1 Hz, 1H).

**$^{13}$C NMR (101 MHz, MeOD)** δ 25.30, 26.35, 27.14, 34.17, 34.23, 45.98, 53.01, 55.92, 56.09, 110.94, 113.04, 119.86, 123.82, 136.77, 139.98, 174.32.

**MS (ESI)** m/z = 385.4 $[M+H]^+$.

**HRMS:** calcd for $C_{23}H_{36}N_4O_1$ 385.2962, found 385.2969 $[M + H]^+$.

*tert*-butyl 4-(3,4-bis(cyclohexylamino)benzoyl)piperazine-1-carboxylate (31)

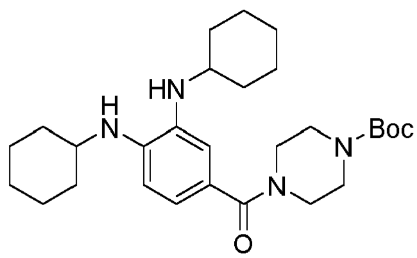

General procedure I was followed using compound **25** (0.300 g, 0.948 mmol) and *tert*-butyl piperazine-1-carboxylate (0.247 g, 1.327 mmol) as the corresponding amine to provide tert-butyl 4-(3,4-bis(cyclohexylamino)benzoyl) piperazine-1-carboxylate **31** (0.202 g, 0.417 mmol, 44 % yield).

**$^1$H NMR (400 MHz, MeOD)** δ 1.19 - 1.47 (m, 9H), 1.49 (s, 9H), 1.71 (dt, *J* = 3.7, 12.7 Hz, 3H), 1.82 (dt, *J* = 3.8, 13.6 Hz, 5H), 2.07 (dt, *J* = 4.1, 11.1 Hz, 5H), 3.20 (tt, *J* = 3.7, 10.5 Hz, 1H), 3.29 (dt, *J* = 3.6, 10.4 Hz, 1H), 3.49 (d, *J* = 6.0 Hz, 4H), 3.64 (dd, *J* = 3.8, 6.7 Hz, 4H), 6.63 (d, *J* = 8.2 Hz, 1H), 6.74 (d, *J*= 1.9 Hz, 1H), 6.82 (dd, *J*= 1.9, 8.1 Hz, 1H).

**$^{13}$C NMR (101 MHz, MeOD)** δ 26.28, 26.38, 27.14, 27.20, 28.61, 30.90, 34.23, 34.37, 52.98, 53.64, 81.63, 111.39, 113.86, 120.17, 123.81, 126.12, 135.94, 140.44, 156.32, 174.49.

**MS (ESI)** m/z = 485.5 $[M+H]^+$.

(3,4-bis(cyclohexylamino)phenyl)(morpholino)methanone **(CPD-009 32)**

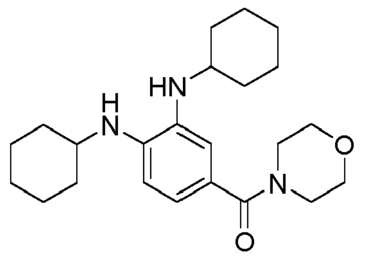

General procedure I was followed using compound **25** (0.200 g, 0.632 mmol) and morpholine (0.077 g, 0.885

mmol) as the corresponding amine to provide bis(cyclohexylamino)phenyl)(morpholino)methanone **32** (0.048 g, 0.126 mmol, 20 % yield).

**$^1$H NMR (400 MHz, MeOD)** δ 1.14 - 1.33 (m, 7H), 1.34 - 1.48 (m, 5H), 1.69 (dt, *J* = 3.6, 12.9 Hz, 2H), 1.78 - 1.86 (m, 4H), 2.05 (dt, *J* = 3.7, 12.7 Hz, 5H), 3.18 (tt, *J* = 3.7, 10.5 Hz, 1H), 3.24 - 3.30 (m, 0H), 3.67 (h, *J* = 4.2 Hz, 8H), 6.61 (d, *J*= 8.2 Hz, 1H), 6.71 (d, *J* = 2.0 Hz, 1H), 6.79 (dd, *J* = 2.0, 8.1 Hz, 1H).

**$^{13}$C NMR (101 MHz, MeOD)** δ 26.29, 26.38, 27.14, 27.21, 34.23, 34.37, 52.99, 53.66, 67.95, 111.41, 113.86, 120.11, 123.75, 135.98, 140.35, 174.28.

**MS (ESI)** m/z = 386.4 $[M+H]^+$.

**HRMS:** calcd for $C_{23}H_{35}N_3O_2$ 386.2802, found 386.2802 $[M + H]^+$.

(3,4-bis(cyclohexylamino)phenyl)(4-methylpiperazin-1-yl)methanone **(CPD-010, 33)**

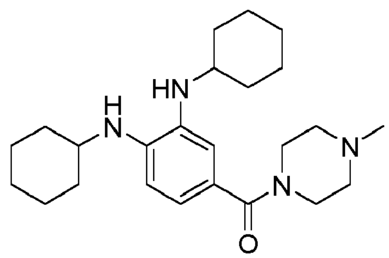

General procedure I was followed using compound **25** (0.200 g, 0.632 mmol) and 1-methylpiperazine (0.089 g, 0.885 mmol) as the corresponding amine to provide (3,4-bis(cyclohexylamino)phenyl)(4-methylpiperazin-1-yl)methanone **33** (0.046 g, 0.115 mmol, 18 % yield).

**$^1$H NMR (400 MHz, MeOD)** δ 1.17-1.33 (m, 5H), 1.34-1.50 (m, 3H), 1.63-1.74 (m, 2H), 1.80 (dp, *J* = 3.6, 10.8 Hz, 4H), 2.05 (dt, *J* = 3.6, 12.8 Hz, 4H), 2.33 (s, 3H), 2.48 (t, *J* = 5.1 Hz, 4H), 3.17 (ddd, *J* = 3.7, 6.8, 10.5 Hz, 1H), 3.24 - 3.31 (m, 1H), 3.64 - 3.71 (m, 4H), 6.61 (d, *J* = 8.2 Hz, 1H), 6.70 (d, *J*= 1.9 Hz, 1H), 6.78 (dd, *J* = 1.9, 8.1 Hz, 1H).

**$^{13}$C NMR (101 MHz, MeOD)** δ 26.29, 26.39, 27.14, 27.21, 34.24, 34.36, 46.00, 52.99, 53.66, 55.93, 111.42, 113.77, 120.05, 123.91, 135.94, 140.32, 174.22.

**MS (ESI)** m/z = 399.4 $[M+H]^+$.

**HRMS:** calcd for $C_{24}H_{38}N_4O_1$ 399.3118, found 399.3124 $[M + H]^+$.

(3,4-bis(cyclohexylamino)phenyl)(4-fluoropiperidin-1-yl)methanone **(CPD-011, 34)**

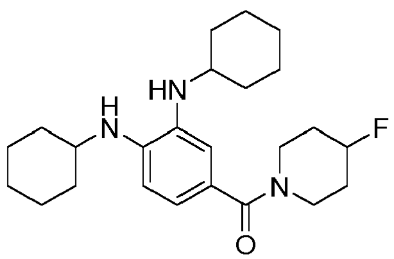

General procedure I was followed using compound **25** (0.200 g, 0.632 mmol) and 4-fluoropiperidine (0.091 g, 0.885 mmol) as the corresponding amine to provide (3,4-bis(cyclohexylamino)phenyl)(4-fluoropiperidin-1-yl)methanone **34** (0.088 g, 0.219 mmol, 35 % yield).

**$^1$H NMR (400 MHz, MeOD)** δ 1.17 - 1.51 (m, 8H), 1.69 (dt, *J* = 3.5, 15.7 Hz, 2H), 1.74 - 1.86 (m, 5H), 1.85-1.99 (m, 0H), 2.05 (dt, *J* = 3.7, 12.5 Hz, 4H), 3.18 (tt, *J* = 3.7, 10.5 Hz, 1H), 3.26 (dd, *J* = 3.7, 10.4 Hz, 0H), 3.69 (t, *J* = 5.9 Hz, 4H), 4.81 (tt, *J* = 3.3, 6.5 Hz, 0H), 4.93 (tt, *J* = 3.3, 6.5 Hz, 0H), 6.62 (d, *J*= 8.2 Hz, 1H), 6.71 (d, *J*= 1.9 Hz, 1H), 6.79 (dd, *J* = 1.9, 8.1 Hz, 1H).

**$^{13}$C NMR (101 MHz, MeOD)** δ 26.29, 26.38, 27.14, 27.20, 32.51, 32.70, 34.24, 34.36, 53.02, 53.65, 88.26, 89.95, 111.55, 113.59, 119.82, 124.29, 135.96, 140.25, 174.36.

**MS (ESI)** m/z = 402.4 $[M+H]^+$.

**HRMS:** calcd for $C_{24}H_{36}N_3O_1F_1$ 402.2915, found 402.2923 $[M + H]^+$.

*tert*-butyl (1-(3,4-bis(cyclohexylamino)benzoyl)pyrrolidin-3-yl)carbamate **(35)**

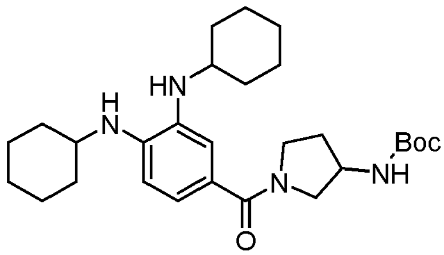

General procedure I was followed using compound **25** (0.150 g, 0.474 mmol) and *tert*-butyl pyrrolidin-3-ylcarbamate (0.124 g, 0.664 mmol) as the corresponding amine to provide *tert*-butyl (1-(3,4-bis(cyclohexylamino) benzoyl)pyrrolidin-3-yl)carbamate **35** (0.089 g, 0.184 mmol, 39 % yield).

**$^1$H NMR (400 MHz, MeOD)** δ 1.24 (ttt, *J* = 12.7, 6.9, 3.6 Hz, 6H), 1.54 - 1.33 (m, 13H), 1.73 - 1.63 (m, 2H), 1.79 (dt, *J* = 13.4, 3.7 Hz, 4H), 1.95 - 1.84 (m, 1H), 2.05 (dt, *J* = 12.7, 3.5 Hz, 4H), 2.23 - 2.10 (m, 1H), 3.18 (tt, *J* = 10.5, 3.7 Hz, 1H), 3.27 (dt, *J* = 10.3, 3.6 Hz, 1H), 3.49 - 3.37 (m, 1H), 3.84 - 3.55 (m, 3H), 4.21 - 3.96 (m, 1H), 6.59 (d, *J*= 8.3 Hz, 1H), 6.86 (d, *J* = 6.1 Hz, 1H), 6.97 - 6.88 (m, 1H).

**MS (ESI)** m/z = 485.4 $[M+H]^+$.

*tert*-butyl 4-(3-(benzylamino)-4-(cyclohexylamino)benzoyl)piperazine-1-carboxylate **(36)**

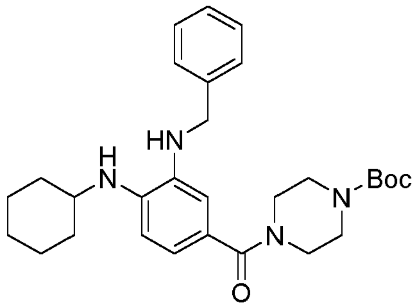

General procedure I was followed using compound **26** (0.200 g, 0.438 mmol) and *tert*-butyl piperazine-1-carboxylate (0.114 g, 0.613 mmol) as the corresponding amine to provide *tert*-butyl 4-(3-(benzylamino)-4-(cyclohexylamino)benzoyl)piperazine-1-carboxylate **36** (0.125 g, 0.254 mmol, 58 % yield).

**$^1$H NMR (400 MHz, $CDCl_3$)** δ 1.30 (dt, *J* = 13.3, 46.6 Hz, 6H), 1.47 (s, 9H), 1.64 (q, *J*= 14.8 Hz, 4H), 1.78 (d, *J*= 12.7 Hz, 3H), 2.07 (d, *J*= 11.9 Hz, 2H), 3.41 (d, *J*= 63.0 Hz, 10H), 4.32 (s, 2H), 6.72 (s, 2H), 6.90 (d, *J* = 8.0 Hz, 1H), 7.30 (dt, *J* = 3.0, 7.3 Hz, 1H), 7.32 - 7.44 (m, 4H).

**MS (ESI)** m/z = 493.4 $[M+H]^+$.

(3-(benzylamino)-4-(cyclohexylamino)phenyl)(morpholino)methanone **(CPD-014, 37)**

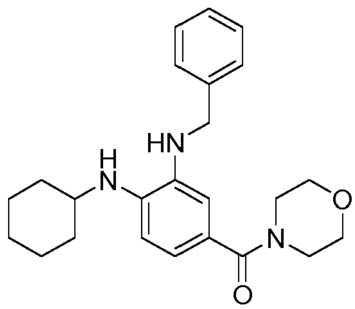

General procedure I was followed using compound **26** (0.200 g, 0.438 mmol) and morpholine (0.074 ml, 0.863 mmol) as the corresponding amine to provide 3-(benzylamino)-4-(cyclohexylamino)phenyl)(morpholino)methanone **37** (0.081 g, 0.206 mmol, 33 % yield).

**$^1$H NMR (400 MHz, DMSO)** δ 1.22 (q, *J* = 11.4 Hz, 4H), 1.36 (q, *J* = 12.7 Hz, 2H), 1.63 (d, *J* = 12.9 Hz, 1H), 1.74 (d, *J* = 12.9 Hz, 2H), 2.00 (d, *J* = 12.1 Hz, 2H), 3.28 (s, 6H), 3.39 (s, 4H), 4.32 (d, *J* = 5.4 Hz, 2H), 4.80 (d, *J* = 7.2 Hz, 1H), 5.54 (t, *J* = 5.7 Hz, 1H), 6.29 (s, 1H), 6.48 (d, *J* = 8.1 Hz, 1H), 6.64 (d, *J* = 8.0 Hz, 1H), 7.23 (h, *J* = 5.0 Hz, 1H), 7.33 (d, *J* = 4.4 Hz, 4H).

**$^{13}$C NMR (101 MHz, DMSO)** δ 24.71, 25.66, 32.69, 46.71, 50.93, 66.10, 108.37, 109.72, 118.08, 121.98, 126.60, 126.95, 128.32, 134.03, 136.79, 139.93, 170.47.

**MS (ESI)** m/z = 394.4 $[M + H]^+$.

**HRMS:** calcd for $C_{24}H_{31}N_3O_2$ 394.2489, found 394.2498 $[M + H]^+$.

(3-(benzylamino)-4-(cyclohexylamino)phenyl)(4-methylpiperazin-1-yl)methanone **(CPD-015, 38)**

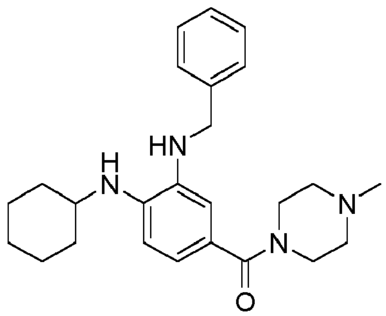

General procedure I was followed using compound **26** (0.200 g, 0.438 mmol) and 1-methylpiperazine (0.061 g, 0.613 mmol) as the corresponding amine to provide (3-(benzylamino)-4-(cyclohexylamino)phenyl)(4-methylpiperazin-1-yl)methanone **38** (0.093 g, 0.229 mmol, 52 % yield).

**$^1$H NMR (400 MHz, MeOD)** δ 1.31 (td, *J* = 3.4, 11.6 Hz, 3H), 1.37 - 1.52 (m, 2H), 1.70 (s, 1H), 1.83 (dt, *J* = 3.7, 13.0 Hz, 2H), 2.12 (dd, *J* = 3.8, 12.5 Hz, 2H), 2.27 (s, 5H), 3.34 - 3.57 (m, 4H), 4.41 (s, 2H), 6.43 (d, *J* = 1.9 Hz, 1H), 6.65 (d, *J* = 8.2 Hz, 1H), 6.80 (dd, *J* = 1.9, 8.1 Hz, 1H), 7.19 - 7.27 (m, 1H), 7.28 - 7.39 (m, 4H).

**$^{13}$C NMR (101 MHz, MeOD)** δ 24.88, 25.75, 32.89, 44.55, 47.30, 51.54, 54.37, 109.76, 110.86, 118.70, 122.31, 126.49, 126.74, 128.18, 134.81, 138.18, 139.78, 172.67.

**MS (ESI)** m/z = 407.2 $[M+H]^+$.

**HRMS:** calcd for $C_{25}H_{34}N_4O_1$ 407.2805 found 407.2814 $[M + H]^+$.

(3-(benzylamino)-4-(cyclohexylamino)phenyl)(4-fluoropiperidin-1-yl)methanone **(CPD-016, 39)**

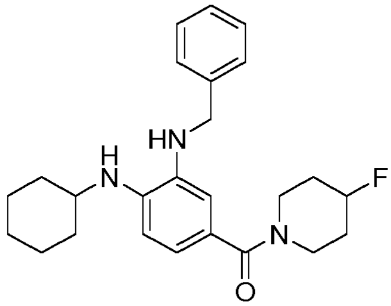

General procedure I was followed using compound **26** (0.300 g, 0.948 mmol) and 4-fluoropiperidine (0.063 g, 0.613 mmol) as the corresponding amine to provide (3-(benzylamino)-4-(cyclohexylamino)phenyl)(4-fluoropiperidin-1-yl) methanone **39** (0.038 g, 0.093 mmol, 21 % yield).

**$^{1}$H NMR (400 MHz, MeOD)** δ 1.13 - 1.27 (m, 4H), 1.35 (t, *J* = 12.5 Hz, 2H), 1.51 (s, 2H), 1.63 (d, *J* = 10.9 Hz, 2H), 1.70 - 1.78 (m, 3H), 2.00 (d, *J* = 12.3 Hz, 2H), 3.23 - 3.30 (m, 2H), 3.38 - 3.46 (m, 2H), 4.31 (d, *J* = 5.3 Hz, 2H), 4.69 - 4.88 (m, 2H), 5.49 (t, *J* = 5.6 Hz, 1H), 6.33 (d, *J* = 1.9 Hz, 1H), 6.47 (d, *J* = 8.4 Hz, 1H), 6.63 (dd, *J* = 1.9, 8.0 Hz, 1H), 7.19 - 7.27 (m, 1H), 7.33 (q, *J* = 3.2 Hz, 4H).

**$^{13}$C NMR (101 MHz, MeOD)** δ 25.19, 26.14, 31.45, 31.64, 33.18, 47.25, 51.41, 88.06, 89.75, 108.82, 109.92, 118.19, 123.01, 127.03, 127.08, 127.50, 128.76, 128.92, 134.63, 137.15, 140.41, 170.97.

**MS (ESI)** m/z = 410.5 $[M+H]^+$.

**HRMS:** calcd for $C_{25}H_{32}N_3O_1F_1$ 410.2601, found 410.2614 $[M + H]^+$.

*tert*-butyl (1-(3-(benzylamino)-4-(cyclohexylamino)benzoyl)pyrrolidin-3-yl)carbamate **(40)**

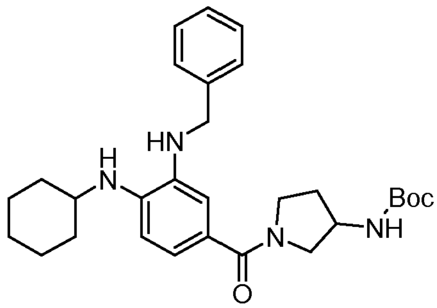

General procedure I was followed using compound **26** (0.200 g, 0.438 mmol) and *tert*-butyl pyrrolidin-3-ylcarbamate (0.114 g, 0.613 mmol) as the corresponding amine to provide *tert*-butyl (1-(3-(benzylamino)-4-(cyclohexylamino)benzoyl)pyrrolidin-3-yl)carbamate **40** (0.125 g, 0.254 mmol, 58 % yield).

**$^{1}$H NMR (400 MHz, MeOD)** δ 1.26 - 1.33 (m, 2H), 1.44 (d, *J* = 16.3 Hz, 12H), 1.69 (tt, *J* = 6.8, 15.1 Hz, 2H), 1.74 - 1.95 (m, 3H), 2.09 (dd, *J* = 3.5, 12.6 Hz, 2H), 2.96 - 3.28 (m, 2H), 3.35 (dd, *J* = 3.7, 10.4 Hz, 1H), 3.61 (ddd, *J* = 7.2, 15.5, 63.5 Hz, 2H), 3.83 - 4.08 (m, 1H), 4.37 (s, 2H), 6.61 (dd, *J* = 4.9, 8.3 Hz, 2H), 6.90 (d, *J*= 8.3 Hz, 1H), 7.21 (t, *J* = 7.4 Hz, 1H), 7.30 (dd, *J* = 6.7, 8.4 Hz, 2H), 7.36 (d, *J* = 7.1 Hz, 2H).

**$^{13}$C NMR (101 MHz, MeOD)** δ 13.07, 19.46, 21.65, 24.88, 25.74, 27.35, 32.84, 32.88, 51.51, 53.40, 60.14, 78.93, 109.36, 111.29, 118.88, 126.52, 126.92, 128.14, 134.56, 138.45, 139.82, 171.59, 171.88.

**MS (ESI)** m/z = 493.4 $[M+H]^+$.

*tert*-butyl 4-(4-(cyclohexylamino)-3-((4-fluorobenzyl)amino)benzoyl)piperazine-1-carboxylate **(41)**

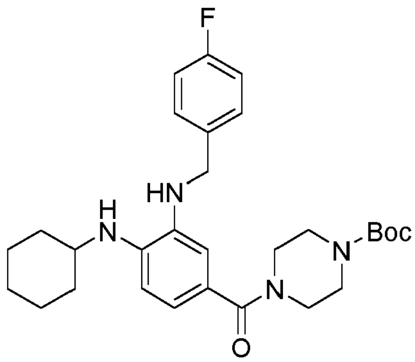

General procedure I was followed using compound **27** (0.200 g, 0.584 mmol) and *tert*-butyl piperazine-1-carboxylate (0.163 g, 0.876 mmol) as the corresponding amine to provide *tert*-butyl 4-(4-(cyclohexylamino)-3-((4-fluorobenzyl)amino)benzoyl)piperazine-1-carboxylate **41** (0.171 g, 0.335 mmol, 57 % yield).

**$^1$H NMR (400 MHz,MeOD)** δ 11.19 - 1.35 (m, 3H), 1.40 (dt, *J* = 3.2, 12.6 Hz, 1H), 1.46 (s, 9H), 1.69 (dt, *J* = 3.1, 12.7 Hz, 1H), 1.81 (dt, *J* = 3.7, 13.1 Hz, 2H), 2.09 (dd, *J* = 3.8, 12.6 Hz, 2H), 3.27 - 3.38 (m, 1H), 3.41 (s, 3H), 4.36 (s, 2H), 4.62 (s, 1H), 6.43 (d, *J* = 2.0 Hz, 1H), 6.63 (d, *J* = 8.2 Hz, 1H), 6.80 (dd, *J* = 1.9, 8.2 Hz, 1H), 6.98 - 7.08 (m, 2H), 7.30 - 7.39 (m, 2H).

**$^{13}$C NMR (101 MHz, MeOD)** δ 26.27, 27.13, 28.60, 34.27, 47.94, 49.28, 49.50, 52.91, 81.63, 111.06, 112.35, 116.12 (d, *J* = 21.5 Hz), 120.41, 123.52, 129.96 (d, *J* = 8.0 Hz) 135.98, 137.01, 137.04, 139.78, 156.22, 162.95 (d, *J* = 243.4 Hz). 164.49, 174.33.

**MS (ESI)** m/z = 511.4 [M+H]$^+$.

(4-(cyclohexylamino)-3-((4-fluorobenzyl)amino)phenyl)(4-methylpiperazin-1-yl)methanone **(CPD-019, 42)**

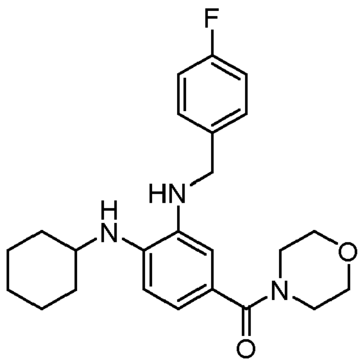

General procedure I was followed using compound **27** (0.200 g, 0.584 mmol) and morpholine (0.071 ml, 0.818 mmol) as the corresponding amine to provide **42** (4-(cyclohexylamino)-3-((4-fluorobenzyl)amino)phenyl)(morpholino) methanone (0.126 g, 0.306 mmol, 52 % yield).

**$^1$H NMR (400 MHz,MeOD)** δ 1.19- 1.35 (m, 4H), 1.36- 1.51 (m, 2H), 1.68 (s, 1H), 1.81 (dt, *J* = 3.7, 13.1 Hz, 2H), 2.04-2.13 (m, 2H), 3.45 (s, 2H), 3.51 (s, 2H), 4.36 (s, 2H), 4.61 (s, 3H), 6.44 (d, *J*= 2.0 Hz, 1H), 6.63 (d, *J*= 8.2 Hz, 1H), 6.79 (dd, *J* = 1.9, 8.1 Hz, 1H), 6.98 - 7.09 (m, 2H), 7.32 - 7.40 (m, 2H).

**$^{13}$C NMR (101 MHz, MeOD)** δ 26.27, 27.13, 34.27, 48.04, 52.92, 67.81, 111.09, 111.21, 112.39, 116.11 (d, *J* = 21.7 Hz), 120.32, 123.50, 129.99 (d, *J* = 8.1 Hz), 135.10, 136.12,137.04 (d, *J* = 2.7 Hz), 139.72, 142.68, 162.08, 174.17.

**MS (ESI)** m/z =412.4 [M+H]$^+$.

**HRMS:** calcd for $C_{24}H_{30}N_3O_2F_1$ 412.2395, found 412.2401 [M + H]$^+$.

(4-(cyclohexylamino)-3-((4-fluorobenzyl)amino)phenyl)(morpholino)methanone **(CPD-020, 43)**

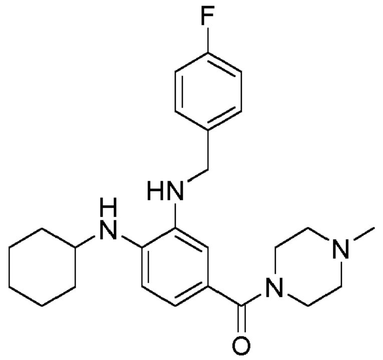

General procedure I was followed using compound **27** (0.200 g, 0.584 mmol) and 1-methylpiperazine (0.088 g, 0.876 mmol) as the corresponding amine to provide ((4-(cyclohexylamino)-3-((4-fluorobenzyl)amino)phenyl)(4-methylpiperazin-1-yl)methanone **43** (0.110 g, 0.259 mmol, 44 % yield).

**$^1$H NMR (400 MHz, DMSO)** δ 1.12 - 1.28 (m, 3H), 1.36 (qt, *J* = 3.3, 12.5 Hz, 2H), 1.63 (dt, *J* = 3.6, 12.7 Hz, 1H), 1.75 (dq, *J* = 3.6, 13.1 Hz, 2H), 1.99 (dd, *J* = 3.8, 12.6 Hz, 2H), 2.08 - 2.11 (m, 4H), 2.13 (s, 3H), 3.21 - 3.32 (m, 5H), 4.29 (d, *J* = 5.3 Hz, 2H), 4.75 (d, *J*= 7.2 Hz, 1H), 5.52 (t, *J* = 5.5 Hz, 1H), 6.26 (d, *J* = 1.9 Hz, 1H), 6.47 (d, *J* = 8.2 Hz, 1H), 6.61 (dd, *J* = 1.9, 8.0 Hz, 1H), 7.15 (t, *J* = 8.9 Hz, 2H), 7.32 - 7.40 (m, 2H).

**$^{13}$C NMR (101 MHz, DMSO)** δ 24.42, 25.38, 32.41, 45.33, 45.77, 50.64, 54.28, 108.11, 109.37, 114.74 (d, *J* = 21.1 Hz), 117.62, 122.28, 128.49 (d, *J*= 8.0 Hz), 133.64, 135.72 (d, *J*= 2.8 Hz), 136.37,160.83 (d, *J*= 241.7 Hz), 170.02.

**MS (ESI)** m/z = 425.4 $[M+H]^+$.

**HRMS:** calcd for $C_{25}H_{33}N_4O_1F_1$ 425.2711, found 425.2720 $[M + H]^+$.

(4-(cyclohexylamino)-3-((4-fluorobenzyl)amino)phenyl)(4-fluoropiperidin-1-yl)methanone **(CPD-021, 44)**

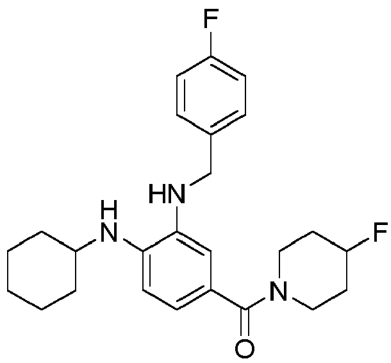

General procedure I was followed using compound **27** (0.200 g, 0.584 mmol) and 4 4-fluoropiperidine (0.084 g, 0.818 mmol) as the corresponding amine to provide (4-(cyclohexylamino)-3-((4-fluorobenzyl)amino)phenyl)(4-fluoropiperidin-1-yl)methanone **44** (0.039 g, 0.091 mmol, 16 % yield).

**$^1$H NMR (400 MHz, MeOD)** δ 1.21 - 1.33 (m, 4H), 1.35 - 1.49 (m, 3H), 1.68 (s, 4H), 1.81 (d, *J* = 13.9 Hz, 3H), 2.09 (d, *J*= 12.3 Hz, 2H), 3.51 (s, 3H), 4.35 (s, 2H), 4.61 (s, 7H), 4.72 (dq, *J* = 3.2, 6.4 Hz, 1H), 6.45 (d, *J* = 2.0 Hz, 1H), 6.63 (d, *J* = 8.2 Hz, 1H), 6.78 (dd, *J* = 1.9, 8.2 Hz, 1H), 6.97 - 7.08 (m, 2H), 7.31 - 7.39 (m, 2H).

**$^{13}$C NMR (101 MHz, MeOD)** δ 26.27, 27.14, 34.28, 48.10, 52.96, 88.13, 89.83, 111.67 (d, *J* = 97.0 Hz), 116.10 (d, *J* = 21.6 Hz), 119.95, 124.12, 130.06 (d, *J* = 8.5 Hz), 136.66 (d, *J* = 80.5 Hz), 139.60, 152.56, 159.38, 164.53, 174.25.

**MS (ESI)** m/z = 428.4 $[M+H]^+$.

**HRMS:** calcd for $C_{25}H_{31}N_3O_1F_2$ 428.2508, found 428.2524 $[M + H]^+$.

(3,4-bis(cyclohexylamino)phenyl)(piperazin-1-yl)methanone **(CPD-005, 45)**

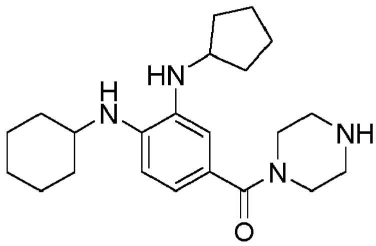

General procedure D was followed using compound **28** (0.140 g, 0.297 mmol), to provide (3,4-bis(cyclohexylamino)phenyl)(piperazin-1-yl)methanone **45** (0.126 g, 0.273 mmol, 88 % yield).

**$^1$H NMR (400 MHz, MeOD)** δ 1.19 - 1.51 (m, 5H), 1.63 - 1.72 (m, 3H), 1.72 - 1.91 (m, 7H), 1.95 - 2.09 (m, 4H), 3.31 (t, *J* = 6.1 Hz, 5H), 3.46 (td, *J* = 3.9, 10.4 Hz, 1H), 3.87 (s, 4H), 3.99 (q, *J* = 6.4 Hz, 1H), 7.11 (d, *J*= 8.0 Hz, 1H), 7.23 (d, *J*= 8.0 Hz, 1H), 7.30 (s, 1H).

**$^{13}$C NMR (101 MHz, MeOD)** δ 24.93, 25.85, 26.46, 31.82, 32.34, 44.40, 56.42, 60.37, 119.43, 121.87, 125.53, 171.62.

**MS (ESI)** m/z = 371.4 [M + H]$^+$.

**HRMS:** calcd for $C_{22}H_{34}N_4O_1$ 371.2805, found 371.2810 [M + H]$^+$.

(3-(benzylamino)-4-(cyclohexylamino)phenyl)(piperazin-1-yl)methanone **(CPD-008, 46)**

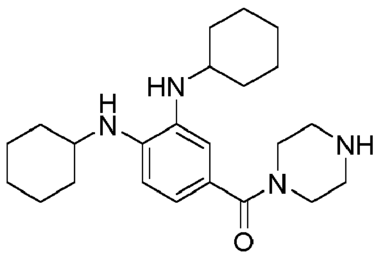

General procedure D was followed using compound **31** (0.202 g, 0.417 mmol), to provide (3-(benzylamino)-4-(cyclohexylamino)phenyl)(piperazin-1-yl)methanone **46** (0.152 g, 0.308 mmol, 74 % yield).

**$^1$H NMR (400 MHz, MeOD)** δ 1.20 - 1.60 (m, 10H), 1.70 (dd, *J* = 5.9, 9.9 Hz, 2H), 1.86 (ddd, *J* = 3.4, 7.9, 12.4 Hz, 5H), 2.00 - 2.12 (m, 5H), 3.30 - 3.38 (m, 7H), 3.43 - 3.58 (m, 2H), 3.89 (t, *J* = 5.2 Hz, 4H), 7.06 (d, *J* = 8.4 Hz, 1H), 7.32 - 7.40 (m, 2H).

**MS (ESI)** m/z = 385.4 [M+H]$^+$.

**HRMS:** calcd for $C_{23}H_{36}N_4O_1$ 385.2962, found 385.2953 [M + H]$^+$.

(3-aminopyrrolidin-1-yl)(3,4-bis(cyclohexylamino)phenyl)methanone **(CPD-012, 47)**

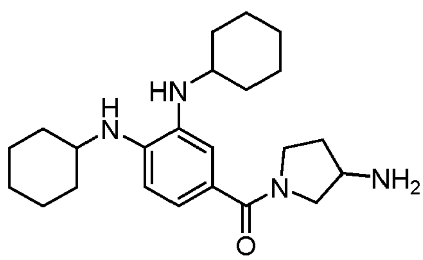

General procedure D was followed using compound **35** (0.089 g, 0.184 mmol), to provide (3-aminopyrrolidin-1-yl)(3,4-bis(cyclohexylamino)phenyl)methanone **47** (0.090 g, 0.182 mmol, 94 % yield).

**$^1$H NMR (400 MHz, MeOD)** δ 1.21 - 1.61 (m, 11H), 1.70 (d, *J* = 11.9 Hz, 3H), 1.80 - 1.92 (m, 5H), 2.02 - 2.09 (m, 5H), 2.09 - 2.19 (m, 2H), 2.41 (s, 1H), 3.49 (qd, *J* = 3.9, 10.2 Hz, 2H), 3.64 - 3.79 (m, 2H), 3.86 (s, 1H), 3.99 (s, 2H), 7.03 (d, *J* = 8.8 Hz, 1H), 7.49 (s, 2H).

**MS (ESI)** m/z = 385.4 $[M+H]^+$.
**HRMS:** calcd for $C_{23}H_{36}N_4O_1$ 385.2962, found 385.2966 $[M + H]^+$.

(3-(benzylamino)-4-(cyclohexylamino)phenyl)(piperazin-1-yl)methanone **(CPD-013, 48)**

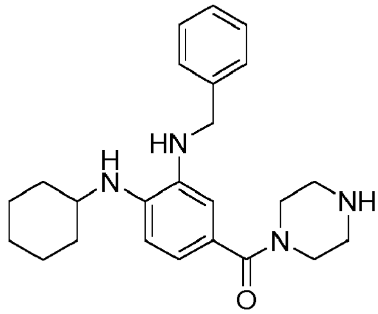

General procedure D was followed using compound **36** (0.125 g, 0.254 mmol), to provide (3-(benzylamino)-4-(cyclohexylamino)phenyl)(piperazin-1-yl)methanone **48** (0.059 g, 0.117 mmol, 46 % yield).
**$^1$H NMR (400 MHz, MeOD)** δ 1.27 - 1.40 (m, 3H), 1.48 (td, *J* = 3.2, 11.8 Hz, 2H), 1.71 (d, *J* = 11.2 Hz, 1H), 1.88 (dt, *J* = 3.2, 13.1 Hz, 2H), 2.06 (dd, *J* = 3.9, 12.4 Hz, 2H), 3.12 (s, 2H), 3.66 (s, 2H), 3.90 (t, *J*= 5.3 Hz, 1H), 4.53 (s, 2H), 6.79 (d, *J*= 1.8 Hz, 1H), 6.96 (dd, *J* = 1.8, 8.2 Hz, 1H), 7.25 (d, *J* = 8.2 Hz, 1H), 7.30 - 7.41 (m, 5H).
**$^{13}$C NMR (101 MHz, MeOD)** δ 24.36, 24.82, 30.03, 42.81, 57.50, 114.48, 118.60, 122.15, 126.16, 127.53, 127.56, 128.63, 131.92, 136.95, 170.55.
**MS (ESI)** m/z = 393.4 $[M]^+$.
**HRMS:** calcd for $C_{24}H_{32}N_4O_1$ 393.2649, found 392.2654 $[M + H]^+$.

(3-aminopyrrolidin-1-yl)(3-(benzylamino)-4-(cyclohexylamino)phenyl)methanone **(CPD-017, 49)**

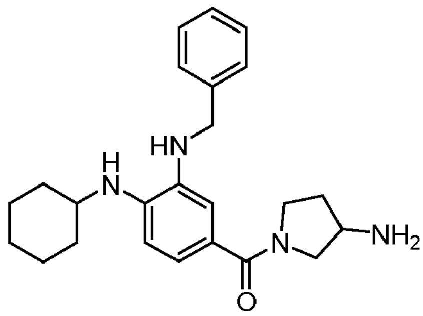

General procedure D was followed using compound **40** (0.115 g, 0.233 mmol), to provide (3-aminopyrrolidin-1-yl)(3-(benzylamino)-4-(cyclohexylamino)phenyl)methanone **49** (0.111 g, 0.206mmol, 88 % yield).
**$^1$H NMR (400 MHz, $D_2O$)** δ 1.11 - 1.22 (m, 3H), 1.33 (qd, *J* = 7.4, 12.6 Hz, 2H), 1.54 (d, *J* = 12.3 Hz, 1H), 1.66 - 1.75 (m, 2H), 1.85 - 1.91 (m, 2H), 2.07 - 2.29 (m, 1H), 2.95 - 3.14 (m, 2H), 3.20 - 3.39 (m, 1H), 3.42 - 3.57 (m, 2H), 3.73 (ddd, *J* = 6.6, 10.3, 13.2 Hz, 1H), 3.90 (t, *J* = 5.6 Hz, 1H), 4.39 (d, *J* = 9.6 Hz, 2H), 6.71 (dd, *J* = 1.8, 5.4 Hz, 1H), 6.81 - 6.89 (m, 1H), 7.16 (dd, *J* = 5.5, 8.2 Hz, 1H), 7.21 - 7.29 (m, 5H).
**$^{13}$C NMR (101 MHz, $D_2O$)** δ 18.82, 24.12, 24.33, 24.54, 25.06, 27.71, 29.42, 29.50, 29.58, 31.38, 43.90, 46.84, 47.49, 48.92, 49.34, 50.17, 52.07, 58.48, 58.76, 114.53, 115.08, 117.67, 118.27, 123.56, 123.96, 127.65, 127.72, 127.75, 127.82, 128.90, 128.93, 134.06, 134.69, 136.84, 137.15, 170.63.
**MS (ESI)** m/z = 393.4 $[M]^+$.
**HRMS:** calcd for $C_{24}H_{32}N_4O_1$ 393.2649, found 393.2660 $[M + H]^+$.

(4-(cyclohexylamino)-3-((4-fluorobenzyl)amino)phenyl)(piperazin-1-yl)methanone **(CPD-018, 50)**

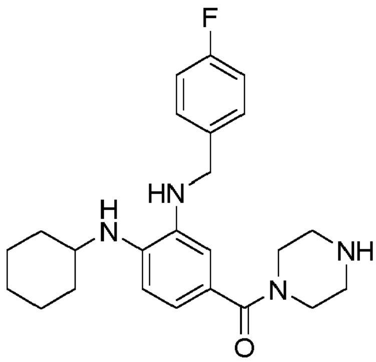

General procedure D was followed using compound **41** (0.165 g, 0.323 mmol), to provide (4-(cyclohexylamino)-3-((4-fluorobenzyl)amino)phenyl)(piperazin-1-yl)methanone **50** (0.119 g, 0.290 mmol, 90 % yield).

**$^1$H NMR (400 MHz,DMSO** δ 1.06 - 1.34 (m, 4H), 1.45 (q, *J* = 12.0 Hz, 2H), 1.60 (d, 1H), 1.76 (dt, *J* = 3.5, 13.2 Hz, 2H), 1.94 - 2.02 (m, 2H), 3.02 (br. s, 4H), 3.31 - 3.48 (m, 5H), 4.37 (s, 2H), 6.71 (s, 1H), 6.82 (d, *J*= 8.0 Hz, 1H), 7.12 (s, 1H), 7.16 (t, *J* = 8.7 Hz, 2H), 7.45 (dd, *J* = 5.5, 8.4 Hz, 2H), 9.35 (br. s, 2H).

**$^{13}$C NMR (101 MHz, DMSO)** δ 24.21, 25.00, 25.23, 29.95, 42.41, 46.18, 66.36, 115.00, 115.21, 129.77, 132.58, 133.96, 156.59, 160.15, 162.57, 168.95.

**MS (ESI)** m/z = 411.2 $[M]^+$.

**HRMS:** calcd for $C_{24}H_{31}N_4O_1F_1$ 411.2555 found 411.2564 $[M + H]^+$.

## 2. Biological evaluation of the compounds of the invention

Fluorescence-Enabled Inhibited Autoxidation (FENIX)

Ref. Shah *et al.* (2019) Cell Chem. Biol. 26, 1594-1607

Unless otherwise stated, laboratory reagent grade solvents were used. Reagents were obtained from various commercial sources and were used without any prior purification. L-α-phosphatidylcholine (Egg, Chicken), powder was purchased from Merck Life Science B.V.. DTUN and STY-BODIPY were synthesized according to methods described in Shah et al. (2019) Cell Chem. Biol. 26, 1594-1607. Fluorescence was measured using the UV/vis spectrophotometer Synergy MX, Biotek with Gen5.

To quantify radical trapping antioxidant activity in phospholipid bilayers to a clear bottom black-walled 96-well plate for fluorescence-base assays (Invitrogen by Thermo Fisher Scientific) was added 250 μL of a solution containing liposomes (1 mM), styrene-conjugated BODIPY (STY-BODIPY, 1 μM), and the respective radical trapping antioxidant (RTA, 4 μM). The solutions were made in larger volumes in Eppendorfs, pre-mixed and 250 μL aliquot was transferred to each well. The plate was incubated for 10 minutes at 37°C in the BioTek SynergyMx plate reader, followed by a fast-mixing protocol for 5 minutes. The plate was ejected from the plate reader and autoxidation was initiated by the addition of a 50 μL aliquot of (E)-1,2-*bis*((2-methyldecan-2-yl)oxy)diazene (DTUN, 0.2 mM in EtOH/PBS 3/47, v/v), followed by another mixing protocol for 5 minutes. Data were acquired by excitation probes at 488 nm and emission was measured at 518 nm (read intervals 1.0 min). Kinetic read parameters: (i) optic position: bottom, (ii) gain: 80, (iii) bandwidth: 9.0. The results of the FENIX assay are presented in Table 2 as inhibition rate constants ($k_{inh}$), log$k_{inh}$ and stoichiometries (n) of tested compounds.

**Table 2.**

| **Compound code** | **FENIX assay** | | |
|---|---|---|---|
| | **$k_{inh}$ ($10^4$ $M^{-1}s^{-1}$) ± SD** | **Stoichiometry ± SD** | **Log$k_{inh}$ ± SD** |
| **CPD-001** | 0.1 | 0 | 3 |
| **CPD-002** | 0.1 | 0 | 3 |
| **CPD-003** | 0.1 | 0 | 3 |
| **CPD-004** | 0.59 | 5.0 | 3.77 |
| **CPD-005** | 1.5 | 3.9 | 4.19 |
| **CPD-006** | 5.1 | 2.8 | 4.71 |

(continued)

| Compound code | FENIX assay | | |
|---|---|---|---|
| | $k_{inh}$ ($10^4$ $M^{-1}s^{-1}$) ± SD | Stoichiometry ± SD | $Logk_{inh}$ ± SD |
| **CPD-007** | 3.5 | 3.5 | 4.54 |
| **CPD-008** | 4.8 | 2.6 | 4.68 |
| **CPD-009** | 1.4 | 3.6 | 4.15 |
| **CPD-010** | 2.2 | 2.7 | 4.38 |
| **CPD-011** | 4.6 | 2.9 | 4.67 |
| **CPD-012** | 3.4 | 3.7 | 4.54 |
| **CPD-013** | 5.5 | 2.6 | 4.74 |
| **CPD-014** | 1.9 | 3.0 | 4.28 |
| **CPD-015** | 8.9 | 2.1 | 4.84 |
| **CPD-016** | 6.4 | 2.0 | 4.81 |
| **CPD-017** | 1.7 | 3.8 | 4.22 |
| **CPD-018** | 9.1 | 2.2 | 4.96 |
| **CPD-019** | 6.8 | 2.2 | 4.83 |
| **CPD-020** | 9.1 | 2.9 | 4.55 |
| **CPD-021** | 1.4 | 3.7 | 4.53 |

Inhibition of ML162-Induced Ferroptosis in HT1080 human fibrosarcoma cells

Human fibrosarcoma cells HT1080 cells were obtained from American Type Culture Collection (ATCC). HT1080 cells were cultured in EMEM medium supplemented with 10% FCS and L-glutamine (1 mM), sodium pyruvate and nonessential amino acids. Cell death was measured using Envision multimode plate reader (PE). To determine $IC_{50}$ values, HT1080 were seeded in a 384-well plate at a density of 3500 cells/well in 40μl in an incubator overnight at 37 °C with 5% CO2. The next day, the cells were pretreated for 1h (in triplicates) with a 1/3 dilution series of ferrostatin-1 analogues ranging from 1 μM to 0.5 nM and Sytox Green (1.6 μM). All the compound's stock solutions were prepared in DMSO at 100 mM concentration. After stimulating the cells with ML-162 1 μM the plate was transferred to the incubator. SytoxGreen intensity was measured after 8, 12, 16 and 24 h using an excitation filter of 485 nm and an emission filter of 535 nm. Dose-response curves were made as % cell death inhibition, taking ML162 treated samples as 0 % inhibition and Fer-1 (1 μM) + ML-162 samples as 100 % inhibition. Cell death percentage was calculated as 100 - (( x - 100%inh) / ( 0%inh - 100%inh))*100). Curves were plotted in Spotfire software, and $IC_{50}$ values were calculated using logistic regression curves. Data at 16 h is presented in Table 3.

Kinetic solubility

A turbidimetric method was used. First, a series of DMSO compound stock solutions were prepared (0.15-5 mM) from the stock solution of the compound in DMSO (10 mM). An aliquot of 4μL stock solution was added to 196μL PBS buffer (pH 7.4). A series of concentrations were prepared (3.13-200 μM), including a blank on a microtiter plate. The microtiter plate was shaken for 10 seconds and incubated for 2 hours at 37°C. Turbidity was measured using the UV/vis spectrophotometer Synergy MX, Biotek with Gen5. When there was no turbidity measured at a given concentration the sample was assumed to be dissolved. Data is presented in Table 3.

Central nervous system multiparameter optimization (CNS MPO) score

To evaluate brain penetration, central nervous system multiparameter optimization (CNS MPO) score was calculated using CDD Vault software (Collaborative Drug Discovery Inc., Burlingame, California, USA). CNS MPO score consists of six fundamental physicochemical properties: lipophilicity, calculated partition coefficient (ClogP), calculated distribution coefficient at pH 7.4 (ClogD), molecular weight (MW), topological polar surface area (TPSA), number of hydrogen-bond donors (HBDs), and most basic center ($pK_a$). A CNS MPO score of > 4.0 is preferable to penetrate the

brain. Data is presented in Table 3.

**Table 3.**

| Compound code | Kin. solubility (μM) | Ferroptosis inhibition $IC_{50}$ (nM) | CNS MPO score |
|---|---|---|---|
| **CPD-001** | >200 | 40.7 ± 9.73 | 5.0 |
| **CPD-002** | >200 | 309 ± 129 | 5.2 |
| **CPD-003** | >200 | 111 ± 2.61 | 5.2 |
| **CPD-004** | >200 | 108 ± 3.19 | 4.0 |
| **CPD-005** | >200 | 5.87 ± 2.71 | 3.9 |
| **CPD-006** | >200 | 13.9 ± 1.80 | 3.5 |
| **CPD-007** | 50-100 | 5.48 ± 0.07 | 3.7 |
| **CPD-008** | 25-50 | 13.1 ± 0.585 | 3.1 |
| **CPD-009** | >200 | 8.10 ± 7.02 | 3.4 |
| **CPD-010** | >200 | 8.79 ± 5.63 | 3.6 |
| **CPD-011** | 12.5-25 | 8.82 ± 2.69 | 3.3 |
| **CPD-012** | 25-50 | 6.11 ± 0.528 | 3.4 |
| **CPD-013** | 12.5-25 | 12.9 ± 0.348 | 3.0 |
| **CPD-014** | >200 | 8.24 ± 7.40 | 4.2 |
| **CPD-015** | 50-100 | 14.6 ± 0.890 | 3.8 |
| **CPD-016** | >200 | 6.63 ± 1.77 | 4.0 |
| **CPD-017** | >200 | 4.51 ± 3.24 | 3.8 |
| **CPD-018** | 50-100 | 12.6 ± 3.13 | 3.5 |
| **CPD-019** | 100-200 | 7.02 ± 1.78 | 3.6 |
| **CPD-020** | 12.5-25 | 10.3 ± 3.76 | 3.2 |
| **CPD-021** | >200 | 4.20 ± 3.14 | 3.1 |

Human and mouse microsomal stability

**Experimental procedure**

Pooled liver microsomes (Ultrapool Human Liver microsomes or Mouse CD-1 male Liver microsomes), with a protein concentration of 20 mg/ml, were purchased from Corning Gentest™ (now Discovery Life Science - Gentest) and stored at -80°C until use. The microsomes (final protein concentration 0.5 mg/ml), 0.1 M phosphate buffer pH 7.4 and test compound (final substance concentration 1 μM), were preincubated at 37°C for 10 min before the addition of NADPH Regenerating System (solution A and B from Corning Gentest™, final concentration 1 mM) to initiate the reaction. For each compound tested, a control without the cofactor was included. In this case, 0.1 M phosphate buffer at pH 7.4 was added instead of the NADPH regenerating system. In addition, with each new batch of microsomes, two positive control compounds were incorporated. These controls represented substances with high (verapamil, with human $Cl_{int}$ = 178.9 μL/min*mg; diazepam, with mouse $Cl_{int}$ = 549 μL/min*mg) and low clearance (dextromethorphan, with human $Cl_{int}$= 34.6 μL/min*mg; diphenhydramine, with mouse Clint = 64.0 μL/min*mg).

Each compound was incubated for 45 minutes at 37°C and shaken at 500 rpm. The reactions were stopped by transferring the incubate into acetonitrile, containing an internal standard, in Eppendorfs at the appropriate time points in a 1:3 ratio (0, 5, 10, 15, 30, and 45 minutes for compounds, and 0, 15, and 45 minutes for the negative control of those compounds). Subsequently, the Eppendorfs were centrifuged at 3000 rpm for 20 minutes at 4°C to precipitate the protein.

**Quantitative Analysis**

After protein precipitation, the sample supernatant from each time point was analysed using UPLC-MS/MS. The

analysis followed the general UPLC method for microsomal stability described below. The UPLC (ultra-performance liquid chromatography), used to quantify the microsomal stability of the products, was an ACQUITY UPLC H-Class system with a TUV detector Waters (not used in this assay) coupled to an MS/MS detector Xevo Waters TQD. Waters Acquity UPLC BEH C18 1.7 μm, 2.1 mm × 50 mm column was used. The eluent was composed of two different solvents. Solvent A consisted of water with 0.1% formic acid, solvent B was acetonitrile with 0.1% formic acid. The column was first equilibrated for with a mixture of 95% solvent A and 5% solvent B until the delta of psi decrease below 40 psi. The method began with a short equilibration to reach 50% of solvent A and B in 0.15 min. Following this, solvent B was increased linearly to 95% over 2.75 min before being held constant for 0.70 min (flow rate 0.7 ml/min). The specific masses of the compounds were tracked using tuning files generated via Intellistart®, and quantification was aided by an internal standard.

**Data processing**

All obtained readouts were processed using Microsoft Excel. From a plot of ln peak area ratio (compound peak area/ internal standard peak area) against time, the gradient of a line is determined by linear regression. Subsequently several parameters were calculated using the equations below:

- $$\text{Elimination rate constant } (k) = (-\text{gradient})$$
- $$\text{Half Time } (t_{1/2}) = \ln(2)/k$$
- $$Cl_{int}\ (\mu L/min/mg \text{ of protein}) = V \times \ln(2) \times t_{1/2}$$
- Where

$$V = (\text{Incubation volume } \mu L)/(\text{Microsomal protein mg/g liver})$$

Data is presented in Table 4.

**Table 4.**

| **Compound code** | **Microsomal stability in mouse** | | **Microsomal stability in human** | |
|---|---|---|---|---|
| | **$t_{1/2}$ (min)[a]** | **$Cl_{int}$ in liver microsomes (μL/min*mg)[b]** | **$t_{1/2}$ (min)[a]** | **$Cl_{int}$ in liver microsomes (μL/min*mg)[b]** |
| Diazepam | 2.0 | 707.0 | n.d | n.d |
| Diphenhydramine | 30.7 | 45.1 | n.d | n.d |
| Verapamil | n.d. | n.d. | 16.3 | 85 |
| Dextromethorphan | n.d. | n.d. | 39.9 | 34.7 |
| **CPD-001** | 212.3 | 6.5 | 191.8 | 7.2 |
| **CPD-002** | n.d. | n.d. | n.d. | n.d. |
| **CPD-003** | n.d. | n.d. | n.d. | n.d. |
| **CPD-004** | n.d. | n.d. | n.d. | n.d. |
| **CPD-005** | 37 | 37.5 | > 250 | 5.5 |
| **CPD-006** | 1.8 | 776.8 | n.d. | n.d. |
| **CPD-007** | 5.6 | 247.9 | n.d. | n.d. |
| **CPD-008** | 2.2 | 635.9 | n.d. | n.d. |

(continued)

| Compound code | Microsomal stability in mouse | | Microsomal stability in human | |
|---|---|---|---|---|
| | $t_{1/2}$ (min)[a] | $Cli_{nt}$ in liver microsomes (μL/min*mg)[b] | $t_{1/2}$ (min)[a] | $Cl_{int}$ in liver microsomes (μL/min*mg)[b] |
| **CPD-009** | 83.2 | 16.7 | >250 | <5.5 |
| **CPD-010** | 29.7 | 46.6 | 193.5 | 7.2 |
| **CPD-011** | 0.6 | 2493.8 | n.d. | n.d. |
| **CPD-012** | 6.6 | 208.7 | n.d. | n.d. |
| **CPD-013** | 2.9 | 475.7 | n.d. | n.d. |
| **CPD-014** | 97.6 | 14.2 | 245.8 | 5.6 |
| **CPD-015** | 28.0 | 49.6 | > 250 | <5.5 |
| **CPD-016** | 19.2 | 72.3 | n.d. | n.d. |
| **CPD-017** | 82.2 | 16.9 | >250 | <5.5 |
| **CPD-018** | 18.1 | 76.8 | n.d. | n.d. |
| **CPD-019** | 9.8 | 142.1 | n.d. | n.d. |
| **CPD-020** | 6.9 | 199.7 | n.d. | n.d. |
| **CPD-021** | >250 | 5.5 | >250 | <5.5 |

[a]Metabolism determined in Corning ultra pool human liver microsome (HLM) or Mouse CD-1 liver microsome (MLM) activated with NAPDH and expressed as half-life ($t_{1/2}$ in min); [b]The $Cl_{int}$ (liver microsomes) was calculated using the measured microsomal $t_{1/2}$ and considers several experimental variables such as the protein concentration and the volume of incubation. Diazepam, diphenhydramine, verapamil and dextromethorphan were used as controls.

## Claims

**1.** A compound of formula (I) or a stereoisomer, or tautomer thereof,

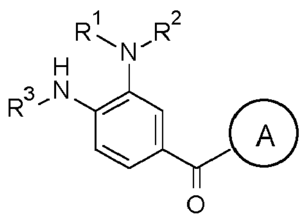

(I)

wherein, cycle A is selected from:

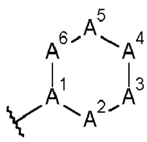

or

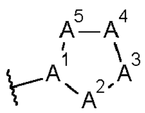

;

wherein the wavy line

(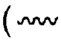)

indicates the point of attachment of cycle A to the rest of the molecule,
$A^1$ is selected from N or $CR^4$;
$A^2$ is selected from the group consisting of $NR^5$, $CR^6R^7$, S, $SO_2$ and O;
$A^3$ is selected from the group consisting of $NR^5$, $CR^6R^7$, S, $SO_2$ and O;
$A^4$ is selected from the group consisting of $NR^5$, $CR^6R^7$, S, $SO_2$ and O;
$A^5$ is selected from the group consisting of $NR^5$, $CR^6R^7$, S, $SO_2$ and O;
$A^6$ is selected from the group consisting of $NR^5$, $CR^6R^7$, S, $SO_2$ and O;
wherein when $A^1$ is $CR^4$, then at least one of $A^2$, $A^3$, $A^4$, $A^5$, or $A^6$ is selected from the group consisting of $NR^5$, $CR^6R^7$, S, $SO_2$ and O;
$R^1$ is selected from the group consisting of hydrogen, $C_{3-10}$cycloalkyl, $C_{6-12}$aryl$C_{1-6}$alkyl, $C_{3-1Q}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^1$;
$R^2$ is selected from the group consisting of $C_{3-10}$cycloalkyl, $C_{6-12}$aryl$C_{1-6}$alkyl, hydrogen, $C_{3-10}$cycloalkenyl, $C_{3-10}$cycloalkynyl, $C_{6-12}$aryl, heterocyclyl, heteroaryl, heterocyclyl$C_{1-6}$alkyl, and heteroaryl$C_{1-6}$alkyl, wherein said $C_{3-10}$cycloalkyl, $C_{6-12}$aryl$C_{1-6}$alkyl, $C_{3-10}$cycloalkenyl, $C_{3-10}$cycloalkynyl, $C_{6-12}$aryl, heterocyclyl, heteroaryl, heterocyclyl$C_{1-6}$alkyl, or heteroaryl$C_{1-6}$alkyl can be unsubstituted or substituted with one or more $Z^2$;
$R^3$ is selected from the group consisting of $C_{3-10}$cycloalkyl, $C_{3-10}$cycloalkenyl, $C_{3-10}$cycloalkynyl, $C_{6-12}$aryl, heterocyclyl, and heteroaryl, wherein said $C_{3-10}$cycloalkyl, $C_{3-10}$cycloalkenyl, $C_{3-10}$cycloalkynyl, $C_{6-12}$aryl, heterocyclyl or heteroaryl can be unsubstituted or substituted with one or more $Z^3$;
$R^4$ is selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$, wherein said $C_{1-6}$alkyl, $C_{1-10}$cycloalkyl, $C_{6-12}$aryl, $C_{6-12}$aryl$C_{1-6}$alkyl, heterocyclyl, or heteroaryl can be unsubstituted or substituted with one or more $Z^4$;
each $R^5$ is independently selected from the group consisting of hydrogen, $C_{1-6}$alkyl, halogen, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-C(O)R^{10}$, $-C(O)_2R^{10}$ and $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl, or heteroaryl can be unsubstituted or substituted with one or more $Z^5$;
each $R^6$ is independently selected from the group consisting of hydrogen, $C_{1-6}$alkyl, halogen, hydroxy, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-NR^8R^9$, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl or heteroaryl can be unsubstituted or substituted with one or more $Z^6$;
each $R^7$ is independently selected from the group consisting of hydrogen, $C_{1-6}$alkyl, halogen, hydroxy, halo$C_{1-6}$alkyl, $C_{1-6}$alkyloxy, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, cyano, $-NR^8R^9$, $-NR^8R^9S(O)_2R^{10}$, $-NR^8R^9C(O)_2R^{10}$, $-C(O)R^{10}$, $-C(O)_2R^{10}$, $-S(O)_2R^{10}$, wherein said $C_{1-6}$alkyl, $C_{3-10}$cycloalkyl, $C_{6-12}$aryl, $C_{6-12}$aryl$C_{1-6}$alkyl, heterocyclyl or heteroaryl can be unsubstituted or substituted with one or more $Z^7$;
each $R^8$ and $R^9$ is independently selected from the group consisting of hydrogen, $C_{1-6}$alkyl, $C_{3-10}$cycloalkyl, $C_{6-12}$aryl, $C_{6-12}$aryl$C_{1-6}$alkyl, heterocyclyl, and heteroaryl;
each $R^{10}$ is independently selected from the group consisting of $C_{1-6}$alkyl, hydrogen, $C_{3-10}$cycloalkyl, $C_{6-12}$aryl, $C_{6-12}$aryl$C_{1-6}$alkyl, heterocyclyl, and heteroaryl;
each $Z^1$, $Z^2$, $Z^3$, $Z^4$, $Z^5$, $Z^6$ and $Z^7$ is independently selected from the group consisting of halo, halo$C_{1-6}$alkyl, $C_{1-6}$alkyl, halo$C_{1-6}$alkyloxy, $C_{3-10}$cycloalkyl, $C_{6-12}$aryl, $C_{6-12}$aryl$C_{1-6}$alkyl, heterocyclyl, heteroaryl, hydroxyl, $-OR^{10}$, cyano, amino, $-NR^8R^9$, $-C(O)_2R^{10}$, $-C(O)NR^8R^9$, $-C(O)R^{10}$, $-S(O)R^{10}$, $-S(O)_2R^{10}$, $-S(O)_2NR^8R^9$, nitro;
or a solvate, hydrate, pharmaceutically acceptable salt, or prodrug thereof;
wherein when the moiety $-NR^1R^2$ is

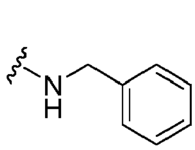
,

cycle A is piperazinyl and $R^3$ is cyclohexyl, then $R^5$ is not substituted benzyl, 4-methylpyridine or methyl.

**2.** The compound according to claim 1, having structural formula (IA),

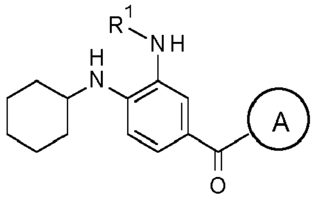

(IA)

wherein $R^1$ and cycle A have the same meaning as that defined in claim 1.

**3.** The compound according to claims 1 or 2, having structural formula (IB), or (IC),

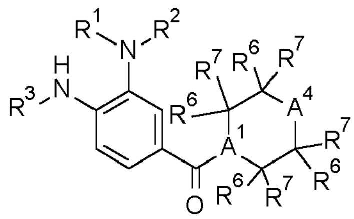

(IB)

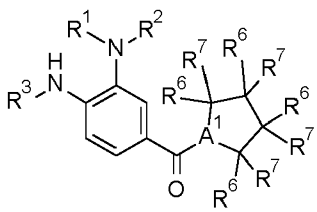

(IC)

wherein $A^1$, $A^4$, $R^1$, $R^2$, $R^3$, $R^6$, $R^7$ and $R^8$ have the same meaning as that defined in claim 1.

**4.** The compound according to claims 1-3, having structural formula (IE), or (IF),

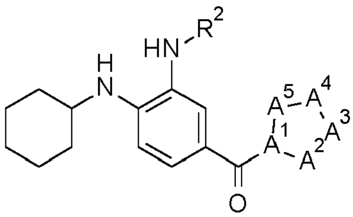

(ID)

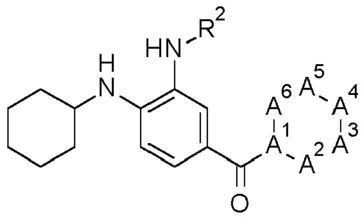

(IE)

wherein $A^1$, $A^2$, $A^3$, $A^4$, $A^5$, $A^6$, and $R^2$ have the same meaning as that defined in claim 1.

**5.** The compound according to claims 1-4, having structural formula (IF), or (IG),

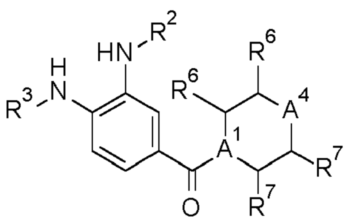

(IF)

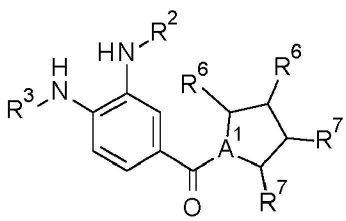

(IG)

wherein $A^1$, $A^4$, $R^2$, $R^3$, $R^6$, and $R^7$ have the same meaning as that defined in claim 1.

**6.** The compound according to claims 1-4, having structural formula (IH), or (IJ),

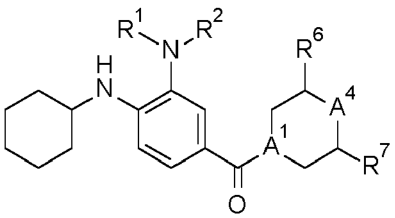
(IH)

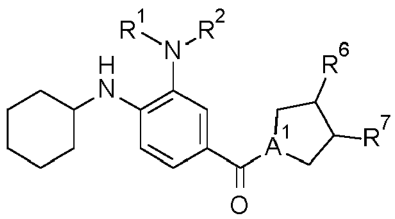
(IJ)

wherein $A^1$, $A^4$, $R^1$, $R^2$, $R^6$ and $R^7$ have the same meaning as that defined in claim 1.

**7.** A pharmaceutical composition comprising a compound of formula (I) according to any one of claims 1 to 6 and a pharmaceutically acceptable carrier.

**8.** A compound of formula (I) according to any one of claims 1 to 6, or a pharmaceutical composition according to claim 7, for use as a medicament.

**9.** A compound of formula (I) according to any one of claims 1 to 6, or a pharmaceutical composition according to claim 7, for use in the prevention or treatment of a disease associated with ferroptosis and/or oxytosis.

**10.** The compound for use according to claim 9, wherein the disease associated with ferroptosis and/or oxytosis is selected from the group consisting of liver disease, chronic kidney disease, lung disease, ocular surface diseases, wound healing, multiple organ dysfunction syndrome, neurological disease, acute renal failure, ischemia-reperfusion injury, sepsis, iron toxicity, prevention of transplant rejection, iron metabolism-related disease and genetic disorders of GPX4.

**11.** The compound for use according to claim 10, wherein the liver disease is selected from hemochromatosis, primary biliary cholangitis, non-alcoholic steatohepatitis or liver fibrosis.

**12.** The compound for use according to claim 10, wherein the neurological disease is selected from Alzheimer's Disease, Parkinson's Disease, Amyotrophic lateral sclerosis, Multiple Sclerosis, Huntington's Disease, Dementia with Lewy bodies, Friedreich's ataxia, multiple sclerosis, stroke, periventricular leukomalacia, intracerebral haemorrhage, frontotemporal dementia, neurodegeneration with brain iron accumulation, or traumatic brain injury.

**13.** The compound for use according to claim 10, wherein the ischemia-reperfusion injury is selected from myocardial ischemia-reperfusion injury, liver ischemia-reperfusion injury, renal ischemia-reperfusion injury, intestinal ischemia-reperfusion injury, cerebral ischemia-reperfusion injury, lung ischemia- reperfusion injury, or any surgical ischemia-reperfusion injury.

**14.** The compound for use according to claim 10, wherein the iron metabolism-related disease is selected from atherosclerosis or diabetes, or wherein the lung disease is selected from acute respiratory distress syndrome (ARDS), lung diseases caused by infections such as COVID-19 pulmonary disease, mucoviscidosis, or asthma.

**15.** The compound for use according to any one of claims 10-14, wherein the disease is attributable to a genetic disorder of GPX4; preferably the disease is Sedaghatian-type spondylometaphyseal dysplasia.

Europäisches Patentamt
European Patent Office
Office européen des brevets

# EUROPEAN SEARCH REPORT

Application Number
EP 24 18 7618

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/151014 A1 (MYOFORTE THERAPEUTICS INC [US]) 29 July 2021 (2021-07-29)<br>* page 174, paragraph 302 *<br>----- | 1,3,5 | INV.<br>C07D211/18<br>C07D295/16<br>C07D207/08<br>A61P11/00<br>A61K31/40<br>A61K31/44<br>A61P13/12<br>A61P25/00 |
| X | DATABASE REGISTRY [Online]<br>CHEMICAL ABSTRACTS SERVICE, COLUMBUS, OHIO, US;<br>16 April 2018 (2018-04-16),<br>XP002812748,<br>Database accession no. 221270072-4<br>* rn 2212700-72-4 *<br>----- | 1 | |
| X | DATABASE REGISTRY [Online]<br>CHEMICAL ABSTRACTS SERVICE, COLUMBUS, OHIO, US;<br>16 April 2018 (2018-04-16),<br>XP002812749,<br>Database accession no. 2212701-90-9<br>* 2212701-90-9 RN *<br>----- | 1 | |
| X | DATABASE REGISTRY [Online]<br>CHEMICAL ABSTRACTS SERVICE, COLUMBUS, OHIO, US;<br>16 April 2018 (2018-04-16),<br>XP002812750,<br>Database accession no. 2212738-34-4<br>* 2212738-34-4 RN *<br>----- | 1 | TECHNICAL FIELDS SEARCHED (IPC)<br>C07D<br>A61P<br>A61K |
| X | DATABASE REGISTRY [Online]<br>CHEMICAL ABSTRACTS SERVICE, COLUMBUS, OHIO, US;<br>16 April 2018 (2018-04-16),<br>XP002812751,<br>Database accession no. 2212738-54-8<br>* 2212738-34-4 RN *<br>-----<br>-/-- | 1 | |

The present search report has been drawn up for all claims

1

| Place of search | Date of completion of the search | Examiner |
|---|---|---|
| Munich | 13 December 2024 | Fazzi, Raffaella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 24 18 7618

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE REGISTRY [Online] CHEMICAL ABSTRACTS SERVICE, COLUMBUS, OHIO, US; 16 April 2018 (2018-04-16), XP002812752, Database accession no. 2212739-25-6 * 2212739-25-6 RN * ----- | 1 | |
| X | VINAYAGAM VINOTHKUMAR ET AL: "AlCl 3 -Mediated CHF 2 Transfer and Cyclocondensation of Difluoromethoxy Functionalized o -Phenylenediamines to Access N-Substituted Benzimidazoles", ORGANIC LETTERS, vol. 24, no. 33, 8 August 2022 (2022-08-08), pages 6142-6147, XP093233785, US ISSN: 1523-7060, DOI: 10.1021/acs.orglett.2c02231 Retrieved from the Internet: URL:https://pubs.acs.org/doi/pdf/10.1021/acs.orglett.2c02231> * page 6144 * | 1,3,5 | |
| X | & Vinayagam Vinothkumar ET AL: "Supporting Information AlCl3-Meditated CHF2 Transfer and Cyclocondensation of Difluoromethoxy Functionalized o-Phenylenediamines to Access N-Substituted Benzimidazoles", Organic Letters, 1 January 2022 (2022-01-01), pages 1-162, XP093233783, Retrieved from the Internet: URL:https://pubs.acs.org/journal/orlef7 * 2818942-71-9 RN; page S5 - page S28; example 5g * ----- -/-- | 1,3,5 | TECHNICAL FIELDS SEARCHED (IPC) |

The present search report has been drawn up for all claims

1

| Place of search | Date of completion of the search | Examiner |
|---|---|---|
| Munich | 13 December 2024 | Fazzi, Raffaella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 24 18 7618

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | RACHID SKOUTA ET AL: "Ferrostatins Inhibit Oxidative Lipid Damage and Cell Death in Diverse Disease Models", JOURNAL OF THE AMERICAN CHEMICAL SOCIETY, vol. 136, no. 12, 14 March 2014 (2014-03-14), pages 4551-4556, XP055648750, ISSN: 0002-7863, DOI: 10.1021/ja411006a * the whole document * ----- | 1-15 | |

TECHNICAL FIELDS SEARCHED (IPC)

The present search report has been drawn up for all claims

1

| Place of search | Date of completion of the search | Examiner |
|---|---|---|
| Munich | 13 December 2024 | Fazzi, Raffaella |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 24 18 7618

This annex lists the patent family members relating to the patent documents cited in the above-mentioned European search report.
The members are as contained in the European Patent Office EDP file on
The European Patent Office is in no way liable for these particulars which are merely given for the purpose of information.

13-12-2024

| Patent document cited in search report | Publication date | Patent family member(s) | Publication date |
|---|---|---|---|
| WO 2021151014 A1 | 29-07-2021 | AU 2021211732 A1 | 25-08-2022 |
| | | CA 3168494 A1 | 29-07-2021 |
| | | CN 115335043 A | 11-11-2022 |
| | | EP 4093385 A1 | 30-11-2022 |
| | | IL 294881 A | 01-09-2022 |
| | | JP 2023511084 A | 16-03-2023 |
| | | KR 20220146458 A | 01-11-2022 |
| | | US 11345702 B1 | 31-05-2022 |
| | | US 2022267325 A1 | 25-08-2022 |
| | | US 2024262820 A1 | 08-08-2024 |
| | | WO 2021151014 A1 | 29-07-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013152039 A
- WO 9933795 A
- WO 9933815 A
- WO 9933793 A
- WO 9933792 A


### Non-patent literature cited in the description


- **SKOUTA R. et al.** *J. Am. Chem. Soc.*, 2014, vol. 136, 4551-4556
- **E. L. ELIEL** ; **S. H. WILEN** ; **L. N. MANDER**. Stereochemistry of Organic Compounds. Wiley-Interscience, 1994
- **GOODMAN** ; **GILMAN**. The Pharmacological Basis of Therapeutics. McGraw-Hill, Int. Ed, 1992
- Biotransformation of Drugs, 13-15
- **DIXON et al.** *Cell*, 2012, vol. 149, 1060-1072
- **HENKE N. et al.** *Cell Death and Disease*, 2013, vol. 4, e470
- **LOUANDRE C. et al.** *Int. J. Cancer*, 2013, vol. 133, 1732
- **VAN COILLIE et al.** *Nat Comm*, 2022, vol. 13, 1046
- **STAHL** ; **WERMUTH**. Handbook of Pharmaceutical Salts: Properties, Selection, and Use. Wiley-VCH, 2002
- **K. R. MORRIS**. Pharmaceutical Solids. Marcel Dekker, 1995
- **O. ALMARSSON** ; **M. J. ZAWOROTKO**. *Chem Commun*, 2004, vol. 17, 1889-1896
- **HALEBLIAN**. *J Pharm Sci*, August 1975, vol. 64 (8), 1269-1288
- **N. H. HARTSHORNE** ; **A. STUART**. *Crystals and the Polarizing Microscope*, 1970
- **SHAH et al.** *Cell Chem. Biol.*, 2019, vol. 26, 1594-1607